# EUROPÄISCHE PATENTSCHRIFT

(11) **EP 2 086 924 B1**
(45) Veröffentlichungstag und Bekanntmachung des Hinweises auf die Patenterteilung: **12.09.2018**
(21) Anmeldenummer: 07819544.3
(22) Anmeldetag: 30.10.2007
(51) Int. Cl.: C07C 229/24, C07B 59/00, C07C 251/24, C07C 309/73, C07C 271/22, C07D 207/16, A61K 51/04, A61K 31/195

(54) **[F-18]MARKIERTE L-GLUTAMINSÄURE, [F-18]MARKIERTES L-GLUTAMIN, IHRE DERIVATE UND IHRE VERWENDUNG SOWIE VERFAHREN ZU IHRER HERSTELLUNG**
[F-18]-LABELED L-GLUTAMIC ACID, [F-18]-LABELED L-GLUTAMINE, DERIVATIVES THEREOF AND USE THEREOF AND PROCESSES FOR THEIR PREPARATION
ACIDE L-GLUTAMIQUE MARQUE AU [F-18], L-GLUTAMINE MARQUÉE AU [F-18], LEURS DERIVES ET LEUR UTILISATION, AINSI QUE LEUR PROCEDE DE FABRICATION

(30) Priorität: 01.11.2006 RU 2006138584; 18.11.2006 EP 06090211
(43) Veröffentlichungstag der Anmeldung: 12.08.2009
(73) Patentinhaber: Piramal Imaging SA, 1753 Matran (CH); A. N. Nesmeyanov Institute of Organoelement Compounds, The Russian Academy of Science (INEOS RAS), Moscow 119991 (RU); Institute of the Human Brain, Russian Academy of Science (IHB RAS), St. Petersburg 197376 (RU)
(72) Erfinder: DINKELBORG, Ludger, 13465 Berlin (DE); FRIEBE, Matthias, 13509 Berlin (DE); KRASIKOWA, Raisa, Nikolaevna, St.-Petersburg, 197341 (RU); BELOKON, Yuri, Moscow, 109240 (RU); KUZNETSOVA, Olga, Fedorovna, St.-Petersburg, 195427 (RU); GRAHAM, Keith, 10115 Berlin (DE); LEHMANN, Lutz, 42329 Wuppertal (DE); BERNDT, Mathias, 12163 Berlin (DE); SCHMITT-WILLICH, Heribert, 10551 Berlin (DE)
(74) Vertreter: Kilger, Ute
(86) Internationale Anmeldenummer: PCT/EP2007/009518
(87) Internationale Veröffentlichungsnummer: WO 2008/052788

(56) Entgegenhaltungen:
- WO-A-03/099746
- WO-A-2004/110500
- FR-A- 1 461 184
- US-A- 5 264 570
- WU F ET AL: "UPTAKE OF 14C- AND 11C-LABELED GLUTAMATE, GLUTAMINE AND ASPARTATE IN VITRO AND IN VIVO" ANTICANCER RESEARCH, Bd. 20, Nr. 1A, Januar 2000 (2000-01), Seiten 251-256, XP008039462 ISSN: 0250-7005
- LAVERMAN P ET AL: "Fluorinated amino acids for tumour imaging with positron emission tomography" EUROPEAN JOURNAL OF NUCLEAR MEDICINE, Bd. 29, Nr. 5, 2002, Seiten 681-690, XP002317618 ISSN: 0340-6997 in der Anmeldung erwähnt
- DATABASE CAPLUS [Online] CHEMICAL ABSTRACTS SERVICE, COLUMBUS, OHIO, US; POHLMAN, MATTHIAS ET AL: "Efficient Stereoselective Syntheses of Cyclic Amino Acids via Michael-Induced Ring-Closing Reactions" XP002432129 gefunden im STN Database accession no. 2003:466860 & ORGANIC LETTERS, Bd. 5, Nr. 15, 2003, Seiten 2631-2633,
- BALDWIN J E ET AL: "SYNTHESIS OF NONPROTEINOGENIC AMINO ACIDS PART 2: PREPARATION OF A SYNTHETIC EQUIVALENT OF THE GAMMA ANION SYNTHON FOR ASYMMETRIC AMINO ACID SYNTHESIS" TETRAHEDRON, Bd. 45, Nr. 5, 1989, Seiten 1453-1464, XP001155100 ISSN: 0040-4020 in der Anmeldung erwähnt
- TATEAKI WAKAMIYA ET AL: "Synthesis and stereochemistry of carnosadine, A NEW CYCLOPROPYL AMINO ACID FROM RED ALGA GRATELOUPIA CARNOSA" TETRAHEDRON LETTERS, Bd. 27, Nr. 19, 1986, Seiten 2143-2144, XP002241722 ISSN: 0040-4039 in der Anmeldung erwähnt
- JAE MIN JEONG ET AL.: "Synthesis of No-Carrier-Added [18F]Fluoroacetate" JOURNAL OF LABELLED COMPOUNDS AND RADIOPHARMACEUTICALS, Bd. 39, Nr. 5, 1997, Seiten 395-399, XP002432125
- C.-Y. SHIUE ET AL.: "Synthesis of No-Carrier-Added (NCA) [18F]fluoroalkyl Halides and their Application in the Synthesis of [18F]Fluoroalkyl Derivatives of Neurotransmitter Receptor Active Compounds" JOURNAL OF LABELLED COMPOUNDS AND RADIOPHARMACEUTICALS, Bd. 24, Nr. 1, 1987, Seiten 55-64, XP002432126
- DATABASE CA [Online] CHEMICAL ABSTRACTS SERVICE, COLUMBUS, OHIO, US; BELOKON, YU. N. ET AL: "Synthesis of enantio- and diastereo-isomerically pure .beta.- and .gamma.-substituted glutamic acids via glycine condensation with activated olefins" XP002432130 gefunden im STN Database accession no. 1988:56549 & JOURNAL OF THE CHEMICAL SOCIETY, PERKIN TRANSACTIONS 1: ORGANIC AND BIO-ORGANIC CHEMISTRY (1972-1999), Nr. 11, 1986, Seiten 1865-1872,
- BAKER S R ET AL: "Radical Reactions Leading to Substituted Pyroglutamates" TETRAHEDRON LETTERS,, Bd. 39, Nr. 18, 30. April 1998 (1998-04-30), Seiten 2815-2818, XP004113357 ISSN: 0040-4039 in der Anmeldung erwähnt
- DATABASE CAPLUS [Online] CHEMICAL ABSTRACTS SERVICE, COLUMBUS, OHIO, US; VISCONTINI, MAX ET AL: "Pyrrolizidine chemistry. VII. Preparation of l-proline and trans-3-hydroxy-dl-proline by electrochemical reduction of the corresponding pyrrolidone-2-carboxylic acids" XP002464596 gefunden im STN Database accession no. 1967:38216 & HELVETICA CHIMICA ACTA, Bd. 49, Nr. 8, 1966, Seiten 2524-2526,

## Beschreibung

Die Erfindung betrifft die in den Patentansprüchen gekennzeichneten Gegenstände, nämlich [F-18] markierte L-Glutaminsäure- und [F-18] markierte L-Glutamin-Derivate der allgemeinen Formel I sowie ihre Verwendung und Verfahren zu ihrer Herstellung.

Die frühe Diagnose von malignen Tumorerkrankungen spielt eine sehr wichtige Rolle für die Überlebensprognose eines Tumorpatienten. Bei dieser Diagnose sind nicht-invasive, bildgebende diagnostische Verfahren ein wichtiges Hilfsmittel. In den letzten Jahren hat sich hier vor allem die PET-Technologie (Positronen-Emissions-Tomographie) als besonders nützlich erwiesen. Die Sensitivität und Spezifität der PET-Technologie hängt wesentlich von der verwendeten signalgebenden Substanz (Tracer) und ihrer Verteilung im Körper ab. Bei der Suche nach geeigneten Tracern versucht man bestimmte Eigenschaften von Tumoren auszunutzen, die Tumorgewebe von gesundem, umliegenden Gewebe unterscheiden. Das bevorzugte kommerziell genutzte Isotop, welches für PET Anwendung findet, ist ¹⁸F. ¹⁸F stellt durch seine kurze Halbwertszeit von unter 2 Stunden besondere Anforderungen an die Herstellung geeigneter Tracer. Aufwändige, lange Synthesewege und Aufreinigungen sind mit diesem Isotop nicht möglich, da sonst ein erheblicher Teil der Radioaktivität des Isotops bereits abgeklungen ist, bevor der Tracer zur Diagnose eingesetzt werden kann. Es ist deshalb häufig nicht möglich etablierte Synthesewege für nichtradioaktive Fluorierungen auf die Synthese von ¹⁸F-Tracern anzuwenden. Des weiteren führt die hohe spezifische Aktivität des ¹⁸F (ca. 80 GBq/nmol) zu sehr niedrigen Substanzmengen an [¹⁸F]Fluorid für die Tracersynthese, was wiederum einen extremen Überschuß an Präkursor bedingt und den Erfolg einer auf nicht-radioaktiven Fluorierungsreaktionen basierender Radiosynthesestrategie unvorhersehbar gestaltet.

FDG ([¹⁸F]2-Fluorodesoxyglukose)-PET ist ein weithin akzeptiertes und verbreitetes Hilfsmittel in der Diagnose und weiteren klinischen Verfolgung von Tumorerkrankungen. Maligne Tumore konkurrieren mit dem Wirtsorganismus um Glukoseversorgung zur Nährstoffversorgung (Warburg O. Über den Stoffwechsel der Carcinomzelle. Biochem. Zeitschrift 1924; 152: 309-339; Kellof G. Progress and Promise of FDG-PET Imaging for Cancer Patient Management and Oncologic Drug Development. Clin Cancer Res. 2005; 11(8): 2785-2807) Dabei haben Tumorzellen im Vergleich zu umliegenden Zellen des Normalgewebes gewöhnlich einen erhöhten Glukosestoffwechsel. Dies wird bei der Verwendung von Fluorodesoxyglukose (FDG), einem Glukosederivat genutzt, welches verstärkt in die Zellen transportiert wird, dort aber nach der Phosphorylierung als FDG-6-phosphat metabolisch eingeschlossen wird ("Warburg-Effekt"). ¹⁸F markiertes FDG ist daher ein wirkungsvoller Tracer zur Detektion von Tumorerkrankungen beim Patienten mittels der PET-Technologie. Auf der Suche nach neuen PET Tracern wurden in jüngster Zeit auch zunehmend Aminosäuren für die ¹⁸F PET Bildgebung eingesetzt (z. B. (review): Eur J Nucl Med Mol Imaging. 2002 May;29(5):681-90). Dabei eignen sich einige der ¹⁸F markierten Aminosäuren für die Messung der Geschwindigkeitsrate der Proteinsynthese, die meisten anderen Derivate aber für die Messung der direkten Zellaufnahme im Tumor. Bekannte ¹⁸F markierte Aminosäuren sind z. B. von Tyrosin-, Phenylalanin-, Prolin-, Asparagin- und unnatürlichen Aminosäuren abgeleitet (z. B. J. Nucl Med 1991; 32:1338-1346, J Nucl Med 1996; 37:320-325, J Nucl Med 2001;42:752-754 bzw. J Nucl Med 1999; 40:331-338.). Glutaminsäure und Glutamin sind als ¹⁸F markierte Derivate nicht bekannt, wohingegen nicht-radioaktive fluorierte Glutamin- und Glutaminsäurederivate geläufig sind; so z. Bsp. jene, die an γ-Position (z. Bsp. (review): Amino Acids. (2003) Apr;24(3):245-61) oder an β-Position (z. Bsp. Tetrahedron Lett.; 30; 14; 1989; 1799-1802, J. Org. Chem.; 54; 2; 1989; 498-500, Tetrahedron: Asymmetry; 12; 9; 2001; 1303 - 1312) Fluor aufweisen.

Von Glutaminsäurederivaten, die an den chemischen Funktionalitäten Schutzgruppen und in β oder γ Position eine Abgangsgruppe aufweisen, ist in der Vergangenheit bereits berichtet worden. So wurde über Glutamat als Mesylat bzw. Bromid in γ-Position informiert, deren Säure- und Aminfunktionen mit Ester- bzw. Z-Schutzgruppen versehen waren (J. Chem. Soc. Perkin Trans. 1; 1986; 1323-1328) oder beispielsweise von γ-Chloro-Glutaminsäure ohne Schutzgruppen (Synthesis; (1973); 44-46). Über ähnliche Derivate, bei denen aber die Fluchtgruppe in β-Stellung positioniert ist, wurde ebenfalls verschiedentlich berichtet: z. Bsp. Chem. Pharm. Bull.; 17; 5; (1969); 879-885, J.Gen.Chem.USSR (Engl.Transl.); 38; (1968); 1645-1648; Tetrahedron Lett.; 27; 19; (1986); 2143-2144, Chem. Pharm. Bull.; EN; 17; 5; 1969; 873-878, Patent FR 1461184, Patent JP 13142.)

Die derzeitigen PET-Tracer, die für die Tumordiagnostik eingesetzt werden, haben einige unbestrittene Nachteile: So reichert sich FDG zwar bevorzugt in solchen Zellen mit erhöhtem Glukosestoffwechsel an, es gibt allerdings auch in anderen pathologischen und physiologischen Zuständen einen erhöhten Glukosestoffwechsel in den beteiligten Zellen und Geweben, z. Bsp. Infektionsherde oder Wundheilung (zusammengefasst in J. Nucl. Med. Technol. (2005), 33, 145-155). Es ist häufig immer noch schwierig zu entscheiden, ob eine mittels FDG-PET detektierte Läsion tatsächlich neoplastischen Ursprungs ist oder auf andere physiologische oder pathologische Zustände des Gewebes zurückzuführen ist. Insgesamt weist die Diagnosetätigkeit mittels FDG-PET in der Onkologie eine Sensitivität von 84% und eine Spezifität von 88% auf (Gambhir et al. "A tabulated summary of the FDG PET literature" J. Nucl. Med. 2001, 42, 1-93S). Tumore im Gehirn lassen sich beispielsweise durch die hohe Anreicherung von FDG in gesundem Hirngewebe nur sehr schwer darstellen.
Die bisher bekannten ¹⁸F markierten Aminosäurederivate sind in einigen Fällen gut geeignet, um Tumore im Gehirn zu detektieren ((review): Eur J Nucl Med Mol Imaging. 2002 May;29(5):681-90), allerdings können sie bei anderen Tumoren nicht mit den Bildgebungseigenschaften des "Goldstandards" [¹⁸F]2-FDG konkurrieren. Die metabolische Anreicherung und Retention der bislang F-18 markierten Aminosäuren in tumorösem Gewebe ist in der Regel niedriger als für FDG. Darüberhinaus, ist die Zugänglichkeit isomerenreiner F-18 markierter nichtaromatischer Aminosäuren chemisch höchst anspruchsvoll.
Ähnlich wie für Glukose wurde auch für Glutaminsäure und Glutamin ein erhöhter Metabolismus in proliferierenden Tumorzellen beschrieben (Medina, J Nutr. 1131:2539S-2542S, 2001; Souba, Ann Surg 218: 715-728, 1993). Die erhöhte Rate an Protein- und Nukleinsäuresynthesen sowie die Energiegewinnung per se werden als Gründe für einen verstärkten Glutaminkonsum von Tumorzellen angenommen. Die Synthese von entsprechenden C-11 und C-14 markierten, mit dem natürlichen Substrat also identischen Verbindungen, wurde in der Literatur bereits beschrieben (z. Bsp. Antoni, Enzyme Catalyzed Synthesis of L-[4-C-11]Aspartate and L-[5-C-11]Glutamate. J. Labelled Compd. Radiopharm. 44;(4) 2001: 287 - 294) und Buchanan, The biosynthesis of showdomycin: studies with stable isotopes and the determination of principal precursors. J. Chem. Soc. Chem. Commun.; EN; 22; 1984; 1515-1517). Erste Hinweise mit der C-11 markierten Verbindung deuten auf keine signifikante Tumorakkumulation hin.

Aufgabe der vorliegenden Erfindung ist es neue Verbindungen zu finden, die sich in [¹⁸F] markierter Form für die PET-basierte Diagnose eignen.

Die Aufgabe wird gelöst durch die erfindungsgemäße Bereitstellung von [¹⁸F] markierten L-Glutaminsäure- und [¹⁸F] markiertem L-Glutamin-Derivaten gemäß der allgemeinen Formel (I), einschließlich ihrer Diastereomere und Enantiomere: 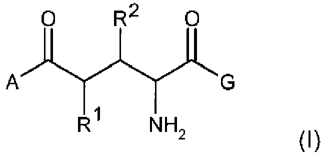 worin
A für
   a) Hydroxyl,
   b) verzweigtes oder unverzweigtes Hydroxy C₁-C₅ Alkoxy,
   c) N(C₁-C₅ Alkyl)₂,
   d) NH₂,
   e) N(H)-L,
   f) O-L, oder
   g) O-Z
   steht,
G für
   a) hydroxyl,
   b) verzweigtes oder unverzweigtes O-C₁-C₅ Alkyl,
   c) verzweigtes oder unverzweigtes O-C₂-C₅ Alkenyl,
   d) verzweigtes oder unverzweigtes O-C₁-C₅ Alkyl-(O-C₁-C₄ alkyl)ₙ-O-C₁-C₄ alkyl oder
   e) verzweigtes oder unverzweigtes O-C₂-C₅ Alkinyl
   steht,
R¹ und R² für
   a) Wasserstoff,
   b) verzweigtes oder unverzweigtes ¹⁸F C₁-C₅ Alkoxy,
   c) verzweigtes oder unverzweigtes ¹⁸F-C₁-C₅ Alkyl,
   d) verzweigtes oder unverzweigtes ¹⁸F-hydroxy-C₁-C₅ Alkyl,
   e) verzweigtes oder unverzweigtes ¹⁸F-C₂-C₅ Alkenyl, oder
   f) verzweigtes oder unverzweigtes ¹⁸F-C₂-C₅ Alkinyl,
   g) Hydroxyl
   h) verzweigtes oder unverzweigtes C₁-C₅ Alkyl oder
   i) verzweigtes oder unverzweigtes C₁-C₅ Alkoxy
   stehen, mit der Maßgabe, dass genau einer der Substituenten R¹ oder R² genau ein ¹⁸F Isotop beinhaltet und der jeweils andere Substituent kein ¹⁸F Isotop beinhaltet,
L für
   a) verzweigtes oder unverzweigtes C₁-C₅ Alkyl,
   b) verzweigtes oder unverzweigtes C₂-C₅ Alkenyl,
   c) verzweigtes oder unverzweigtes C₁-C₅ Alkyl-(O-C₁-C₄ alkyl)ₙ-O-C₁-C₄alkyl oder
   d) verzweigtes oder unverzweigtes C₂-C₅ Alkinyl
   steht, und
Z für ein Metallkationenequivalent steht, welches ein- oder zweiwertig sein kann, wobei zweiwertige Metalle zur ionischen Bindung mit zwei Resten der erfindungsgemäßen Strukturen führen können.
wobei n = 0, 1, 2 oder 3 ist, und wobei alle möglichen Diastereomere und Enantiomere Teil des vorliegenden Erfindungsgegenstandes sind.

Bevorzugte Verbindungen der Erfindung gemäß der Formel (I) zeichnen sich dadurch aus, dass
A für
a) Hydroxyl,
b) Methoxy,
c) Ethoxy,
d) Propoxy,
e) NMe₂,
f) NEt₂,
g) NH₂,
h) N(H)-L oder
i) O-L,
j) O-Z
steht.

Weitere bevorzugte Verbindungen der Erfindung gemäß der Formel (I) zeichnen sich dadurch aus, dass
A für
a) Hydroxyl,
b) Methoxy,
c) Ethoxy,
d) NMe₂,
e) NH₂, oder
f) N(H)-L
steht.

Besonders bevorzugte Verbindungen der Erfindung gemäß der Formel (I) zeichnen sich dadurch aus, dass
A für
a) Hydroxyl,
b) Methoxy oder
c) NH₂
steht.

Bevorzugte Verbindungen der Erfindung gemäß der Formel (I) zeichnen sich dadurch aus, dass
A für
OH
steht.

Bevorzugte Verbindungen der Erfindung gemäß der Formel (I) zeichnen sich dadurch aus, dass
A für
NH₂
steht.

Bevorzugte Verbindungen der Erfindung gemäß der Formel (I) zeichnen sich dadurch aus, dass
G für
a) Hydroxyl,
b) Methoxy,
c) Ethoxy,
d) Propoxy,
e) iso-Propoxy, oder
f) O-C₂H₄-OMe
steht.

Weitere bevorzugte Verbindungen der Erfindung gemäß der Formel (I) zeichnen sich dadurch aus, dass
G für
a) Hydroxyl,
b) Methoxy, oder
c) Ethoxy
steht.

Besonders bevorzugte Verbindungen der Erfindung gemäß der Formel (I) zeichnen sich dadurch aus, dass
G für
a) Hydroxyl oder
b) Methoxy
steht.

Bevorzugte Verbindungen der Erfindung gemäß der Formel (I) zeichnen sich dadurch aus, dass
R¹ und R² für
a) Wasserstoff,
b) ¹⁸F-methoxy,
c) ¹⁸F-ethoxy,
d) ¹⁸F-propoxy
e) ¹⁸F-methyl,
f) ¹⁸F-ethyl, oder
g) ¹⁸F-propyl
mit der Maßgabe, dass genau einer der Substituenten R¹ oder R² genau ein ¹⁸F-Isotop beinhaltet und der jeweils andere Substituent Wasserstoff ist,
steht.

Weitere bevorzugte Verbindungen der Erfindung gemäß der Formel (I) zeichnen sich dadurch aus, dass
R¹ und R² für
a) Wasserstoff,
b) ¹⁸F-methoxy,
c) ¹⁸F-methyl, oder
d) ¹⁸F-ethyl,
mit der Maßgabe, dass genau einer der Substituenten R¹ oder R² genau ein ¹⁸F Isotop beinhaltet und der jeweils andere Substituent Wasserstoff ist,
steht.

Besonders bevorzugte Verbindungen der Erfindung gemäß der Formel (I) zeichnen sich dadurch aus, dass
R¹ und R² für
a) Wasserstoff, oder
b) ¹⁸F-methyl
steht, mit der Maßgabe, dass genau einer der Substituenten R¹ oder R² genau ein ¹⁸F-Isotop beinhaltet und der jeweils andere Substituent Wasserstoff ist.

Bevorzugte Verbindungen der Erfindung gemäß der Formel (I) zeichnen sich dadurch aus, dass
L für
a) Methyl,
b) Ethyl,
c) Propyl,
d) iso-Propyl,
e) -C₂H₄-OMe, oder
f) -C₂H₄-O-C₂H₄-OMe
steht.

Besonders bevorzugte Verbindungen der Erfindung gemäß der Formel (I) zeichnen sich dadurch aus, dass
L für
a) Methyl oder
b) Ethyl
steht.

Ebenfalls bevorzugte Verbindungen der Erfindung gemäß der Formel (I) zeichnen sich dadurch aus, dass
Z für Alkali- und Erdalkaliionen sowie Nickel steht welches ein- oder zweiwertig sein kann, wobei zweiwertige Metalle zur ionischen Bindung mit zwei Resten der erfindungsgemäßen Strukturen führen können.
Bevorzugtes Z ist ausgewählt aus der Gruppe Na⁺, K⁺, Ca²⁺ und Mg²⁺.

Alle möglichen Diastereomere und Enantiomere der bevorzugten Verbindungen nach Formel (I) sind Teil des vorliegenden Erfindungsgegenstandes.

Weiterhin besonders bevorzugt ist jede einzelne der Verbindungen aus der folgenden Gruppe, wobei alle möglichen Diastereomere und Enantiomere Teil des vorliegenden Erfindungsgegenstandes sind:

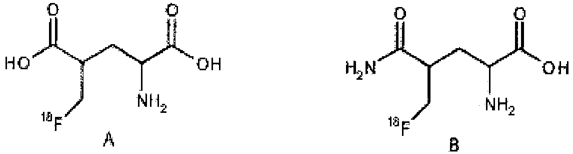

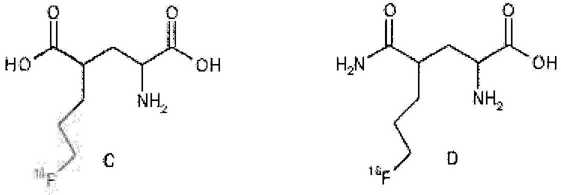

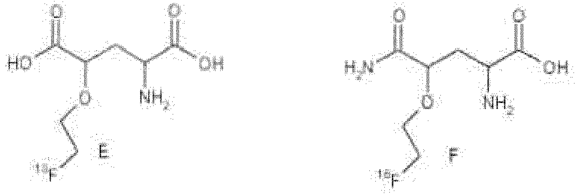

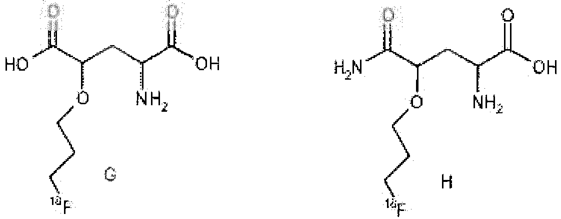

Das Verfahren zur Herstellung der erfindungsgemäßen Verbindungen der allgemeinen Formel (I) zeichnet sich dadurch aus, dass die Verbindung nach Formel (I), aus einer Vorläuferverbindung der Verbindung nach Formel (II) nach Einführung des ¹⁸F Isotops freigesetzt wird. In einem zweiten Aspekt bezieht sich somit die vorliegende Erfindung auf Verbindungen der Formel (II): 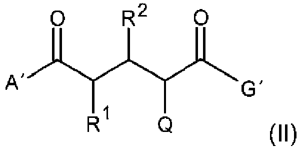
worin A' für
   a) Hydroxyl,
   b) verzweigtes oder unverzweigtes Hydroxy C₁-C₅ Alkoxy, c N(C₁-C₅ Alkyl)₂,
   d) NH₂,
   e) N(H)-U,
   f) N(H)-L' oder
   g) O-L'
   steht,
G' für
   a) hydroxyl,
   b) O-Z',
   c) verzweigtes oder unverzweigtes O-C₁-C₅ Alkyl,
   d) verzweigtes oder unverzweigtes O-C₂-C₅ Alkenyl,
   e) verzweigtes oder unverzweigtes O-C₁-C₅ Alkyl-(O-C₁-C₄ alkyl)ₙ-O-C₁-C₄ alkyl, oder
   f) verzweigtes oder unverzweigtes O-C₂-C₅ Alkinyl
   steht,
R¹ und R² für
   a Wasserstoff,
   b) verzweigtes oder unverzweigtes ¹⁸F C₁-C₅ Alkoxy,
   c) verzweigtes oder unverzweigtes ¹⁸F-C₁-C₅ Alkyl,
   d) verzweigtes oder unverzweigtes ¹⁸F- Hydroxy- C₁-C₅ alkyl,
   e) verzweigtes oder unverzweigtes ¹⁸F-C₂-C₅ Alkenyl, oder
   f) verzweigtes oder unverzweigtes ¹⁸F-C₂-C₅ Alkinyl,
   g) Hydroxyl
   h) verzweigtes oder unverzweigtes C₁-C₅ Alkyl, oder
   i) verzweigtes oder unverzweigtes C₁-C₅ Alkoxy
   steht, mit der Maßgabe, dass genau einer der Substituenten R¹ oder R² genau ein ¹⁸F Isotop beinhaltet und der jeweils andere Substituent kein ¹⁸F Isotop beinhaltet,
Q für
   a) N(H)-tert-Butoxycarbonyl,
   b) N(H)-Allyloxycarbonyl,
   c) N(H)-Benzyloxycarbonyl,
   d) N(H)-Ethoxycarbonyl,
   e) N(H)-Methoxycarbonyl,
   f) N(H)-Propoxycarbonyl,
   e) N(H)-2,2,2-Trichlorethoxycarbonyl,
   f) N(H)-1,1-Dimethylpropinyl,
   g) N(H)-1-Methyl-1-phenyl-ethoxycarbonyl,
   h) N(H)-1-Methyl-1-(4-biphenylyl)-ethoxycarbonyl,
   i) N(H)-cyclobutylcarbonyl,
   j) N(H)-1-Methylcyclobutylcarbonyl,
   k) N(H)-Vinylcarbonyl,
   l) N(H)-Allylcarbonyl,
   m) N(H)-Adamantylcarbonyl,
   n) N(H)-Diphenylmethylcarbonyl,
   o) N(H)-Cinnamylcarbonyl,
   p) N(H)-Formyl,
   q) N(H)-Benzoyl,
   r) N(H)-Trityl,
   s) N(H)-p-Methoxyphenyl-diphenylmethyl,
   t) N(H)-di-(p-Methoxyphenyl)-phenylmethyl,
   u) 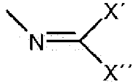 oder
   v) N-(tert-Butoxycarbonyl)₂,
   steht,
L' für
   a) verzweigtes oder unverzweigtes C₁-C₅ Alkyl,
   b) verzweigtes oder unverzweigtes C₂-C₅ Alkenyl,
   c) verzweigtes oder unverzweigtes C₁-C₅ Alkyl-(O-C₁-C₄alkyl),-O-C₁-C₄alkyl, oder
   d) verzweigtes oder unverzweigtes C₂-C₅ Alkinyl
   steht,
U für
   a) tert-Butoxycarbonyl,
   b) Allyloxycarbonyl,
   c) Benzyloxycarbonyl,
   d) Methoxycarbonyl,
   e) Propoxycarbonyl, oder
   d) Ethoxycarbonyl
   steht,
X' und X" unabhängig voneinander für
   a) verzweigtes oder unverzweigtes C₁-C₅ Alkyl,
   b) substituiertes oder unsubstituiertes Aryl,
   c) Aralkyl oder
   d) Heteroaryl
   steht,
   und
Z' für ein Metallkationenequivalent steht, weiches ein- oder zweiwertig sein kann, wobei zweiwertige Metalle zur ionischen Bindung mit zwei Resten der erfindungsgemäßen Strukturen führen können oder koordinative Bindungen mit Carboxyl- und Aminfunktion des Aminosäurederivates ausbilden können,
   Bevorzugtes Z' ist ausgewählt aus der Gruppe Na⁺, K⁺, Ca²⁺ und Mg²⁺ oder
   ist Ni²⁺.
wobei n = 0, 1, 2 oder 3 ist und alle möglichen Diastereomere und Enantiomere Teil des vorliegenden Erfindungsgegenstandes sind.

Bevorzugte Verbindungen der Erfindung gemäß der Formel (II) zeichnen sich dadurch aus, dass
A' für
a) Hydroxyl,
b) Methoxy,
c) Ethoxy,
d) tert-Butoxy,
e) NMe₂,
f) NEt₂,
g) NH₂,
h) N(H)-U,
i) N(H)-L' oder
j) O-L'
steht.

Weitere bevorzugte Verbindungen der Erfindung gemäß der Formel (II) zeichnen sich dadurch aus, dass
A' für
a) Hydroxyl,
b) Methoxy,
c) Ethoxy,
d) NMe₂,
e) N(H)-U
f) NH₂, oder
g) N(H)-L'
steht.

Besonders bevorzugte Verbindungen der Erfindung gemäß der Formel (II) zeichnen sich dadurch aus, dass
A' für
a) Hydroxyl,
b) Ethoxy,
b) Methoxy,
c) N(H)-U oder
d) NH₂
steht.

Besonders bevorzugte Verbindungen der Erfindung gemäß der Formel (II) zeichnen sich dadurch aus, dass
A' für
a) Hydroxyl,
b) verzweigtes oder unverzweigtes C₁-C₅ alkoxy,
c) -NH-tert-Butoxycarbonyl, oder
d) NH₂
steht.

Besonders bevorzugte Verbindungen der Erfindung gemäß der Formel (II) zeichnen sich dadurch aus, dass
A' für Ethoxy steht.

Besonders bevorzugte Verbindungen der Erfindung gemäß der Formel (II) zeichnen sich dadurch aus, dass
A' für NH₂ steht.

Bevorzugte Verbindungen der Erfindung gemäß der Formel (II) zeichnen sich dadurch aus, dass
G' für
a) Hydroxyl,
b) OZ',
c) Methoxy,
d) Ethoxy,
e) tert-Butoxy
f) iso-Propoxy, oder
g) O-C₂H₄-OMe
steht.

Weitere bevorzugte Verbindungen der Erfindung gemäß der Formel (II) zeichnen sich dadurch aus, dass
G' für
a) Hydroxyl,
b) OZ',
c) Methoxy, oder
d) Ethoxy
steht.

Besonders bevorzugte Verbindungen der Erfindung gemäß der Formel (II) zeichnen sich dadurch aus, dass
G' für
a) Hydroxyl,
b) OZ', oder
c) Methoxy
steht.

Besonders bevorzugte Verbindungen der Erfindung gemäß der Formel (II) zeichnen sich dadurch aus, dass
G' für Ethoxy steht.

Bevorzugte Verbindungen der Erfindung gemäß der Formel (II) zeichnen sich dadurch aus, dass
R¹ und R² für
a) Wasserstoff,
b) ¹⁸F-methoxy,
c) ¹⁸F-ethoxy,
d) ¹⁸F-propoxy,
e) ¹⁸F-methyl,
f) ¹⁸F-ethyl, oder
g) ¹⁸F-propyl
steht, mit der Maßgabe, dass genau einer der Substituenten R¹ oder R² genau ein ¹⁸F-Isotop beinhaltet und der jeweils andere Substituent Wasserstoff ist.

Weitere bevorzugte Verbindungen der Erfindung gemäß der Formel (II) zeichnen sich dadurch aus, dass
R¹ und R² für
a) Wasserstoff,
b) ¹⁸F-methoxy,
c) ¹⁸F-methyl, oder
d) ¹⁸F-ethyl,
steht, mit der Maßgabe, dass genau einer der Substituenten R¹ oder R² genau ein ¹⁸F-Isotop beinhaltet und der jeweils andere Substituent Wasserstoff ist.

Besonders bevorzugte Verbindungen der Erfindung gemäß der Formel (II) zeichnen sich dadurch aus, dass
R¹ und R² für
a) Wasserstoff, oder
b) ¹⁸F-methyl,
steht, mit der Maßgabe, dass genau einer der Substituenten R¹ oder R² genau ein ¹⁸F-Isotop beinhaltet und der jeweils andere Substituent Wasserstoff ist.

Bevorzugte Verbindungen der Erfindung gemäß der Formel (II) zeichnen sich dadurch aus, dass
Q für
a) N(H)-tert-Butoxycarbonyl,
b) N(H)-Benzyloxycarbonyl,
c) N-(tert-Butoxycarbonyl)₂, oder
d) 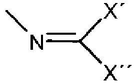
steht.

Weitere bevorzugte Verbindungen der Erfindung gemäß der Formel (II) zeichnen sich dadurch aus, dass
Q für
a) N(H)-tert-Butoxycarbonyl,
b) N-(tert-Butoxycarbonyl)₂, oder
c) 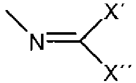
steht.

Besonders bevorzugte Verbindungen der Erfindung gemäß der Formel (II) zeichnen sich dadurch aus, dass
Q für
a) N(H)-tert-Butoxycarbonyl, oder
b) 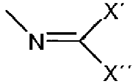
steht.

Besonders bevorzugte Verbindungen der Erfindung gemäß der Formel (II) zeichnen sich dadurch aus, dass
Q für N(H)-tert-Butoxycarbonyl steht.

Besonders bevorzugte Verbindungen der Erfindung gemäß der Formel (II) zeichnen sich dadurch aus, dass Q für 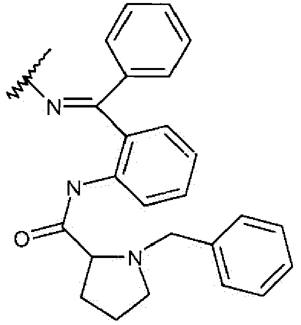 steht.

Bevorzugte Verbindungen der Erfindung gemäß der Formel (II) zeichnen sich dadurch aus, dass
L' für
a) Methyl,
b) Ethyl,
c) Propyl,
d) iso-Propyl,
e) -C₂H₄-OMe, oder
f) -C₂H₄-O-C₂H₄-OMe
steht.

Weitere bevorzugte Verbindungen der Erfindung gemäß der Formel (II) zeichnen sich dadurch aus, dass
L' für
a) Methyl, oder
b) Ethyl
steht.

Bevorzugte Verbindungen der Erfindung gemäß der Formel (II) zeichnen sich dadurch aus, dass
U für
a) tert-Butoxycarbonyl,
b) Allyloxycarbonyl, oder
c) Ethoxycarbonyl
steht.

Weitere bevorzugte Verbindungen der Erfindung gemäß der Formel (II) zeichnen sich dadurch aus, dass
U für
a) tert-Butoxycarbonyl, oder
b) Ethoxycarbonyl
steht.

Besonders bevorzugte Verbindungen der Erfindung gemäß der Formel (II) zeichnen sich dadurch aus, dass
U für
tert-Butoxycarbonyl
steht.

Bevorzugte Verbindungen der Erfindung gemäß der Formel (II) zeichnen sich dadurch aus, dass
X' und X" unabhängig voneinander für
a) verzweigtes oder unverzweigtes C₁-C₅ Alkyl,
b) substituiertes oder unsubstituiertes Aryl oder
c) Aralkyl
steht.

Weitere bevorzugte Verbindungen der Erfindung gemäß der Formel (II) zeichnen sich dadurch aus, dass
X' und X" unabhängig voneinander für
a) verzweigtes oder unverzweigtes C₁-C₅ Alkyl, oder
b) substituiertes oder unsubstituiertes Aryl
steht.

Besonders bevorzugte Verbindungen der Erfindung gemäß der Formel (II) zeichnen sich dadurch aus, dass
X' und X" für Phenyl bzw. für in 2-Position substituiertes Phenyl
steht.

Z' für ein Metallkationenequivalent steht, welches ein- oder zweiwertig sein kann, wobei zweiwertige Metalle zur ionischen Bindung mit zwei Resten der erfindungsgemäßen Strukturen führen können oder koordinative Bindungen mit Carboxyl- und Aminfunktion des Aminosäurederivates ausbilden können,

Bevorzugte Verbindungen der Erfindung gemäß der Formel (II) zeichnen sich dadurch aus, dass
Z' für Ni²⁺ Ion steht oder
Z' ausgewählt ist aus der Gruppe: Na⁺, K⁺, Ca²⁺ und Mg²⁺.

Wobei alle möglichen Diastereomere und Enantiomere der bevorzugten Verbindungen der Erfindung gemäß Formel (II) Teil des vorliegenden Erfindungsgegenstandes sind.

Weiterhin besonders bevorzugt ist jede einzelne der Verbindungen aus der folgenden Gruppe wobei alle möglichen Diastereomere und Enantiomere Teil des vorliegenden Erfindungsgegenstandes sind:

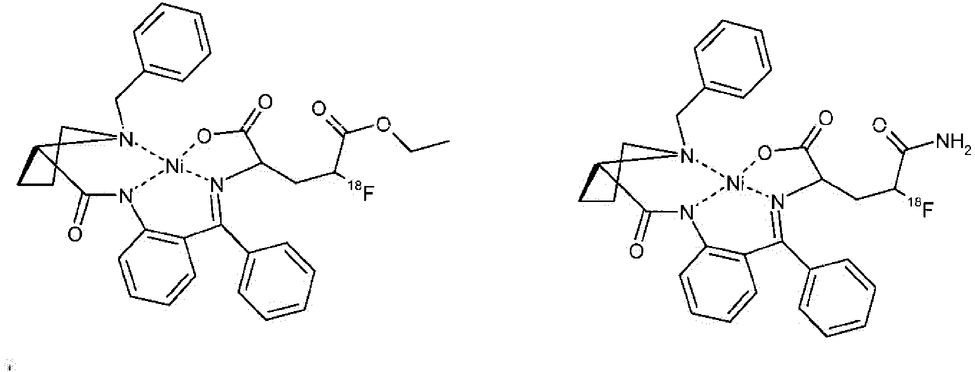

Das Verfahren zur Herstellung der erfindungsgemäßen Verbindungen der allgemeinen Formel (II) zeichnet sich dadurch aus, dass die Mehrzahl der Verbindung nach Formel (II), aus einer Vorläuferverbindung der Verbindung der Formel (III) nach Einführung des ¹⁸F Isotops entstehen kann.

In einem dritten Aspekt bezieht sich die vorliegende Erfindung auf Verbindungen der Formel (III): 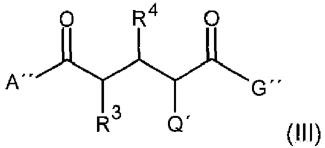 worin
A" für
   a) Hydroxyl,
   b) verzweigtes oder unverzweigtes Hydroxy C₁-C₅ Alkoxy,
   c) N(C₄-C₅ Alkyl)₂,
   d) NH₂,
   e) N(H)-U',
   f) N(H)-L" oder
   g) O-L"
   steht,
G" für
   a) hydroxyl,
   b) O-Z",
   c) verzweigtes oder unverzweigtes O-C₁-C₅ Alkyl,
   d) verzweigtes oder unverzweigtes O-C₂-C₅ Alkenyl,
   e) verzweigtes oder unverzweigtes O-C₁-C₅ Alkyl-(O-C₁-C₄alkyl)ₙ-O-C₁-C₄alkyl,
   f) verzweigtes oder unverzweigtes O-C₂-C₅ Alkinyl, oder
   g) triphenylmethoxy
   steht,
R³ und R⁴ für
   a) Wasserstoff
   b) verzweigtes oder unverzweigtes E-C₁-C₅ Alkoxy
   c) verzweigtes oder unverzweigtes E-C₁-C₅ Alkyl,
   d) verzweigtes oder unverzweigtes E- Hydroxy-C₁-C₅ -alkyl,
   e) verzweigtes oder unverzweigtes E-C₂-C₅ Alkenyl,
   f) verzweigtes oder unverzweigtes E-C₂-C₅ Alkinyl,
   g) Hydroxyl,
   h) verzweigtes oder unverzweigtes C₁-C₅ Alkyl, oder
   i) verzweigtes oder unverzweigtes C₁-C₅ Alkoxy
   steht, mit der Maßgabe, dass genau einer der Substituenten R³ oder R⁴ ein E beinhaltet und der jeweils andere Substituent kein E beinhaltet,
E für
   a) Chloro,
   b) Bromo,
   c) Mesyloxy,
   d) Trifluormesyloxy,
   e) Nonafluorbutyloxy, oder
   f) Tosyloxy
   steht,
Q' für
   a) N(H)-tert-Butoxycarbonyl,
   b) N(H)-Allyloxycarbonyl,
   c) N(H)-Benzyloxycarbonyl,
   d) N(H)-Ethoxycarbonyl
   e) N(H)-Methoxycarbonyl
   f) N(H)-Propoxycarbonyl
   g) N(H)-2,2,2-Trichlorethoxycarbonyl
   h) N(H)-1,1-Dimethylpropinyl
   i) N(H)-1-Methyl-1-phenyl-ethoxycarbonyl
   j) N(H)-1-Methyl-1-(4-biphenylyl)-ethoxycarbonyl
   k) N(H)-Cyclobutylcarbonyl
   l) N(H)-1-Methylcyclobutylcarbonyl
   m) N(H)-Vinylcarbonyl
   n) N(H)-Allylcarbonyl
   o) N(H)-Adamantylcarbonyl
   p) N(H)-Diphenylmethylcarbonyl
   q) N(H)-Cinnamylcarbonyl
   r) N(H)-Formyl
   s) N(H)-Benzoyl
   t) N(H)-Trityl
   u) N(H)-p-Methoxyphenyl-diphenylmethyl
   v) N(H)-di-(p-Methoxyphenyl)-phenylmethyl, oder
   w) 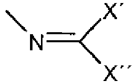
   x) N-(tert-Butoxycarbonyl)₂,
   steht,
L" für
   a) verzweigtes oder unverzweigtes C₁-C₅ Alkyl,
   b) verzweigtes oder unverzweigtes C₂-C₅ Alkenyl,
   c) verzweigtes oder unverzweigtes C₁-C₅ Alkyl-(O-C₁-C₄alkyl)ₙ-O-C₁-C₄ alkyl oder
   d) verzweigtes oder unverzweigtes C₂-C₅ Alkinyl
   steht,
U' für
   a) tert-Butoxycarbonyl,
   b) Allyloxycarbonyl,
   c) Benzyloxycarbonyl, oder
   d) Ethoxycarbonyl
   steht,
X' and X" unabhängig voneinander für
   a) verzweigtes oder unverzweigtes C₁-C₅ Alkyl,
   b) substituiertes oder unsubstituiertes Aryl
   c) Aralkyl oder
   d) Heteroaryl
   steht, und
Z" für Metallkationenequivalent steht, welches ein- oder zweiwertig sein kann, wobei zweiwertige Metalle zur ionischen Bindung mit zwei Resten der erfindungsgemäßen Strukturen führen können oder koordinative Bindungen mit Carboxyl- und Aminfunktion des Aminosäurederivates ausbilden können,
   Bevorzugtes Z" ist ausgewählt aus der Gruppe Na⁺, K⁺, Ca²⁺ und Mg²⁺ oder
   ist Ni²⁺ ,
wobei n = 0, 1 oder 2 ist und alle möglichen Diastereomere und Enantiomere Teil des vorliegenden Erfindungsgegenstandes sind.

Bevorzugte Verbindungen der Erfindung gemäß der Formel (III) zeichnen sich dadurch aus, dass
A" für
a) Hydroxyl,
b) Methoxy,
c) Ethoxy,
d) tert-Butoxy,
e) NMe₂,
f) NEt₂,
g) NH₂,
h) N(H)-U',
i) N(H)-L" oder
j) O-L"
steht.

Weiter bevorzugte Verbindungen der Erfindung gemäß der Formel (III) zeichnen sich dadurch aus, dass
A" für
a) Hydroxyl,
b) Methoxy,
c) Ethoxy,
d) NMe₂,
e) N(H)-U',
f) NH₂, oder
g) N(H)-L"
steht.

Besonders bevorzugte Verbindungen der Erfindung gemäß der Formel (III) zeichnen sich dadurch aus, dass
A" für
a) Hydroxyl,
b) Ethoxy,
b) Methoxy,
c) N(H)-U, oder
d) NH₂
steht.

Bevorzugte Verbindungen der Erfindung gemäß der Formel (III) zeichnen sich dadurch aus, dass
G" für
a) Hydroxyl,
b) OZ",
c) Methoxy,
d) Ethoxy,
e) tert-Butoxy,
f) iso-Propoxy, oder
g) O-C₂H₄-OMe
steht.

Weiler bevorzugte Verbindungen der Erfindung gemäß der Formel (III) zeichnen sich dadurch aus, dass
G" für
a) Hydroxyl,
b) OZ",
c) Methoxy, oder
d) Ethoxy
steht.

Besonders bevorzugte Verbindungen der Erfindung gemäß der Formel (III) zeichnen sich dadurch aus, dass
G" für
a) Hydroxyl,
b) OZ", oder
c) Methoxy
steht.

Bevorzugte Verbindungen der Erfindung gemäß der Formel (III) zeichnen sich dadurch aus, dass
R³ und R⁴ für
a) Wasserstoff,
b) E-methoxy,
c) E-ethoxy,
d) E-propoxy,
e) E-methyl,
f) E-ethyl, oder
g) E-propyl
steht, mit der Maßgabe, dass genau einer der Substituenten R³ oder R⁴ ein E beinhaltet und der jeweils andere Substituent Wasserstoff ist.

Weiter bevorzugte Verbindungen der Erfindung gemäß der Formel (III) zeichnen sich dadurch aus, dass
R³ und R⁴ für
a) Wasserstoff,
b) E-methoxy,
c) E-methyl, oder
d) E-ethyl
steht, mit der Maßgabe, dass genau einer der Substituenten R³ oder R⁴ ein E beinhaltet und der jeweils andere Substituent Wasserstoff ist.

Besonders bevorzugte Verbindungen der Erfindung gemäß der Formel (III) zeichnen sich dadurch aus, dass
R³ und R⁴ für
a) Wasserstoff oder
b) E-methyl
steht, mit der Maßgabe, dass genau einer der Substituenten R³ oder R⁴ ein E beinhaltet und der jeweils andere Substituent Wasserstoff ist.

Bevorzugte Verbindungen der Erfindung gemäß der Formel (III) zeichnen sich dadurch aus, dass
E für
a) Chloro,
b) Bromo,
c) Mesyloxy,
d) Trifluormesyloxy oder
e) Tosyloxy
steht.

Weiter bevorzugte Verbindungen der Erfindung gemäß der Formel (III) zeichnen sich dadurch aus, dass
E für
a) Chloro,
b) Bromo,
c) Mesyloxy,
d) Trifluormesyloxy oder
e) Tosyloxy
steht.

Besonders bevorzugte Verbindungen der Erfindung gemäß der Formel (III) zeichnen sich dadurch aus, dass
E für
a) Bromo, oder
b) Mesyloxy
steht.

Bevorzugte Verbindungen der Erfindung gemäß der Formel (III) zeichnen sich dadurch aus, dass
Q' für
a) N(H)-tert-Butoxycarbonyl,
b) N(H)-Benzyloxycarbonyl, oder
c) 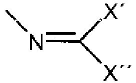
steht.

Weiter bevorzugte Verbindungen der Erfindung gemäß der Formel (III) zeichnen sich dadurch aus, dass
Q' für
a) N(H)-tert-Butoxycarbonyl, oder
b) 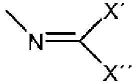
steht.

Bevorzugte Verbindungen der Erfindung gemäß der Formel (III) zeichnen sich dadurch aus, dass
L" für
a) Methyl,
b) Ethyl,
c) Propyl,
d) iso-Propyl,
e) -C₂H₄-OMe, oder
f) -C₂H₄-O-C₂H₄-OMe
steht.

Weiter bevorzugte Verbindungen der Erfindung gemäß der Formel (III) zeichnen sich dadurch aus, dass
L" für
a) Methyl, oder
b) Ethyl
steht.

Bevorzugte Verbindungen der Erfindung gemäß der Formel (III) zeichnen sich dadurch aus, dass
U' für
a) tert-Butoxycarbonyl,
b) Allyloxycarbonyl, oder
c) Ethoxycarbonyl
steht.

Weiter bevorzugte Verbindungen der Erfindung gemäß der Formel (III) zeichnen sich dadurch aus, dass
U' für
a) tert-Butoxycarbonyl, oder
b) Benzyloxycarbonyl
steht.

Besonders bevorzugte Verbindungen der Erfindung gemäß der Formel (III) zeichnen sich dadurch aus, dass
U' für
tert-Butoxycarbonyl
steht.

Bevorzugte Verbindungen der Erfindung gemäß der Formel (III) zeichnen sich dadurch aus, dass
X' and X" unabhängig voneinander für
a) verzweigtes oder unverzweigtes C₁-C₅ Alkyl,
b) substituiertes oder unsubstituiertes Aryl, oder
c) Aralkyl
steht.

Weiter bevorzugte Verbindungen der Erfindung gemäß der Formel (III) zeichnen sich dadurch aus, dass
X' und X" unabhängig voneinander für
a) verzweigtes oder unverzweigtes C₁-C₅ Alkyl, und
b) substituiertes oder unsubstituiertes Aryl
steht.

Besonders bevorzugte Verbindungen der Erfindung gemäß der Formel (III) zeichnen sich dadurch aus, dass
X' und X" für Phenyl bzw. für in 2-Position substituiertes Phenyl steht.

Bevorzugte Verbindungen der Erfindung gemäß der Formel (III) zeichnen sich dadurch aus, dass
Z" für Ni²⁺ steht.

Wobei alle möglichen Diastereomere und Enantiomere der bevorzugten Verbindungen gemäß Formel (III) Teil des vorliegenden Erfindungsgegenstandes sind.

Der Begriff "Aryl", so wie er hier selbst stehend oder als Teil einer anderen Gruppe angewendet wird, bezieht sich auf mono-zyklische oder zweifach-zyklische aromatische Gruppen, die sechs bis zwölf Kohlenstoffatome im Ring beinhalten können, wie z. Bsp. Phenyl oder Naphtyl, und in Position 2 beliebig substituiert sein können.
Die Arylgruppen können an jeder geeigneten Stelle, die zu einer stabilen Verbindung führt, substituiert sein durch einen oder mehrere Reste aus der Gruppe: Hydroxy, Halogen, C₁-C₅-Alkyl, C₁-C₅-Alkoxy, Cyano, CF₃, Nitro.
Als Substituenten seien Methoxy-, Ethoxy-, Propoxy-,iso-Propoxy, Hydroxy, Fluor-, Chlor-, Brom-, Methyl-, Ethyl-, Propyl-, iso-Propyl oder Trifluormethylgruppen genannt.

Unter Halogen ist jeweils Fluor, Chlor, Brom oder Iod zu verstehen.

Der Begriff "Alkyl", so wie er hier selbst stehend oder als Teil einer anderen Gruppe angewendet wird, bezieht sich auf C₁-C₆-Alkylgruppen und können geradkettig oder verzweigt sein und für eine Methyl-, Ethyl-, n-Propyl-, iso-Propyl-, n-Butyl, iso-Butyl, tert.-Butyl- oder n-Pentyl-, 2,2-Dimethylpropyl-, 2-Methylbutyl- oder 3-Methylbutylgruppe stehen. Eine Methyl- oder Ethylgruppe ist bevorzugt. Unter Alkyl ist jeweils ein geradkettiger oder verzweigter Alkylrest, wie beispielsweise Methyl, Ethyl, Propyl, Isopropyl, Butyl, Isobutyl, sek-Butyl, tert-Butyl, Pentyl, Isopentyl oder Hexyl, Heptyl, Octyl, Nonyl, Decyl, Undecyl, Dodecyl zu verstehen.

Die Alkenyl-Substituenten sind jeweils geradkettig oder verzweigt, wobei beispielsweise folgenden Reste gemeint sind: Vinyl, Propen-1-yl, Propen-2-yl, But-1-en-1-yl, But-1-en-2-yl, But-2-en-1-yl, But-2-en-2-yl, 2-Methyl-prop-2-en-1-yl, 2-Methyl-prop-1-en-1-yl, But-1-en-3-yl, Ethinyl, Prop-1-in-1-yl, But-1-in-1-yl, But-2-in-1-yl, But-3-en-1-yl, Allyl.

Die Alkinylgruppen können geradkettig oder verzweigt sejn und beispielsweise C≡C, -CH₂-C≡CH, -C≡C-CH₃, -CH(CH₃)-C≡CH, -C≡C-CH₂(CH₃), -C(CH₃)₂-C≡CH, -C≡C-CH(CH₃)₂-,-CH(CH₃)-C≡C-CH₃,, -CH₂-C≡C- CH₂(CH₃) bedeuten.

Die C₁-C₅-Alkoxygruppen können geradkettig oder verzweigt sein und für eine Methoxy-, Ethoxy-, n-Propoxy-, iso-Propoxy-, n-Butoxy, iso-Butoxy, tert.-Butoxy- oder n-Pentoxy-, 2,2-Dimethylpropoxy-, 2-Methylbutoxy- oder 3-Methylbutoxygruppe stehen. Eine Methoxy- oder Ethoxygruppe ist bevorzugt.

Der Heteroarylrest umfaßt jeweils 5 - 16 Ringatome und kann anstelle eines Kohlenstoffatoms ein- oder mehrere, gleiche oder verschiedene Heteroatome, wie Sauerstoff, Stickstoff oder Schwefel im Ring enthalten, und kann mono-, bi- oder tricyclisch sein, und kann zusätzlich jeweils benzokondensiert sein.

### Beispielsweise seien genannt:

Thienyl, Furanyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, etc.
und
Benzoderivate davon, wie z. B.
Benzofuranyl, Benzothienyl, Benzothiazol, Benzoxazolyl, Benzimidazolyl, Indazolyl, Indolyl, Isoindolyl, etc.;
oder
Pyridyl, Pyridazinyl, Pyrimidinyl, Pyrazinyl, Triazinyl, etc. und Benzoderivate davon, wie z. B.
Chinolyl, Isochinolyl, etc.;
oder
Azocinyl, Indolizinyl, Purinyl, etc. und Benzoderivate davon; oder Chinolinyl, Isochinolinyl, Cinnolinyl, Phthalazinyl, Chinazolinyl, Chinoxalinyl, Naphthyridinyl, Pteridinyl, Carbazolyl, Acridinyl, Phenazinyl, Phenothiazinyl, Phenoxazinyl, Xanthenyl, Oxepinyl.

Die Erfindung umfasst auch den Ni²⁺ Komplex enthaltend die Schiff'sche Base einer Verbindung nach Formel (I) und der Verbindung (S)-2-[N-(N-benzylprolyl)amino]benzophenon als solchen und seine Verwendung zur Herstellung einer Verbindung nach Formel (I).

Weiter umfasst die Erfindung auch den Ni²⁺ Komplex enthaltend die Schiff'sche Base einer Vorläuferverbindung der Verbindung nach Formel (I) und der Verbindung (S)-2-[N-(N-benzylprolyl)amino]benzophenon als solchen und seine Verwendung zur Herstellung einer Verbindung nach Formel (I).

Erfindungsgemäße Verbindungen lassen sich beispielsweise wie in Schema 1 dargestellt, durch metallkatalysierte asymmetrische Synthese mit Hilfe eines Ni²⁺-Komplexes einer Glycin enthaltenden Schiff'schen Base und (*S*)-2-[*N*-(*N*-benzylprolyl)amino]benzophenon (BPB) (**1**)³ sowie Ethyl-α-Bromoacrylsäureethylester oder α-Bromoacrylamid (**2**), herstellen .

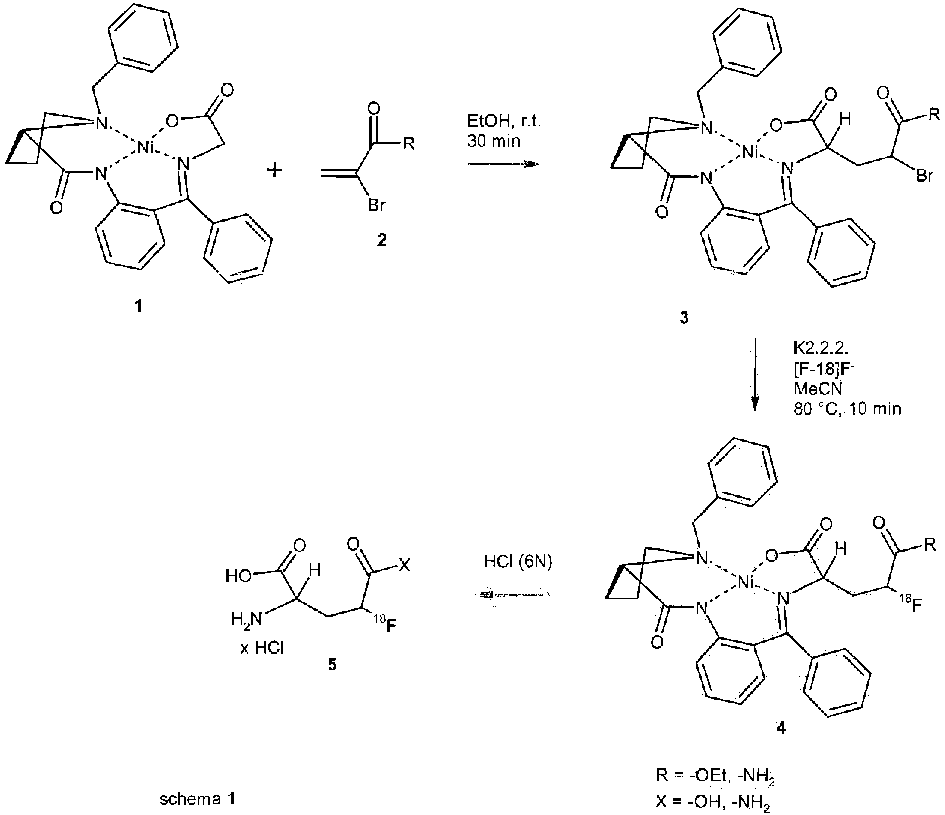

Dabei kann die C-C-Verknüpfung sowohl in protischen als auch aprotischen Lösungsmitteln erfolgen. Die Reaktion kann unter milden Bedingungen, wie zum Beispiel Raumtemperatur, oder erhöhten Temperaturen durchgeführt werden.

Dabei ist der Zusatz einer Base hilfreich. So kann zum Beispiel Diisopropylamin, Diisopropylethylamin, Triethylamin oder ähnliches verwendet werden. Die Reaktionsmischung wird günstiger Weise für 1 - 3 h gerührt und anschließend erneut mit Ethyl-α-bromoacrylat oder α-Bromoacrylamid versetzt. Die Reaktion kann mittels DC verfolgt werden. Hierzu eignen sich unter anderem Kieselgel/ Essigester/Chloroform-Systeme. Nach der Zugabe von Ethyl-α-Bromoacrylat oder α-Bromoacrylamid und mindestens 1 stündigem Rühren, ist die Reaktion zu einem erheblichen Teil abgeschlossen. Die Reaktionsmischung wird dann neutralisiert durch die Zugabe von organischer oder mineralischer Säure (oder einer Kombination aus beidem), zum Beispiel Essigsäure, Ameisensäure, Trifluoressigsäure, Trichloressigsäure, Propionsäure, Salzsäure, Schwefelsäure, Perchlorsäure, Phosphorsäure etc. Der 4-Bromo-glutaminsäure-ethylester-Ni-Komplex kann extraktiv aus dem Reaktionsgemisch separiert werden. Dafür eignen sich zum Beispiel organische halogenierte oder nicht-halogenierte Lösungsmittel, wie z. Bsp. Chloroform, Methylenchlorid, Dialkylether, Essigester, Alkane, etc. Nach Trocknung der organischen Phase mittels Trockenmittel (z. Bsp. Natriumsulfat, Calziumsulfat oder ähnlichem) wird im Vakuum bis zur Trockene eingeengt.

Durch präparative DC (z. Bsp. an Kieselgel mit einem Laufmittelgemisch aus AcOEt/ CHCl₃, 1 : 1) kann eine Mischung stereoisomerer Komplexe wie **3** (S,S,S) und **3** (S,R,S/R) festgestellt werden. Komplex **3** (S,S,R) kann zum Beispiel in vorbenanntem Trennsystem mit einen *R*_{f}-Wert von 0.49 separiert werden. Die Komplexe **3** können z. Bsp. mit MeOH, EtOH etc. vom Kieselgel eluiert und anschließend durch Säulenchromatographie, z. Bsp. an Sephadex mit EtOH/ C₆H₆ Mischungen gereinigt werden.

Der erhaltene Komplex kann dann für weitere Substitutionsreaktionen an dem mit Brom substituierten Kohlenstoffatom eingesetzt werden.

Die erfindungsgemäße Vorläuferverbindung **3** kann durch nukleophile Substitution mit [¹⁸F]F⁻ in die entsprechende fluorierte Vorläuferverbindung **4** überführt werden. Dazu können die Komplexe **3** in Gegenwart einer Base wie zum Beispiel NBu₄OH, (NBu₄)₂CO₃, K₂CO₃ etc. mit der entsprechenden Fluoridlösung umgesetzt werden. Die Reaktion läuft bevorzugt bei erhöhten Temperaturen ab. Der Zusatz von Kronenethern, wie z. Bsp. Kryptofix (K2.2.2) kann die Reaktion positiv beinflussen, besonders in Kombination mit K₂CO₃ als katalysierende Base.

Die fluorierten Verbindungen 4-Fluoroglutaminsäure oder 4-Fluoroglutamin können durch Behandlung mit Säuren, wie z. Bsp. Salzsäure, Phosphorsäure, Perchlorsäure, Schwefelsäure etc. aus den entsprechenden Komplexen **3** freigesetzt werden. Dabei kommt es sowohl zur Abspaltung des Aminosäurederivates als auch zur Spaltung des Esters in 5-Position bei der Verwendung von 4-Fluoroglutaminsäure-5-methylester.

4-Fluoroglutaminsäure kann über eine Kartusche (z. Bsp. QMA (Waters), LiChrolut (VWR/ Merck) or WHAT6803-2005 SPE COLSAX (VWR/ Merck)) vorgereinigt werden. Die Abtrennung von Verunreinigungen (Komplexverbindung **3, 4,** 4-Bromoglutaminsäure etc.) kann mittels HPLC erfolgen. Geeignete HPLC-Systeme können z. Bsp. sein: Säule: aminogruppentragendes Kieselgel (z. Bsp. Zorbax-NH₂); Eluent: 20 mM NaH₂PO₄ in Wasser, Fluß: 4 ml/ min Die gereinigte Verbindung **5** kann durch geeignetes Entfernen der HPLC-Lösungsmittel konzentriert und für eine Verwendung im Sinne dieser Erfindung eingesetzt werden. Die Entfernung der HPLC-Lösungsmittel kann auf unterschiedliche Weise erfolgen. Geeignet ist ein Einengen am Rotationsverdampfer unter vermindertem Druck, eine Aufheizung der Probe im Heizblock unter Stickstoffstrom etc. oder die Aufbringung auf eine Konzentratorkartusche (z. Bsp. C-18 SepPack etc.), gefolgt von einer Elution mit wenig EtOH/ wässriger Kochsalzlösung oder ähnlichem mit anschließender weiterer Konzentration durch vorgenannte Methoden.

Verbindungen, bei denen das [F-18]-Isotop ebenfalls in 4-Position des Glutaminsäuregerüstes positioniert ist (**5**), lassen sich auch wie in Schema 2 dargestellt, herstellen. So gelingt beispielshalber die saure Abspaltung der Schutzgruppen der Verbindung **6,** um die Verbindung 4-Fluoromethyl-glutaminsäure (**5**) zu erhalten.

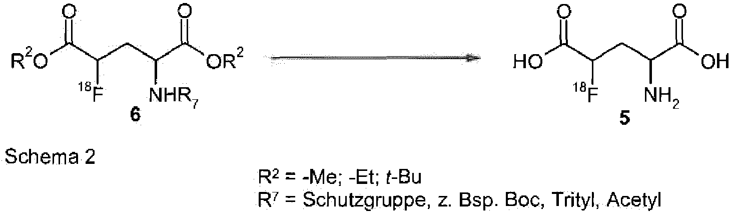

Dabei können verschiedene organische (z. Bsp. Trifluoressigsäure), vor allem aber anorganische Säuren wie z. Bsp. Bromwasserstoffsäure, Salzsäure, Schwefelsäure, Perchlorsäure oder Phosphorsäure zum Einsatz kommen. Die Reinigung der Verbindung 5 nach Formel (I) ist per HPLC möglich, wobei verschiedene Reinigungsschritte prinzipiell vorgeschaltet und nachgeschaltet sein können, wie z. Bsp. die Reinigung über eine RP-C18 Kartusche oder andere Trennmaterialien.

Die radiochemische Fluorierung von Tosylat **7,** dessen Synthese analog der in der Literatur beschriebenen Methode (X. Zhang Tetrahedron Lett. 2001, 42, 5335-5338.) aus **8** (N. Sharma et al. Tetrahedron Lett. 2004, 45, 1403-1406.) erfolgte, zum [F-18]-markierten Glutaminsäurederivat **6** ist nach den dem Fachmann bekannten Methoden durchführbar (s. Schema 3).

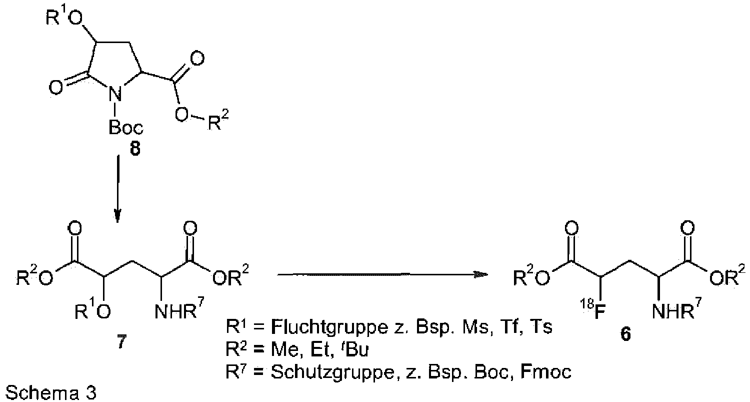

Dabei kann Verbindung 7 in Gegenwart einer Base wie zum Beispiel Tetra-alkyl ammonium und Tetra-alkyl-phosphonium-carbonat und Kaliumcarbonat etc. mit der entsprechenden [F-18]-Fluoridlösung umgesetzt werden. Die Reaktion läuft bevorzugt bei erhöhten Temperaturen ab. Der Zusatz von Kronenethern, wie z. Bsp. Kryptofix (K2.2.2) kann die Reaktion positiv beeinflussen, besonders in Kombination mit K₂CO₃ als katalysierende Base. Mögliche Lösungsmittel sind bevorzugt aprotisch, aber auch protische Lösungsmittel oder aber aprotische Lösungsmittelzusätze, wie z. Bsp. Wasser, können zur Anwendung kommen. Üblicherweise werden für die radiochemische Fluorierung mit [F-18]-Fluoridanionen Acetonitril, Dimethylsulfoxid oder Dimethylformamid als optimale Lösungsmittel angewandt. Erfindungsgemäße Verbindungen wie **6** können per HPLC und/ oder Kartuschen gereinigt werden, wobei verschiedene Reinigungsschritte prinzipiell vorgeschaltet und nachgeschaltet sein können, wie z. Bsp. die Reinigung über eine RP-C18 Kartusche oder andere Trennmaterialien.

Die Darstellung der F-19 Referenzverbindungen **10** und **11** gelingt wie in Schema 4 dargestellt.

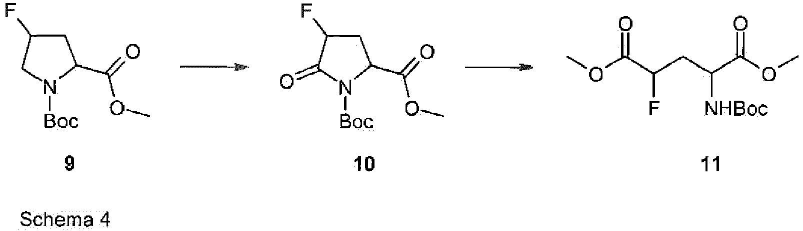

So kann beispielsweise **10** durch Oxidation des Fluorprolinderivats **9** hergestellt werden. Die offenkettige Referenzsubstanz **11** kann durch Ringöffnung von **10** erhalten werden.

Weiterhin können Verbindungen wie **5** auch direkt aus der zyklischen Verbindung **8** (Schema 3) hergestellt werden unter Verwendung der beschriebenen Fluorierungsbedingungen. Die Abspaltung der Schutzgruppen beziehungsweise die Ringöffnung kann analog den Bedingungen zur sauren Schutzgruppenabspaltung (Schema 2) durchgeführt werden. Dafür können verschiedene organische (z. Bsp. Trifluoressigsäure), vor allem aber anorganische Säuren wie z. Bsp. Bromwasserstoffsäure, Salzsäure, Schwefelsäure, Perchlorsäure oder Phosphorsäure zum Einsatz kommen. Weiterhin ist auch eine basische Ringöffnung möglich mit Lithiumhydroxid, Natriumhydroxid, Kaliumhydroxid etc. (S. Baker et al. Tetrahedron Lett. 1998, 39, 2815-2818.).

Verbindungen, bei denen das [F-18]-Isotop in β-Stellung positioniert ist, wie z. Bsp. bei 3-Fluoro-glutaminsäure (**12**), lassen sich wie in Schema 5 dargestellt herstellen. So gelingt beispielshalber die saure Abspaltung der Schutzgruppen der Verbindung **13,** um die Verbindung 3-Fluoro-glutaminsäure (**12**) zu erhalten.

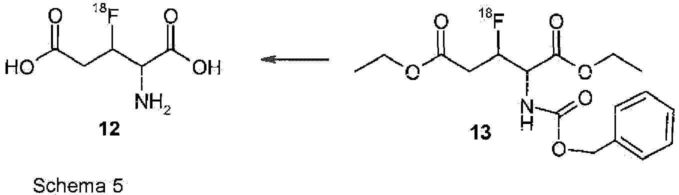

Dabei können verschiedene organische, vor allem aber anorganische Säuren wie z. Bsp. Bromwasserstoffsäure, Salzsäure, Schwefelsäure, Perchlorsäure oder Phosphorsäure zum Einsatz kommen. Eine Abspaltung der Schutzgruppen unter stark basischen Bedingungen mit z. Bsp. Natriumhydroxydlösung oder Kaliumhydroxydlösung ist weniger günstig, prinzipiell aber auch anwendbar und durchführbar. Die Reinigung der Verbindung **12** nach Formel (I) ist per HPLC möglich, wobei verschiedene Reinigungsschritte prinzipiell vorgeschaltet und nachgeschaltet sein können, wie z. Bsp. die Reinigung über eine RP-C18 Kartusche oder andere Trennmaterialien.

Die radiochemische Fluorierung von Tosylat **14,** dessen Synthese in der Literatur beschrieben ist (Chem. Pharm. Bull., 17, 5, (1969), 879-885), zum [F-18]-markierten Glutaminsäurederivat **13** ist nach den dem Fachmann bekannten Methoden durchführbar (s. Schema 6).

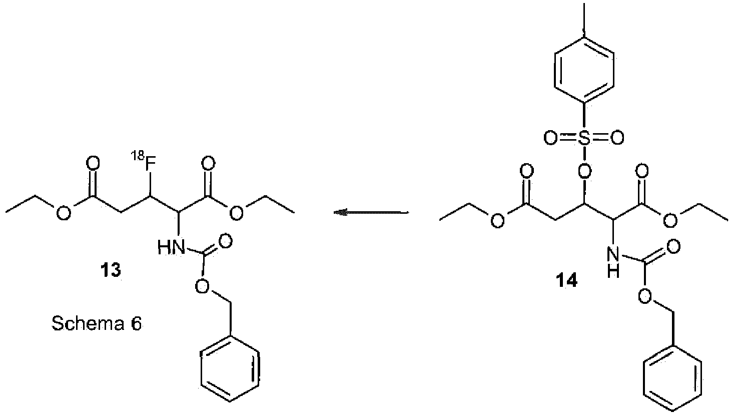

Dabei kann Verbindung **14** in Gegenwart einer Base wie zum Beispiel Tetra-alkyl ammonium und Tetra-alkl-phosphonium-carbonat und Kaliumcarbonat etc. mit der entsprechenden [F-18]-Fluoridlösung umgesetzt werden. Die Reaktion läuft bevorzugt bei erhöhten Temperaturen ab. Der Zusatz von Kronenethern, wie z. Bsp. Kryptofix (K2.2.2) kann die Reaktion positiv beeinflussen, besonders in Kombination mit K₂CO₃ als katalysierende Base. Mögliche Lösungsmittel sind bevorzugt aprotisch, aber auch protische Lösungsmittel oder aber aprotische Lösungsmittelzusätze, wie z. Bsp. Wasser, können zur Anwendung kommen. Üblicherweise werden für die radiochemische Fluorierung mit [F-18]-Fluoridanionen Acetonitril, Dimethylsulfoxid oder Dimethylformamid als optimale Lösungsmittel angewandt. Verbindung **13** muss üblicherweise keiner Reinigung unterzogen werden, sondern kann umgehend mit den für die Umsetzung von **13** nach **12** beschriebenen Methoden behandelt werden. Eine Reinigung der Verbindung **13** ist aber prinzipiell möglich, bevorzugt mittels einer präparativen HPLC mit einer unpolaren Phase, wie z. Bsp. RP C-18. Darüber hinaus ist auch eine Reinigung mittels Kartuschen möglich.

Erfindungsgemäße Verbindungen, bei denen das [F-18]-Isotop über eine Methylengruppe in 4-Position des Glutaminsäuregerüstes positioniert ist, wie z. Bsp. bei 4-[F-18]Fluoromethyl-glutaminsäure (**15**), lassen sich wie in Schema 7 dargestellt, herstellen. So gelingt beispielshalber die saure Abspaltung der Schutzgruppen der Verbindung **16,** um die erfindungsgemäße Verbindung 4-[F-18]Fluoromethyl-glutaminsäure (**15**) zu erhalten.

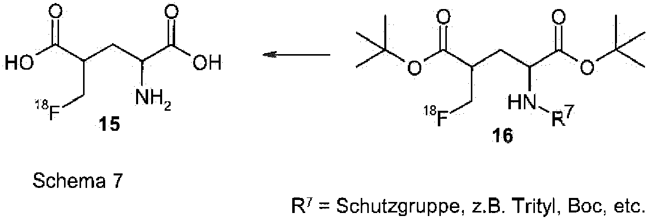

Dabei können verschiedene organische (z. Bsp. Trifluoressigsäure), vor allem aber anorganische Säuren wie z. Bsp. Bromwasserstoffsäure, Salzsäure, Schwefelsäure, Perchlorsäure oder Phosphorsäure zum Einsatz kommen. Die Reinigung der erfindungsgemäßen Verbindung **16** nach Formel (I) ist per HPLC möglich, wobei verschiedene Reinigungsschritte prinzipiell vorgeschaltet und nachgeschaltet sein können, wie z. Bsp. die Reinigung über eine RP-C18 Kartusche oder andere Trennmaterialien.

Die radiochemische Fluorierung von Tosylat **17,** dessen Synthese analog der in der Literatur beschriebenen Methode (Chem. Pharm. Bull., 17, 5, (1969), 879-885) aus **18** (Tetrahedron, 45, 5, (1989) 1453-1464) erfolgte, zum [F-18]-markierten Glutaminsäurederivat **16** ist nach den dem Fachmann bekannten Methoden durchführbar (s. Schema 8).

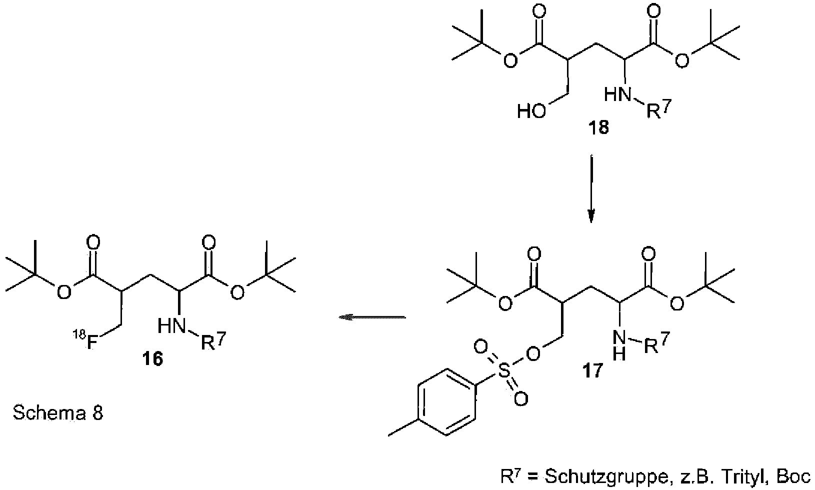

Dabei kann Verbindung **17** in Gegenwart einer Base wie zum Beispiel Tetra-alkyl ammonium und Tetra-alkyl-phosphonium-carbonat und Kaliumcarbonat etc. mit der entsprechenden [F-18]-Fluoridlösung umgesetzt werden. Die Reaktion läuft bevorzugt bei erhöhten Temperaturen ab. Der Zusatz von Kronenethern, wie z. Bsp. Kryptofix (K2.2.2) kann die Reaktion positiv beeinflussen, besonders in Kombination mit K₂CO₃ als katalysierende Base. Mögliche Lösungsmittel sind bevorzugt aprotisch, aber auch protische Lösungsmittel oder aber aprotische Lösungsmittelzusätze, wie z. Bsp. Wasser, können zur Anwendung kommen. Üblicherweise werden für die radiochemische Fluorierung mit [F-18]-Fluoridanionen Acetonitril, Dimethylsulfoxid oder Dimethylformamid als optimale Lösungsmittel angewandt. Verbindung **16** muss üblicherweise keiner Reinigung unterzogen werden, sondern kann umgehend mit den für die Umsetzung von **16** nach **15** beschriebenen Methoden behandelt werden. Eine Reinigung der Verbindung **16** ist aber prinzipiell möglich, bevorzugt mittels einer präparativen HPLC mit einer unpolaren Phase, wie z. Bsp. RP C-18.

Erfindungsgemäße Verbindungen, bei denen das [F-18]-Isotop über eine Alkoxygruppe in 4-Position des Glutaminsäuregerüstes positioniert ist, wie z. Bsp. bei 4-[F-18]Fluoroethoxy-glutaminsäure (**20**), lassen sich wie in Schema 9 dargestellt, herstellen. So gelingt beispielshalber die saure Abspaltung der Schutzgruppen der Verbindung **21** oder **22** um die erfindungsgemäße Verbindung 4-[F-18]Fluoroethoxy-glutaminsäure (**20**) zu erhalten.

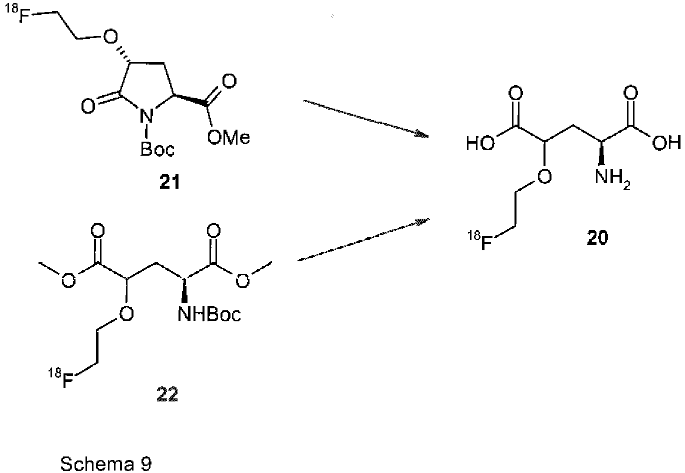

Dabei können verschiedene organische (z. Bsp. Trifluoressigsäure), vor allem aber anorganische Säuren wie z. Bsp. Bromwasserstoffsäure, Salzsäure, Schwefelsäure, Perchlorsäure oder Phosphorsäure zum Einsatz kommen. Weiterhin ist auch eine basische Ringöffnung von **21** möglich mit Lithiumhydroxid, Natriumhydroxid, Kaliumhydroxid etc. (S. Baker et al. Tetrahedron Lett. 1998, 39, 2815-2818.).
Die Reinigung der erfindungsgemäßen Verbindung **20** nach Formel (I) ist per HPLC möglich, wobei verschiedene Reinigungsschritte prinzipiell vorgeschaltet und nachgeschaltet sein können, wie z. Bsp. die Reinigung über eine RP-C18 Kartusche oder andere Trennmaterialien.

Die radiochemische Fluorierung von Tosylat **23** dessen Synthese analog der in der Literatur beschriebenen Methode (N. Sharma et al. Tetrahedron Lett. 2004, 45, 1403-1406.) aus **24** erfolgte, zum [F-18]-markierten Glutaminsäurederivat **21** ist nach den dem Fachmann bekannten Methoden durchführbar (s. Schema 10).

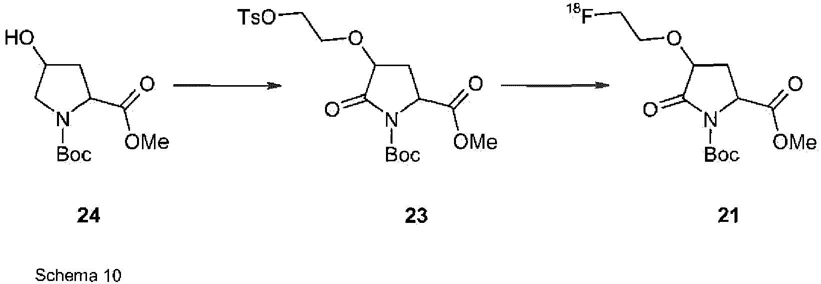

Dabei kann Verbindung **21** in Gegenwart einer Base wie zum Beispiel Tetra-alkyl ammonium und Tetra-alkyl-phosphonium-carbonat und Kaliumcarbonat etc. mit der entsprechenden [F-18]-Fluoridlösung umgesetzt werden. Die Reaktion läuft bevorzugt bei erhöhten Temperaturen ab. Der Zusatz von Kronenethern, wie z. Bsp. Kryptofix (K2.2.2) kann die Reaktion positiv beeinflussen, besonders in Kombination mit K₂CO₃ als katalysierende Base. Mögliche Lösungsmittel sind bevorzugt aprotisch, aber auch protische Lösungsmittel oder aber aprotische Lösungsmittelzusätze, wie z. Bsp. Wasser, können zur Anwendung kommen. Üblicherweise werden für die radiochemische Fluorierung mit [F-18]-Fluoridanionen Acetonitril, Dimethylsulfoxid oder Dimethylformamid ais optimale Lösungsmittel angewandt. Verbindung **21** muss üblicherweise keiner Reinigung unterzogen werden, sondern kann umgehend mit den für die Umsetzung von **21** nach **20** beschriebenen Methoden behandelt werden. Eine Reinigung der Verbindung **21** ist aber prinzipiell möglich, bevorzugt mittels einer präparativen HPLC mit einer unpolaren Phase, wie z. Bsp. RP C-18. Außerdem ist eine Reinigung mittels Kartuschen möglich.

Die radiochemische Fluorierung von Tosylat **25** dessen Synthese analog der in der Literatur beschriebenen Methode (X. Zhang Tetrahedron Lett. 2001, 42, 5335-5338.) aus **23** erfolgte, zum [F-18]-markierten Glutaminsäurederivat **22** ist nach den dem Fachmann bekannten Methoden durchführbar (s. Schema 11).

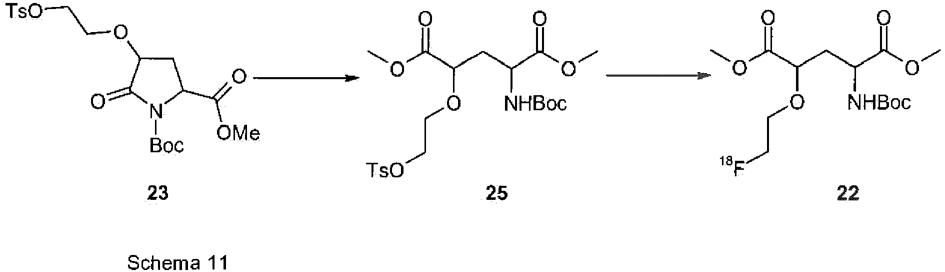

Dabei kann Verbindung **25** in Gegenwart einer Base wie zum Beispiel Tetra-alkyl ammonium und Tetra-alkyl-phosphonium-carbonat und Kaliumcarbonat etc. mit der entsprechenden [F-18]-Fluoridlösung umgesetzt werden. Die Reaktion läuft bevorzugt bei erhöhten Temperaturen ab. Der Zusatz von Kronenethern, wie z. Bsp. Kryptofix (K2.2.2) kann die Reaktion positiv beeinflussen, besonders in Kombination mit K₂CO₃ als katalysierende Base. Mögliche Lösungsmittel sind bevorzugt aprotisch, aber auch protische Lösungsmittel oder aber aprotische Lösungsmittelzusätze, wie z. Bsp. Wasser, können zur Anwendung kommen. Üblicherweise werden für die radiochemische Fluorierung mit [F-18]-Fluoridanionen Acetonitril, Dimethylsulfoxid oder Dimethylformamid als optimale Lösungsmittel angewandt. Verbindung **22** muss üblicherweise keiner Reinigung unterzogen werden, sondern kann umgehend mit den für die Umsetzung von **22** nach **20** beschriebenen Methoden behandelt werden. Eine Reinigung der Verbindung **22** ist aber prinzipiell möglich, bevorzugt mittels einer präparativen HPLC mit einer unpolaren Phase, wie z. Bsp. RP C-18. Außerdem ist eine Reinigung mittels Kartuschen möglich.

Die Synthese von F-19 Referenzverbindungen **26, 27** und **28** kann wie in Schema 12 dargestellt erfolgen.

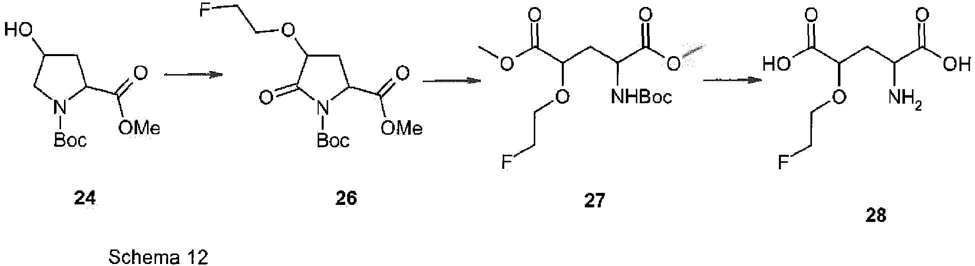

**26** kann durch Alkylierung und Oxidation des Hydroxyprolinderivats **24** erhalten werden. Durch Ringöffnung des Pyroglutaminderivats **26** wird die offenkettige Referenzverbindung **27** erhalten. Die saure Abspaltung der Schutzgruppen führt zum Glutaminsäurederivat **28.**

Erfindungsgemäße Verbindungen, bei denen das [F-18]-Isotop über eine Alkylgruppe in 4-Position des Glutaminsäuregerüstes positioniert ist, wie z. Bsp. bei 4-[F-18]Fluoropropyl-glutaminsäure (**29**) oder 4-[F-18]Fluorobutyl-glutaminsäure (**30**) lassen sich wie in Schema 13 dargestellt, herstellen. So gelingt beispielshalber die saure Abspaltung der Schutzgruppen der Verbindungen **31** und **32,** um die erfindungsgemäßen Verbindung 4-[F-18]Fluoropropyl-glutaminsäure (**29**) oder 4-[F-18]Fluorobutyl-glutaminsäure (**30**) zu erhalten.

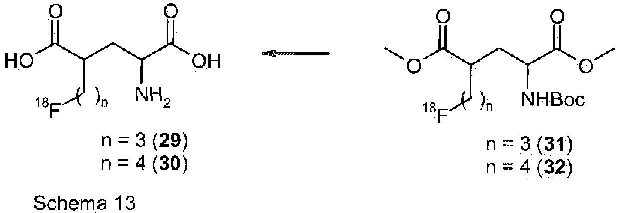

Dabei können verschiedene organische (z. Bsp. Trifluoressigsäure), vor allem aber anorganische Säuren wie z. Bsp. Bromwasserstoffsäure, Salzsäure, Schwefelsäure, Perchlorsäure oder Phosphorsäure zum Einsatz kommen. Die Reinigung der erfindungsgemäßen Verbindung **29** und **30** nach Formel (I) ist per HPLC möglich, wobei verschiedene Reinigungsschritte prinzipiell vorgeschaltet und nachgeschaltet sein können, wie z. Bsp. die Reinigung über eine RP-C18 Kartusche oder andere Trennmaterialien.

Die radiochemische Fluorierung von Bromid **33** oder Tosylat **34,** deren Synthese analog der in der Literatur beschriebenen Methode (S. Hanessian, et al. J. Org. Chem. 2005, 70, 5070-5085) aus **35** erfolgte, zu den [F-18]-markierten Glutaminsäurederivaten **31** und **32** ist nach den dem Fachmann bekannten Methoden durchführbar (s. Schema 14).

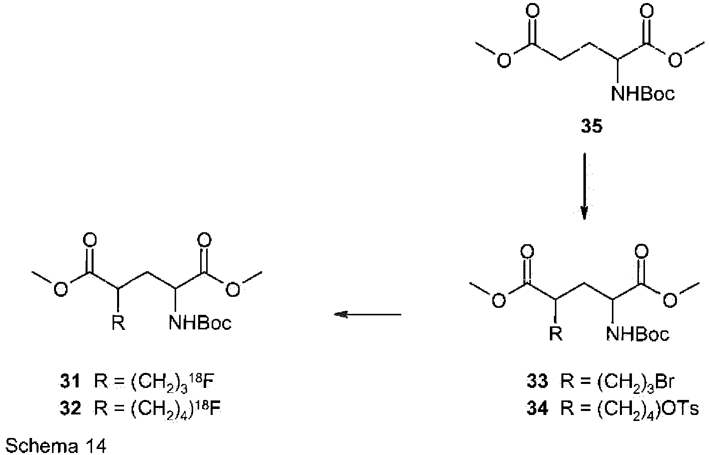

Dabei können die Verbindungen **33** und **34** in Gegenwart einer Base wie zum Beispiel Tetra-alkyl ammonium und Tetra-alkyl-phosphonium-carbonat und Kaliumcarbonat etc. mit der entsprechenden [F-18]-Fluoridlösung umgesetzt werden. Die Reaktion läuft bevorzugt bei erhöhten Temperaturen ab. Der Zusatz von Kronenethern, wie z. Bsp. Kryptofix (K2.2.2) kann die Reaktion positiv beeinflussen, besonders in Kombination mit K₂CO₃ als katalysierende Base. Mögliche Lösungsmittel sind bevorzugt aprotisch, aber auch protische Lösungsmittel oder aber aprotische Lösungsmittelzusätze, wie z. Bsp. Wasser, können zur Anwendung kommen. Üblicherweise werden für die radiochemische Fluorierung mit [F-18]-Fluoridanionen Acetonitril, Dimethylsulfoxid oder Dimethylformamid als optimale Lösungsmittel angewandt. Die Verbindungen **31** und **32** müssen üblicherweise keiner Reinigung unterzogen werden, sondern können umgehend mit den für die Umsetzung von **31** nach **29** bzw. **32** nach **30** beschriebenen Methoden behandelt werden. Eine Reinigung der Verbindungen **31** bzw. **32** ist aber prinzipiell möglich, bevorzugt mittels einer präparativen HPLC mit einer unpolaren Phase, wie z. Bsp. RP C-18.

Die Synthese der F-19 Referenzverbindungen **36** und **37** kann durch Alkylierung des Glutaminsäurederivats **35** erfolgen (Schema 15).

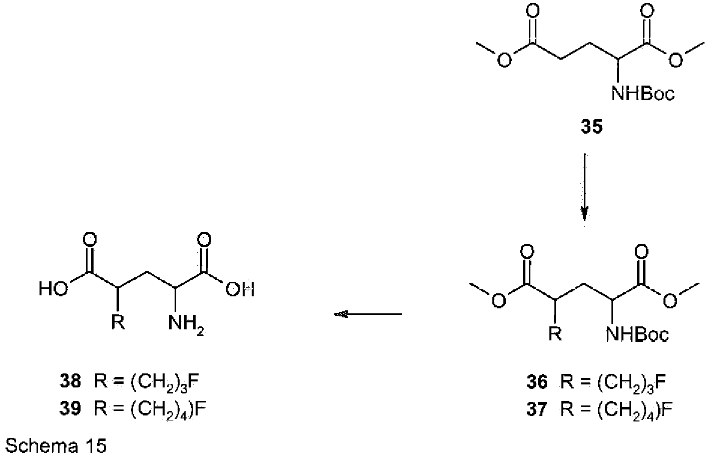

Die Abspaltung der Schutzgruppen führt zu den Fluoralkylierten Glutaminsäurederivaten **38** und **39.**

In einem vierten Aspekt der Erfindung werden Verbindungen der Formel (IV) verwendet zur Herstellung von Verbindungen der Formel (I) oder (II): 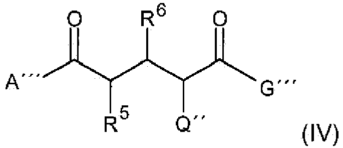 worin
A''' für
   a) hydroxyl,
   b) verzweigtes oder unverzweigtes Hydroxy C₁-C₅ Alkoxy,
   c) N(C₁-C₅ Alkyl)₂,
   d) NH₂,
   e) N(H)-U",
   f) N(H)-L''' oder
   g) O-L'''
   steht,
G''' für
   a) hydroxyl,
   b) verzweigtes oder unverzweigtes O-C₁-C₅ Alkyl,
   c) verzweigtes oder unverzweigtes O-C₂-C₅ Alkenyl,
   d) verzweigtes oder unverzweigtes O-C₁-C₅ Alkyl-(O-C₁-C₄ alkyl)ₙ-O-C₁-C₄ alkyl oder
   e) verzweigtes oder unverzweigtes O-C₂-C₅ Alkinyl,
   f) triphenylmethoxy
   steht,
R⁵ und R⁶ für
   a) Wasserstoff oder
   b) E'
   steht, mit der Maßgabe, dass genau einer der Substituenten R⁵ oder R⁶ ein E' beinhaltet und der jeweils andere Substituent kein E' beinhaltet,
E' für
   a) Chloro,
   b) Bromo,
   c) Mesyloxy,
   d) Trifluormesyloxy,
   e) Nonafluorbutyloxy oder
   f) Tosyloxy
   steht,
Q" für
   a) N(H)-tert-Butoxycarbonyl,
   b) N(H)-Allyloxycarbonyl,
   c) N(H)-Benzyloxycarbonyl,
   d) N(H)-Ethoxycarbonyl,
   e) N(H)-Methoxycarbonyl,
   f) N(H)-Propoxycarbonyl,
   g) N(H)-2,2,2-Trichlorethoxycarbonyl,
   h) N(H)-1,1-Dimethylpropinyl,
   i) N(H)-1-Methyl-1-phenyl-ethoxycarbonyl,
   j) N(H)-1-Methyl-1-(4-biphenylyl)-ethoxycarbonyl,
   k) N(H)-cyclobutylcarbonyl,
   l) N(H)-1-Methylcyclobutylcarbonyl,
   m) N(H)-Vinylcarbonyl,
   n) N(H)-Allylcarbonyl,
   o) N(H)-Adamantylcarbonyl,
   p) N(H)-Diphenylmethylcarbonyl,
   q) N(H)-Cinnamylcarbonyl,
   r) N(H)-Formyl,
   s) N(H)-Benzoyl,
   t) N(H)-Trityl,
   u) N(H)-p-Methoxyphenyl-diphenylmethyl,
   v) N(H)-di-(p-Methoxyphenyl)-phenylmethyl, oder
   w) 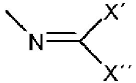
   x) N-(tert-Butoxycarbonyl)₂,
   steht,
L''' für
   a) verzweigtes oder unverzweigtes C₁-C₅ Alkyl,
   b) verzweigtes oder unverzweigtes C₂-C₅ Alkenyl,
   c) verzweigtes oder unverzweigtes C₁-C₅ Alkyl-(O-C₁-C₄alkyl)ₙ-O-C₁-C₄ alkyl, oder
   d) verzweigtes oder unverzweigtes C₂-C₅ Alkinyl
   steht,
U" für
   a) tert-Butoxycarbonyl,
   b) Allyloxycarbonyl,
   c) Benzyloxycarbonyl, oder
   d) Ethoxycarbonyl
   steht,
X' and X" unabhängig voneinander für
   a) verzweigtes oder unverzweigtes C₁-C₅ Alkyl,
   b) substituiertes oder unsubstituiertes Aryl,
   c) Aralkyl, oder
   d) Heteroaryl
   steht
wobei n = 0, 1, 2 oder 3 ist und alle möglichen Diastereomere und Enantiomere Teil des vorliegenden Erfindungsgegenstandes sind.

Das Verfahren zur Herstellung der erfindungsgemäßen Verbindungen der allgemeinen Formel (I) oder (II) zeichnet sich dadurch aus, dass die Mehrzahl der Verbindungen nach Formel (I) oder (II), aus einer Vorläuferverbindung der Verbindungen der Formel (IV) nach Einführung des ¹⁸F Isotops entstehen kann.

Die vorliegende Erfindung betrifft Verbindungen der allgemeinen Formel (IV).

Bevorzugt für die Einführung des ¹⁸F Isotops sind
4,7,13,16,21,24-Hexaoxa-1,10-diazabicyclo[8.8.8]-hexacosane K18F (crownether salt Kryptofix K18F),
K¹⁸F,
H¹⁸F,
KH¹⁸F₂,
Cs¹⁸F,
Na¹⁸F oder
¹⁸F tetraalkylammonium salz (z.b [F-18] tetrabutylammonium fluoride).

Die vorliegende Erfindung betrifft Verbindungen der allgemeinen Formel (I) oder (II) und Verfahren wobei die Fluorisotope ¹⁸F und ¹⁹F verwendet sind.

Wenn ein oder mehrere chirale Zentren in einer Verbindung des vorliegenden Erfindungsgegenstandes der Formel (I), Formel (II), Formel (III), oder Formel (IV) vorhanden ist bzw. sind, so sollen alle Formen dieses Isomers, einschließlich beider Enantiomere und aller möglichen Diastereomere, hierin beinhaltet sein. Verbindungen, die mindestens ein chirales Zentrum enthalten, können als racemische Mischung, gegebenenfalls als Diastereomeren- oder Diastereomeren-angereicherte Mischung oder als Enantiomeren-angereicherte Mischung eingesetzt werden. Die racemische, enantiomeren-angereicherte Mischung oder Diastereomerenmischung kann gegebenenfalls nach den dem Fachmann bekannten Methoden getrennt werden, so dass die Enantiomere oder Diastereomere einzeln eingesetzt werden können. In solchen Fällen, in denen eine Kohlenstoff-Kohlenstoff-Doppelbindung vorliegt, so sind beide Isomere "cis" und "trans" Teil der vorliegenden Erfindung. In solchen Fällen, in denen tautomere Formen vorliegen können, wie z. Bsp. Keto-enol Tautomerie, sind alle tautomeren Formen in der vorliegenden Erfindung beinhaltet, wobei diese Formen im Gleichgewicht oder bevorzugt in einer Form vorliegen können.

Die Verbindungen der allgemeinen Formel I oder II und ihre bevorzugten Ausführungsformen werden verwendet als Arzneimittel.
Die erfindungsgemäßen Verbindungen der allgemeinen Formel I oder II und ihre bevorzugten Ausführungsformen werden verwendet in der Diagnose von physiologischen oder pathologischen Zuständen.
Bevorzugt finden diese Verbindungen Anwendung in der nicht-invasiven PET-basierten Diagnose am menschlichen oder tierischen Körper.
Besonders bevorzugt finden die erfindungsgemäßen Verbindungen der allgemeinen Formel I oder II und ihre bevorzugten Ausführungsformen Anwendung in der Diagnose von Tumorerkrankungen. Beispiele für solche Tumorerkrankungen sind Malignome des Gastrointestinal- oder Kolorektaltraktes, Leber-, Pankreas-, Nieren-, Blasen-, Schilddrüsen-, Prostata-, Endometrium-, Ovar-, Testes-, Melanom-, kleinzelliges und nicht-kleinzelliges Bronchialkarzinom, dysplastisches orales Mucosa-Karzinom, invasiver Oral-Krebs; Brustkrebs, einschließlich hormonabhängigem und hormonunabhängigem Brustkrebs, Plattenepithelkarzinom, neurologische Krebserkrankungen einschließlich Neuroblastom, Gliom, Astrocytom, Osteosarcom, Meningiom; Weichteilsarkom; Hämangioam und endokrine Tumore, einschließlich Hypophysenadenome, Chromocytome, Paragangliome, haematologische Tumorerkrankungen einschließlich Lymphoma and Leukämien; oder Metastasen einer der oben genannten Tumoren.
Die erfindungsgemäßen Verbindungen der allgemeinen Formel I oder II und ihre bevorzugten Ausführungsformen werden verwendet zur Herstellung eines Arzneimittels für die Diagnose von Tumorerkrankungen. Beispiele für solche Tumorerkrankungen sind Malignome des Gastrointestinal- oder Kolorektaltraktes, Leber-, Pankreas-, Nieren-, Blasen-, Schilddrüsen-, Prostata-, Endometrium-, Ovar-, Testes-, Melanom-, kleinzelliges und nicht-kleinzelliges Bronchialkarzinom, dysplastisches orales Mucosa-Karzinom, invasiver Oral-Krebs; Brustkrebs, einschließlich Hormon-abhängigem und Hormon-unabhängigem Brustkrebs, Plattenepithelkarzinom, neurologische Krebserkrankungen einschließlich Neuroblastom, Gliom, Astrocytom, Osteosarcom, Meningiom, Weichteilsarkom; Hämangioam und endokrine Tumore, einschließlich Hypophysenadenome, Chromocytome, Paragangliome, haematologische Tumorerkrankungen einschließlich Lymphoma and Leukämien; oder Metastasen einer der oben genannten Tumoren.

Die Erfindung betrifft pharmazeutische Präparate, welche mindestens eine Verbindung der Formel I oder II sowie einen pharmazeutisch verträglichen Träger enthalten.

Zur Verwendung der Verbindungen der Formel I oder II als Arzneimittel werden diese in die Form eines pharmazeutischen Präparats gebracht, welches neben dem Wirkstoff für die enterale oder parenterale Applikation geeignete pharmazeutische, organische oder anorganische inerte Trägermaterialien, wie zum Beispiel, Wasser, Gelatine, Gummi arabicum, Milchzucker, Stärke, Magnesiumstearat, Talk, pflanzliche Öle, Polyalkylenglykole usw. enthält.

Die Erfindung betrifft eine Einrichtung (Kit) enthaltend mindestens eine Verbindung der Formel I, II, III, oder IV.

### Beispiele:

### Beispiel 1

### Synthese von 4-[F-18]Fluoroglutaminsäure

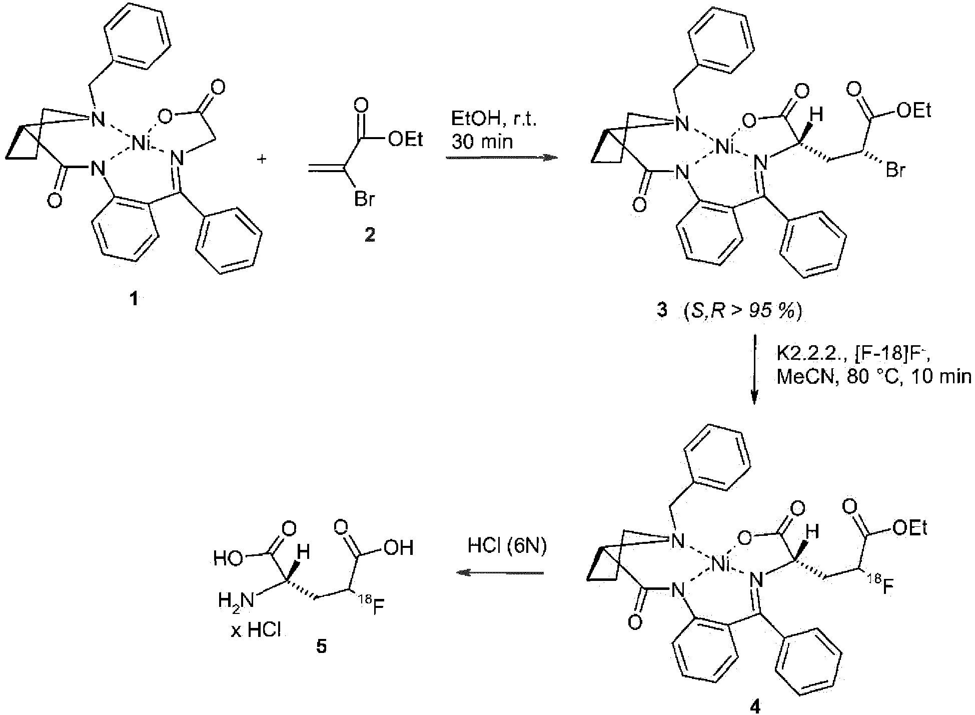

### Synthese von 3:

Diisopropylamin (0.031 ml, 0.22 mmol) wurde zu einer Suspension von Komplex 1 (0.1 g, 0.2 mmol) (J. Am. Chem. Soc., 1985, 107, 4252 oder Tetrahedron Asymmetry, 1998, 9, 4249) in EtOH (0.4 ml) bei Raumtemperatur (RT) gegeben. Die Reaktionsmischung wurde für 30 min. gerührt und anschließend mit frisch destilliertem Ester **2** (0.04 mL, 0.33 mmol) (Gazz. Chim. Ital., 1981, 111, 249) versetzt. Die Reaktion wurde mittels DC verfolgt (SiO₂, AcOEt/ CHCl₃, 1 : 1). Nach Abschluß der Reaktion (∼ 2.5 h) wurde die Reaktionsmischung neutralisiert durch die Zugabe von AcOH (1.5 ml; 2 %). Danach wurde CHCl₃ (15 ml) zugegeben, mit Wasser gewaschen (3 x 15 ml), die organische Phase separiert, über Na₂SO₄ getrocknet und im Vakuum bis zur Trockene eingeengt. Preparative DC (SiO₂, AcOEt/ CHCl₃, 1 : 1) lieferte eine Mischung der Komplexe **3** (S,S,S) und **3** (S,R,S/R) in einem Verhältnis von 11 : 1 und einer Ausbeute von 28% (*R*_{f} 0.52). Komplex **3** (S,S,R) zeigte einen *R*_{f}-Wert von 0.49. Komplex **3** (S,S,R) wurde mit MeOH (3 x 40 mL) vom Kieselgel eluiert und anschließend durch Säulenchromatographie an Sephadex mit einer 1 : 3 EtOH/ C₆H₆ Mischung gereinigt. Die Reinigung lieferte 0.052 g (52%) of des Produktes. Elementaranalyse: gefunden (%): C, 56.79; H, 4.85; Br, 12.14; N, 6.10; Ni, 8.17. C₃₂H₃₂BrN₃NiO₅. Kalkuliert (%) C, 56.75; H, 4.76; Br, 11.80; N, 6.20; Ni, 8.67.

### 4-[F-19]Fluor-L-glutaminsäure (Nichttradioaktiver Standard für HPLC Identifikation von 4-[F-18]Fluor-L-Glutaminsäure)

**Kondensation des Ni-BPB-Gly mit 2-Fluoro-acrylsäureethylester.** Zu einer Suspension von 3 g (6 mmol) Ni-BPB-Gly in 15 ml MeOH wurde 1 ml (7.2 mmol) i-Pr₂NH bei RT gegeben. Die Reaktionsmischung wurde 30 min. gerührt und anschließend mit 3.7 ml (30 mmol) 2-Fluoro-acrylsäureethylester versetzt. Der Fortschritt der Reaktion wurde mittels DC an SiO₂ (AcOEt:/CHCl₃ (2:3)) verfolgt. Nach ca. 250 Stunden war die Reaktion abgeschlossen. Danach wurde die Mischung neutralisiert durch Zugabe von 42 ml einer 2 % igen wässrigen Lösung von AcOH gemischt mit 25 ml MeOH. Die resultierende Mischung der diasteroisomeren Komplexe Ni-BPB-4-F-GluOMe wurde ausgefällt. Der Niederschlag wurde abfiltriert und mit Wasser (3 x 30 ml) gewaschen. Der resultierende feste Komplex wurde in CCl₄ suspendiert und im Vakuum zur Trockene eingeengt. Diese Prozedur wurde drei mal wiederholt um die Mischung von Wasser zu befreien. Die Komplexe wurden durch Säulenchromatographie (SiO₂, 3x20 cm, AcOEt/CHCl₃, 3:2) gereinigt. Die Hauptfraktion, 2.66 g, (4.4 mmol, 74 %) enthielt eine Mischung von Ni-BPB-(2S,4R)-4-F-GluOMe und Ni-BPB-(2S,4S)-4-F-GluOMe in einem Verhältnis von 1,5/ 1. Schmelzpunkt: 191 - 193 °C. [α]D25 +2477 (c 0.5, CHCl3). Elementaranalyse: gefunden (%): C, 61,76; H, 5,02; N, 6,94; Ni 9,20. Berechnet für C₃₁H₃₀FN₃NiO₅ (%) C, 61,82; H, 5,02; N, 6,98; Ni, 9,74.

**Separierung der Komplexe** wurde durch säulenchromatographische Trennung an Toyopearl HW-55F (Säule 2 × 50 cm, THF/ C₆H₆ (2 : 7)) erzielt.
Komplex Ni-BPB-(2S,4R)-4-F-GluOMe: Schmelzpunkt: 207 - 208 °C., [α]_{D}²⁵ +2617 (c 0.035, MeOH). Elementaranalyse: gefunden (%): C, 61,79; H, 4,95; N, 6,88. Berechnet für C₃₁H₃₀FN₃NiO₅ (%) C, 61,82; H, 5,02; N, 6,98.
Komplex Ni-BPB-(2*S*,4*S*)-4-F-GluOMe. Schmelzpunkt: 233 - 235 °C., [α]_{D}²⁵ +2641 (c 0,039, MeOH). Elementaranalyse: gefunden (%): C, 61,63; H, 4,86; N, 6,84. Berechnet für C₃₁H₃₀FN₃NiO₅ (%) C, 61,82; H, 5,02; N, 6,98.
***Zersetzung des Komplexes und Isolierung der Aminosäure.*** In einem Rundkolben wurden 2.75 g (4.57 mmol) Ni-BPB-4-F-GluOMe-Komplex in 30 ml MeOH gelöst und unter Rühren mit 5.5 ml HCl (6N) versetzt. Die Reaktionsmischung wurde für 15-20 min. am Rückfluß erhitzt, zur Trockene eingeengt, mit 50 ml Wasser verdünnt, das Hydrochlorid des BPB abfiltriert und vorsichtig mit 3 x 30 ml Wasser gewaschen. Die vereinigten Filtrate enthalten die Aminosäure, BPB-Rückstände und Ni²⁺-Salze und wurden mit wässriger NH₃-Lösung auf pH 5 eingestellt. BPB-Rückstände wurden mit CHCl₃ (3 × 30 ml) extraktiv entfernt. Die vereinigten wässrigen Phasen wurden zur Trockene eingeengt, mit 3 ml HCl (6N) versetzt und für 1 h am Rückfluß erhitzt. Die Lösung wurde anschließend zur Trockene eingeengt, in 5 ml H₂O gelöst und mit 5 % iger (wässrig) NH₃-Lösung auf pH 4 eingestellt. Die Aminosäure wurde mittels Ioneneaustauschchromatographie über eine Dowex-Säule 50w×8 in H⁺-Form (Eluent: 5% NH₃ aq) isoliert.
(2*S*,4*R*)-4-Fluoroglutaminsäure: Schmelzpunkt: >300 °C (Zersetzung ohne Schmelzpunkt). (2*S*,4*S*)-4-Fluoroglutaminsäure: Schmelzpunkt: >300 °C (Zersetzung ohne Schmelzpunkt).

### F-18 Radiomarkierung:

Target: Kleinvolumiges Niederdruck-Silber-Target (1 ml) gefüllt mit [O-18] Wasser für ¹⁸O (p,n)¹⁸F Reaktion; Zyklotron: Scanditronix MC 17; Protonenbeschuß bei 17 MeV.
F-18 Fluorid wurde auf einer QMA-Harzkartusche (Waters, Sep Pak Light QMA Part.No.: WAT023525) durch Aufgabe der [F-18]/[O-18] Targetlösung konzentriert. Die Kartusche wurde mit K₂CO₃-Lösung (10 ml; 0.5 M) gefolgt von deionisiertem Wasser (15 ml) präkonditioniert.

[F-18] radioaktiver Komplex: [F-18]Fluorid (15 - 300 mCi) wurde mittels Waschlösung (2 ml, MeCN (2 ml)/ Tetrabutylammoniumkarbonat (TBAC, 0,015 mL, 20 % wässrig, pH 8)) von der QMA-Kartusche eluiert. Das Eluat wurde in einem 5 ml Vial aufgefangen und die Lösungsmittel durch Azeotropdestillation bei 130 °C im Stickstoffstrom entfernt.
Nucleophile Substitution: Das Reaktionsgefäß mit dem trockenen [F-18] TBA-fluorid wurde auf 80 °C (vorhergehender Schritt) abgekühlt und eine Lösung von Präkursor **3** (S,S,R) (5 mg in MeCN (0.5 ml)) wurde zugesetzt. Die Reaktionsmischung wurde für 5 - 10 min. bei 80 °C gehalten. Eine Probe der Reaktionsmischung wurde mittels Radio-DC (Kieselgelplatte (Merck), Eluent: Ethylacetate/ Chloroform/ Essigsäure (4/1/1) untersucht. Basierend auf diesen Radio-DC-Daten wurde eine F-18-Inkorporation von 40 - 60 % in **4** ermittelt.

Zersetzung des ¹⁸F-fluorierten Ni-Komplexes und Freisetzung von 4-[¹⁸F]Fluoroglutamisäure (A). HCl (6 N, 0.3 - 0.5mL) wurde zur MeCN-Lösung des F-18-Glutamate-Nickelpräkursors gegeben und bei 140 °C für 5 min. behandelt. Eine Probe der resultierenden Reaktionsmischung wurde mittels Radio-DC (Kieselgel, Eluent: n-Butanol/Essigsäure/Wasser (12/3/5) analysiert.

Die DC-Analyse wurde auf einem MiniGita DC-Scanner (Raytest, Gemany) durchgeführt. Zersetzung des ¹⁸F-fluorierten Ni-Komplexes und Freisetzung von 4-[¹⁸F]Fluoroglutamisäure (B). HCl (2 N, 0.3 - 0.5mL) wurde zur MeCN-Lösung des F-18-Glutamate-Nickelpräkursors gegeben und bei 140 °C für 5 min. behandelt. Anschließend wurde mit Natronlauge (2 N, 0,8 - 1,0mL) neutralisiert. Eine Probe der resultierenden Reaktionsmischung wurde mittels Radio-DC (Kieselgel, Eluent: n-Butanol/Essigsäure/Wasser (12/3/5) analysiert.
Die DC-Analyse wurde auf einem MiniGita DC-Scanner (Raytest, Gemany) durchgeführt.

Vorreinigung: Das Rohprodukt nach der HCl-Behandlung (vorheriger Schritt) wurde in 1 mL Wasser aufgenommen und auf eine Anionenaustauscherkartusche (Waters, SAX-OH form) gegeben. 80 % der radioaktiven Produkte wurden auf der Kartusche zurückgehalten. Die radioaktiven Produkte wurden mittels wässriger Kochsalzlösung NaCl (0.4 M, 2 ml) von der Kartusche eluiert. Eine Probe wurde mittels HPLC analysiert.

Identifikation durch Radio-HPLC. Pumpe: Gilson 305, Injector: Rheodyne (20 µL Injektionsschleife), Säule: Zorbax -NH₂; 4.6 x 150 mm, mobile Phase: NaH₂PO₄ (10 mM)/ Phosphorsäure, pH 3, Fluß: 1 ml/ min, UV-Absorptionsdetektor: Gilson 116 in Serie mit einem Beckman 170 Radiodetektor, UV-Detektion: 210 nm. Rₜ: F-19-Referenz ((rac)-4-F-Glu-hydrochlorid (Diastereomerengemisch: 1/5; beschrieben vide supra)): 12.22 min (UV); Radioaktivitätsdetektion (Beckman): 12,64 min. Ein einzelner radioaktiver Peak wurde erhalten, welcher mit der Referenzverbindung ko-eluierte.

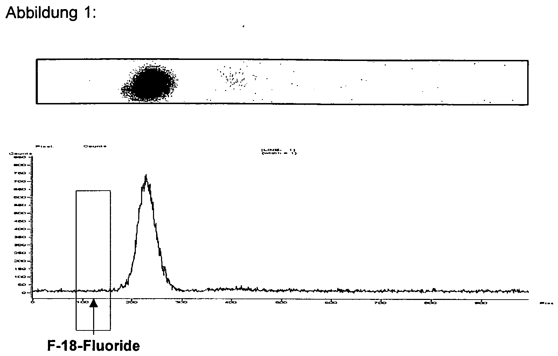

Identifikation durch Radio-DC: Kieselgelplatte (Mesh 60), Laufmittel n-BuOH, AcOH, H20 (12:3:5). Detektion: Phosphorimager: Sl Molecular Dynamics. Abbildung 1.

HPLC-Reinigung: Pumpe: Gilson 305, Injektor: Rheodyne (20 µL Injektionsschleife), Säule: Zorbax -NH₂; 4.6 x 150 mm, mobile Phase: NaH₂PO₄ (10 mM)/ Phosphorsäure, pH 3, Fluß: 1 ml/ min, UV-Absorptionsdetektor: Gilson 116 in Serie mit einem Beckman 170 Radiodetektor, UV-Detektion: 210 nm. Ein einzelner radioactiver Peak wurde erhalten, welcher mit der Referenzverbindung ko-eluierte. Wird das entstandene Produkt über HPLC aufgereinigt, kann auf die zu vor beschriebene Vorreinigung mittels Anionenaustauscherkartusche verzichtet werden.
Das Produkt konnte mit einer radiochemischen Reinheit von > 90 % und einer Radioaktivität von 15 - 200 mCi (zerfallskorrigiert) erhalten werden.

### Beispiel 2

### Synthese von 2-Amino-4-[F-19]fluoroglutamin (HPLC-Standard)

Zu einer Lösung von 0.27 g (0,448 mmol) Ni-BPB-4-F-GluOMe (M = 602.28, hergestellt in Analogie zu Beispiel 1) in 7 ml MeOH wurde flüssiger Ammoniak (18 g) gegeben. Die Reaktionslösung wurde für 2 h bei RT belassen. Die Lösung wurde am Vakuum eingeengt und der Rückstand per präparativer DC gereinigt (SiO₂, CHCl₃/Me₂CO (3:1)). Danach wurde das Produkt an Sephadex LH-20 (C₆H₆/EtOH (3:1) weiter gereinigt. Es wurden 0.15 g (0,255 mmol, 57 %) Ni-BPB-4-F-Gln (M.W. 587.28) erhalten.

### Synthese von 2-Amino-4-[F-18]fluoroglutamin

[F-18] radioaktiver Ni-BPB-4-F-GluOMe Komplex: [F-18]Fluorid (15 - 300 mCi) wurde mittels Waschlösung (2 ml, MeCN (2 ml)/ Tetrabutylammoniumkarbonat (TBAC, 0,015 mL, 20 % wässrig, pH 8)) von der QMA-Kartusche eluiert. Das Eluat wurde in einem 5 ml Vial aufgefangen und die Lösungsmittel durch Azeotropdestillation bei 130 °C im Stickstoffstrom entfernt.

Nucleophile Substitution: Das Reaktionsgefäß mit dem trockenen [F-18] TBA-fluorid wurde auf 80 °C (vorhergehender Schritt) abgekühlt und eine Lösung von Präkursor Ni-BPB-4-Br-GluOMe (5 mg in MeCN (0.5 ml)) wurde zugesetzt. Die Reaktionsmischung wurde für 5 - 10 min. bei 80 °C gehalten. Eine Probe der Reaktionsmischung wurde mittels Radio-DC (Kieselgelplatte (Merck), Eluent: Ethylacetate/ Chloroform/ Essigsäure (4/1/1) untersucht. Basierend auf diesen Radio-DC-Daten wurde eine F-18-Inkorporation von 40 - 60 % in Ni-BPB-4-F-GluOMe ermittelt.

### [F-18] radioaktiver Ni-BPB-4-F-Gln Komplex:

Zu einer Lösung von Ni-BPB-4-F-GluOMe (5 - 50 mCi) in 0.5 ml *t*-BuOH wurde eine Mischung aus 1 ml of *t*-BuOH und 1 g trockenem NH₃ (getrocknet über NaOH) gegeben. Die Reaktionsmischung wurde für 7 min. auf 42 °C erhitzt, bis kein Esterkomplex mehr nachweisbar war (DC, Kieselgelplatte (Merck), Eluent: Ethylacetate/ Chloroform/ Essigsäure (4/1/1)). Die Reaktion verlief quantitative und lieferte 4-40 mCi Ni-BPB-4-F-Gln.

Zersetzung des ¹⁸F-fluorierten Ni-BPB-4-F-Gln-Komplexes und Freisetzung von 4-[¹⁸F]Fluoroglutamisäure HCl (2 N, 0.3 - 0.5 mL) wurde zur MeCN-Lösung des [F-18] Ni-BPB-4-F-Gln Nickelpräkursors gegeben und bei 140 °C für 5 min. behandelt. Anschließend wurde mit Natronlauge (2 N, 0,8 - 1,0mL) neutralisiert. Eine Probe der resultierenden Reaktionsmischung wurde mittels Radio-DC (Kieselgel, Eluent: n-Butanol/Essigsäure/Wasser (12/3/5) analysiert.
Die DC-Analyse wurde auf einem MiniGita DC-Scanner (Raytest, Gemany) durchgeführt.

Vorreinigung: Das Rohprodukt nach der HCl-Behandlung (vorheriger Schritt) wurde in 1 mL Wasser aufgenommen und auf eine Anionenaustauscherkartusche (Waters, SAX-OH form) gegeben. 70 % der radioaktiven Produkte wurden auf der Kartusche zurückgehalten. Die radioaktiven Produkte wurden mittels wässriger Kochsalzlösung NaCl (0.4 M, 2 ml) von der Kartusche eluiert. Eine Probe wurde mittels HPLC analysiert.

Identifikation durch Radio-HPLC. Pumpe: Gilson 305, Injector: Rheodyne (20 µL Injektionsschleife), Säule: Zorbax -NH₂; 4.6 x 150 mm, mobile Phase: NaH₂PO₄ (10 mM)/ Phosphorsäure, pH 3, Fluß: 1 ml/ min, UV-Absorptionsdetektor: Gilson 116 in Serie mit einem Beckman 170 Radiodetektor, UV-Detektion: 210 nm. Rₜ: F-19-Referenz ((rac)-4-F-Gln-hydrochlorid: 8.04 min (UV); Radioaktivitätsdetektion (Beckman): 8,45 min. Ein einzelner radioaktiver Peak wurde erhalten, welcher mit der Referenzverbindung ko-eluierte.

HPLC-Reinigung: Pumpe: Gilson 305, Injektor: Rheodyne (20 µL Injektionsschleife), Säule: Zorbax -NH₂; 4.6 x 150 mm, mobile Phase: NaH₂PO₄ (10 mM)/ Phosphorsäure, pH 3, Fluß: 1 ml/ min, UV-Absorptionsdetektor: Gilson 116 in Serie mit einem Beckman 170 Radiodetektor, UV-Detektion: 210 nm. Ein einzelner radioactiver Peak wurde erhalten, welcher mit der Referenzverbindung ko-eluierte. Wird das entstandene Produkt über HPLC aufgereinigt, kann auf die zu vor beschriebene Vorreinigung mittels Anionenaustauscherkartusche verzichtet werden.
Das Produkt konnte mit einer radiochemischen Reinheit von > 92 % und einer Radioaktivität von 3 - 31 mCi (zerfallskorrigiert) erhalten werden.

### Beispiel 3

### Synthese von 2-Amino-3-[F-18]fluoro-pentandicarbonsäure

Zu einem Gemisch aus 60 µl wässriger 20%iger Tetrabutylammoniumcarbonat-Lösung in 1,5 ml Acetonitril (1.0 ml) wurden [F-18]-fluoridhaltige Lösung (33 µl, 789 MBq) gegeben. Das Lösungsmittel wurde bei 120°C Ofentemperatur im Stickstoffstrom durch Verdampfen entfernt. 1 ml wasserfreies Acetonitril wurde hinzu gegeben und erneut durch Evaporation entfernt. Dieser letzte Schritt wurde nochmals wiederholt. Eine Lösung von 3 mg 2-Benzyloxycarbonylamino-3-(toloyl-sulfonyloxy)-pentan-di-säure-diethylester (Chem. Pharm. Bull., 17, 5, (1969), 879-885) in 0,3 ml wasserfreiem Acetonitril wurden zum Rückstand gegeben und gut gerührt. Nach Erhitzen bei 90°C für 15 min wurden 2 ml 40%ige wässrige Bromwasserstoff-Lösung hinzugegeben. Die Reaktionsmischung wurde für 30 min und Überdruck bei 130 °C Ofentemperatur gerührt.

Das Rohprodukt wurde radio-analytisch per HPLC analysiert: Pumpe: Gilson 305, Injector: Rheodyne (20 µL Säule: Zorbax -NH₂; 4.6 x 150 mm, mobile Phase: NaH₂PO₄ (10 mM)/ Phosphorsäure, pH 3, Fluß: 1 ml/ min, UV-Absorptionsdetektor: Gilson 116 in Serie mit einem Beckman 170 Radiodetektor, UV-Detektion: 210 nm. ¹⁹F-Referenz (J. Org. Chem.; 50; 17; (1985); 3163-3167). (UV); Radioaktivitätsdetektion (Beckman). Ein einzelner radioactiver Peak wurde erhalten, welcher mit der Referenzverbindung ko-eluierte.

Die Reinigung der [F-18]-markierten Verbindung wurde mittels HPLC-Reinigung durchgeführt: Pumpe: Gilson 305, Injektor: Rheodyne (20 µL Injektionsschleife), Säule: Zorbax -NH₂; 4.6 x 150 mm, mobile Phase: NaH₂PO₄ (10 mM)/ Phosphorsäure, pH 3, Fluß: 1 ml/ min, UV-Absorptionsdetektor: Gilson 116 in Serie mit einem Beckman 170 Radiodetektor, UV-Detektion: 210 nm. Ein einzelner radioaktiver Peak wurde erhalten, welcher zeitgleich mit der ¹⁹F-Referenzverbindung (J. Org. Chem.; 50; 17; (1985); 3163-3167) eluierte. Wird das entstandene Produkt über HPLC aufgereinigt, kann auf die zu vor beschriebene Vorreinigung mittels Anionenaustauscherkartusche verzichtet werden. Das Produkt konnte mit einer radiochemischen Reinheit von ca. 92 % und einer Radioaktivität von 103 MBq erhalten werden.

### Beispiel 4

### Di-t-butyl-N-trityl-glutamat

Zu Di-t-butyl-glutaminsäurehydrochlorid (20,0 g, 68 mmol, SIGMA, cat#: G-7501), gelöst in MeCl₂ (100 ml), wurden Triethylamin (40 ml) und Tritylchlorid (19,0 g, 68,5 mmol) gegeben. Die Lösung wurde für 24 h bei Raumtemperatur gerührt und anschließend mit gesättigter Natriumkarbonatlösung (3 x) und Wasser (3 x) gewaschen. Die organische Phase wurde über MgSO₄ getrocknet, das Lösungsmittel unter Vakuum entfernt und das entstandene orangefarbene Öl per Flash-Chromatographie an Kieselgel in Hexan/ MeCl₂ (30/ 70) gereinigt. Der gewonnene weiße Feststoff enthielt das Produkt begleitet von einem Rückstand an Trityl-OH. Der Tritylalkohol wurde auskristallisiert durch Lösen des Produktgemisches in einer minimalen Menge an MeCl₂ und Zugabe von Hexan. Nach Filtration und Entfernung des Lösungsmittelgemisches wurde ein farbloses Öl erhalten. Ausbeute: 6,0 g, (18 %). DC: Rf = 0,5 (MeCl₂). Elementaranalyse C₃₂H₃₉NO₄: gefunden C: 76,4; H: 7,6; N: 2,9; berechnet: C: 76,6; H: 7,8; N: 2,8.

### t-Butyl-2-trityl-4-carbo-t-butyloxy-5-hydroxy-pentanoat

*n*-Butylithium (5,0 ml, 11 mmol) wurde bei 0 °C zu einer Cyclohexylisopropylaminlösung (3,0 ml, 15 mmol) in Hexan (50 ml) in einem Dreihalskolben gegeben. Die Lösung wurde 30 min. bei 0 °C gerührt, anschließend auf -78 °C abgekühlt und mit Di-t-butyl-N-trityl-glutamat (5,0 g, 10 mmol) in Hexan (50 ml) versetzt. Der Kolben wurde mit einem Gaseinleitungsrohr versehen und mit einem Vorratsgefäß, welches Paraformaldehyd und einen Gaseinlaß für Argon enthielt, verbunden. Nach der Bildung des Carbanions wurde der Paraformaldehyd auf 180 °C erhitzt und das entstehende Formaldehydgas in einem Argonstrom über 30 min. in das Reaktionsgefäß eingeleitet. Dabei wurde die Badtemperatur bei -78 °C gehalten. Anschließend wurde das Kältebad entfernt die Reaktionsmischung langsam auf Raumtemperatur erwärmt und filtriert um Paraformaldehydrückstände zu entfernen. Das Filtrat wurde in gesättigte wässrige Ammoniumchloridlösung gegeben und mit Diethylether (3 x 250 ml) extrahiert. Die vereinigten organischen Phasen wurden mit Magnesiumsulfat getrocknet und das Lösungsmittel anschließend am Vakuum entfernt. Das entstandene gelbliche Öl wurde mittels Flashchromatographie (Essigester/ MeCl₂ (10/ 90)) gereinigt. Nach Entfernung des Lösungsmittelgemisches wurde ein farbloses Öl erhalten. Ausbeute: 1,2 g, (25 %). DC: Rf = 0,3 (MeCl₂). Elementaranalyse C₃₃H₄₁NO₅: gefunden C: 74,5; H: 7,6; N: 2,8; berechnet: C: 74,6; H: 7,8; N: 2,6.

### t-Butul-2-trityl-4-carbo-t-butyloxy-5-toluoylsulfonsäure-pentanoat

t-Butyl-2-trityl-4-carbo-t-butyloxy-5-hydroxy-heptanoat (532 mg, 1,00 mmol) wurde in MeCl₂ (6 ml) und Pyridin (1,2 ml) gelöst. Anschließend wurden *p*-Toluolsulfonylchlorid (118 mg, 0,62 mmol) und Dimethylaminopyridin (13,4 mg, 0,11 mmol) zugegeben und die Reaktionsmischung wurde unter Stickstoffatmosphäre über Nacht bei Raumtemperatur gerührt. Die Reaktionsmischung wurde mit Essigester (3 x) extrahiert, die vereinigten organischen Phasen über Natriumsulfat getrocknet, filtriert und die Lösungsmittel am Rotationsverdampfer entfernt. Das Rohprodukt wurde in wenig MeCl₂ gelöst, auf NH₂-Material aufgezogen und über Säulenchromatographie in Essigester/ Hexan (8:2) gereinigt. Ausbeute: 339 mg, (70 %). Elementaranalyse C₄₀H₄₇NO₇: gefunden C: 70,3; H: 7,1; N: 2,2; S: 5,0 berechnet: C: 70,1; H: 6,9; N: 2,0; S: 4,7.

### t-Butyl-2-trityl-4-carbo-t-butyloxy-5-fluoro-pentanoat (HPLC-Referenz)

Wasserfreies Tetrabutylammoniumfluorid (102 mg, 0.4 mmol) wurde zu einer Lösung von t-Butyl-2-trityl-4-carbo-t-butyloxy-5-toluylsulfonsäure-(2S)-heptanoat (54 mg, 0,08 mmol) in wasserfreiem THF (3 ml) gegeben. Die Mischung wurde 4 h am Rückfluß erhitzt. Nach dem die Reaktionsmischung auf Raumtemperatur abgekühlt war, wurde die Mischung mit MeCl₂ versetzt und wässrig extrahiert (3 x). Präparative Dünnschichtchromatographie (MeCl₂/ MeOH (90/10)) lieferte das Produkt als gelbliches Öl. Ausbeute: 22 mg, (51 %). Elementaranalyse C₃₃H₄₀NO₄: gefunden C: 74,4; H: 7,7; N: 2,8; berechnet: C: 74,3; H: 7,6; N: 2,6.

### [F-18]-t-Butyl-2-trityl-4-carbo-t-butyloxy-5-fluoro-gentanoat

[F-18]Fluorid wurde über die [O-18](p,n)[F-18]-Reaktion in einem Zyklotron hergestellt. Die Isotopenlösung wurde auf eine Sep-Pack Light QMA-Kartusche gegeben und im Luftstrom getrocknet. Das [F-18]Fluorid wurde mit einer Kryptofix 2.2.2/ K₂CO₃-Lösung (22 mg K2.2.2, 4,6 mg K₂CO₃, 2 ml, MeCN (1,77 ml), Wasser (0,23 ml)) von der Kartusche eluiert. Die Lösungsmittel wurden bei 120 °C in einem Argonstrom entfernt. Der Rückstand wurde wurde mit 1 ml wasserfreien MeCN zweimal bei 120 °C in einem Argonstrom azeotrop destilliert. Eine Lösung des Tosylatpräkursors t-Butyl-2-trityl-4-carbo-t-butyloxy-5-toluoylsulfonsäure-(2S)-heptanoat (4 mg) in MeCN (0,2 ml) wurde zu einem Gefäß mit dem getrockneten [F-18]Fluorid gegeben. Die Reaktionsmischung wurde für 10 min. auf 120 °C erhitzt. Anschließend wurde das Lösungsmittel in einem Argonstrom entfernt. Das Lösungsmittel (MeCN) wurde in einem Stickstoffstrom entfernt und der Rückstand mit HCl (6 N, 0.3 - 0.5mL) bei 140 °C für 5 min. behandelt. Eine Probe der resultierenden Reaktionsmischung wurde mittels Radio-DC (Kieselgel, Eluent: n-Butanol/Essigsäure/Wasser (12/3/5) analysiert.
Die DC-Analyse wurde auf einem MiniGita DC-Scanner (Raytest, Gemany) durchgeführt.

Vorreinigung: Das Rohprodukt nach der HCl-Behandlung (vorheriger Schritt) wurde in 1 mL Wasser aufgenommen und auf eine Anionenaustauscherkartusche (Waters, SAX-OH form) gegeben. 80 % der radioaktiven Produkte wurden auf der Kartusche zurückgehalten. Die radioaktiven Produkte wurden mittels wässriger Kochsalzlösung NaCl (0.4 M, 2 ml) von der Kartusche eluiert. Eine Probe wurde mittels HPLC analysiert.

Identifikation durch Radio-HPLC. Pumpe: Gilson 305, Injector: Rheodyne (20 µL Säule: Zorbax -NH₂; 4.6 x 150 mm, mobile Phase: NaH₂PO₄ (10 mM)/ Phosphorsäure, pH 3, Fluß: 1 ml/ min, UV-Absorptionsdetektor: Gilson 116 in Serie mit einem Beckman 170 Radiodetektor, UV-Detektion: 210 nm. Rₜ: F-19-Referenz ((rac)-4-F-Glu-hydrochlorid): 14.53 min (UV); Radioaktivitätsdetektion (Beckman): 14,68 min. Ein einzelner radioactiver Peak wurde erhalten, welcher mit der Referenzverbindung ko-eluierte.

HPLC-Reinigung: Pumpe: Gilson 305, Injektor: Rheodyne (20 µL Säule: Zorbax -NH₂; 4.6 x 150 mm, mobile Phase: NaH₂PO₄ (10 mM)/ Phosphorsäure, pH 3, Fluß: 1 ml/ min, UV-Absorptionsdetektor: Gilson 116 in Serie mit einem Beckman 170 Radiodetektor, UV-Detektion: 210 nm. Ein einzelner radioactiver Peak wurde erhalten, weicher mit der Referenzverbindung ko-eluierte. Wird das entstandene Produkt über HPLC aufgereinigt, kann auf die zu vor beschriebene Vorreinigung mittels Anionenaustauscherkartusche verzichtet werden.
Das Produkt konnte mit einer radiochemischen Reinheit von > 90 % und einer Radioaktivität von 20 - 200 mCi (zerfallskorrigiert) erhalten werden.

### Beispiel 5

### Synthese von 4-Methansulfonyloxy-5-oxo-pyrrolidin-1,2-dicarbonsäure-1-tert-butylester-2-methylester (8a) (nach N. Sharma et al. Tetrahedron Lett. 2004, 45, 1403-1406.)

Zu 5.78 g (17.9 mmol) Boc-gamma-MsO-prolin methylester in 230 mL Essigsäureethylester wurde eine Lösung von 15.29 g (71.5 mmol) Natriumperiodat und 0.18 g (0.87 mmol) Ruthenium(III)-chlorid-Hydrat in 230 mL Wasser gegeben. Die Mischung wurde unter kräftigem Rühren drei Tage bei Raumtemperatur belassen. Anschließend wurden die Phasen getrennt, die wässrige Phase wurde zweimal mit Essigsäureethylester (80 mL) extrahiert und die vereinigten organischen Phasen wurden mit 50 mL Isopropanol 30 min gerührt. Es wurde über Magnesiumsulfat getrocknet, filtriert und das Lösungsmittel wurde im Vakuum entfernt. Das Rohprodukt wurde durch Säulenchromatographie (Kieselgel, Hexan/Essigsäureethylester = 6.5:3.5 bis 5:5) gereinigt. Es wurden 1.29 g (20 %) 4-Methansulfonyloxy-5-oxo-pyrrolidin-1,2-dicarbonsäure-1-tert-butylester-2-methylester (**8a**) als farbloser Feststoff erhalten.
Elementaranalyse C₁₂H₁₉NO₈S: gefunden C: 42,90; H: 5,68; N: 4,14; berechnet: C: 42,73; H: 5,68; N: 4,15.

### Synthese von 2-tert-Butoxycarbonylamino-4-methansulfonyloxy-pentandicarbonsäuredimethylester (7a) (nach: X. Zhang Tetrahedron Lett. 2001, 42, 5335-5338.)

600 mg (1.78 mmol) 4-Methansulfonyloxy-5-oxo-pyrrolidin-1,2-dicarbonsäure-1-tert-butylester-2-methylester wurden in 7.5 mL Dichlormethan gelöst. Es wurden 1.5 mL Methanol und 12.3 mg (0.089 mmol) Kaliumcarbonat zugegeben. Die resultierende Mischung wurde drei Stunden bei Raumtemperatur gerührt. Anschließend wurde das Lösungsmittel im Vakuum entfernt und das Rohprodukt wurde durch Säulenchromatographie (Kieselgel, Dichlormethan/Methanol = 99.7:0.3 bis 99.6:0.4) gereinigt. Es wurden 608 mg (83 %) 2-*tert*-Butoxycarbonylamino-4-methansulfonyloxy-pentandicarbonsäuredimethylester (**7a**) als farbloses Öl erhalten.
Elementaranalyse C₁₃H₂₃NO₉S: gefunden C: 42,10; H: 6,29; N: 3,69; berechnet: C: 42,27; H: 6,28; N: 3,79.

### F-18 Markierung von 2-tert-Butoxycarbonylamino-4-methansulfonyloxypentandicarbonsäuredimethylester (7a)

[F-18]Fluorid wurde über die [O-18](p,n)[F-18]-Reaktion in einem Zyklotron hergestellt. Die Isotopenlösung (2.47 GBq) wurde auf eine Sep-Pack Light QMA-Kartusche gegeben. Das [F-18]Fluorid wurde mit einer Kryptofix 2.2.2/ K₂CO₃-Lösung (5 g K2.2.2, 1mg K₂CO₃, MeCN (1,5ml), Wasser (0,5ml)) von der Kartusche eluiert. Das Lösungsmittel wurde bei 120 °C in einem Stickstoffstrom unter Zugabe von Acetonitril (dreimal 1 mL) entfernt.

5 mg (13.6 µmol) 2-*tert*-Butoxycarbonylamino-4-methansulfonyloxy-pentandicarbonsäuredimethylester (**7a**) in 1 mL Acetonitril wurden zugegeben und die resultierende Mischung wurde 10 min bei 100 °C gerührt. Nach Abkühlen auf ca. 60 °C wurde die Mischung durch eine Silica-Plus Kartusche gegeben.

Das Intermediat **6a** wurde durch HPLC (C18, Acetonitril/Wasser) gereinigt. Die HPLC-Fraktion wurde mit Wasser (ca. 50 mL) verdünnt und über eine C18-Kartusche gegeben. Das Intermediat wurde mit 1 mL Acetonitril eluiert. In einer Synthesezeit von 64 min wurden 533 MBq (34 % d.c.) 2-*tert*-Butoxycarbonylamino-4-[F-18]fluoropentandicarbonsäuredimethylester erhalten (**6a**).

### Synthese von 4-[F-18]Fluorglutaminsäure (5) durch Entschützung von 2-tert-Butoxycarbonylamino-4-[F-18]fluoro-pentandicarbonsäuredimethylester (6a)

533 MBq 2-*tert*-Butoxycarbonylamino-4-[F-18]fluoro-pentandicarbonsäuredimethylester (**6a**) in 1 mL Acetonitril wurden mit 0.5 mL 4N HCl versetzt. die Mischung wurde für 5 min in einem offenen Vial unter Rühren auf 140 °C (Ölbadtemperatur) erhitzt. Weitere 0.5 mL 4N HCl wurden zugegeben und die Mischung wurde für 5 min in einem geschlossenen Vial unter Rühren auf 140 °C (Ölbadtemperatur) erhitzt.

Nach Abkühlen auf Raumtemperatur wurde die Lösung durch Zugabe von ca. 1.5 mL 2N NaOH neutralisiert.

(2-*tert*-Butoxycarbonylamino-4-[F-18]fluoro-pentandicarbonsäuredimethylester (**6a**) konnte quantitativ (d.c.) zu 4-[F-18]Fluorglutaminsäure (**5**) umgesetzt werden.

### Beispiel 6

### F-18 Markierung von 4-Methansulfonyloxy-5-oxo-pyrrolidin-1,2-dicarbonsäure-1-tert-butylester-2-methylester (8a)

[F-18]Fluorid wurde über die [O-18](p,n)[F-18]-Reaktion in einem Zyklotron hergestellt. Die Isotopenlösung (3.27 GBq) wurde auf eine Sep-Pack Light QMA-Kartusche gegeben. Das [F-18]Fluorid wurde mit einer Kryptofix 2.2.2/ K₂CO₃-Lösung (5 g K2.2.2, 1mg K₂CO₃, MeCN (1,5ml), Wasser (0,5ml)) von der Kartusche eluiert. Das Lösungsmittel wurde bei 120 °C in einem Stickstoffstrom unter Zugabe von Acetonitril (dreimal 1 mL) entfernt.

5 mg (14.9 µmol) 4-Methansulfonyloxy-5-oxo-pyrrolidin-1,2-dicarbonsäure-1-tert-butylester-2-methylester (**8a**) in 1 mL Acetonitril wurden zugegeben und die resultierende Mischung wurde 10 min bei 100 °C gerührt. Nach Abkühlen auf ca. 60 °C wurde die Mischung durch eine Silica-Plus Kartusche gegeben.

Das Intermediat wurde durch HPLC (C18, Acetonitril/Wasser) gereinigt. Die HPLC-Fraktion wurde mit Wasser (ca. 50 mL) verdünnt und über eine C18-Kartusche gegeben. Das Intermediat wurde mit 1 mL Acetonitril eluiert. In einer Synthesezeit von 95 min wurden 421 MBq (23 % d.c.) 4-[F-18]Fluoro-5-oxo-pyrrolidin-1,2-dicarbonsäure-1-tert-butylester-2-methylester erhalten.

### Synthese von 4-[F-18]Fluorglutaminsäure (5) durch Entschützung von 4-[F-18]Fluoro-5-oxo-pyrrolidin-1,2-dicarbonsäure-1-tert-butylester-2-methylester

221 MBq 4-[F-18]Fluoro-5-oxo-pyrrolidin-1,2-dicarbonsäure-1-tert-butylester-2-methylester enthalten in 0.5 mL Acetonitril wurden mit 0.5 mL 6N HCl versetzt. Die Mischung wurde für 10 min unter Rühren auf 130 °C (Ölbadtemperatur) erhitzt. Nach Abkühlen auf Raumtemperatur wurde die Lösung durch Zugabe von ca. 600 µL 4N NaOH neutralisiert. Es wurden 172 MBq (91 % d.c.) 4-[F-18]Fluorglutaminsäure (**5**) erhalten.

### Beispiel 7

### Synthese von 4-[2-(Toluol-4-sulfonyloxy)-ethoxy]-pyrrolidin-1,2-dicarbonsäure-1-tert-butylester-2-methylester

Zu einer Suspension von 0.65 g (15 mmol) Natriumhydrid in DMF (20 mL) wurde eine Lösung von 2.45 g (10.0 mmol) 4-[Hydroxy]-pyrrolidin-1,2-dicarbonsäure-1-tert-butylester-2-methylester (**24**) in DMF (10 mL) gegeben. Nach 15 min wurde eine Lösung von 5.56 g (15.0 mmol) 1,2-Ethandiol-bis-tosylat in DMF (10 mL) zugegeben. Der Ansatz wurde anschließend in 3 Portionen 45 Minuten bei 100°C in der Mikrowelle umgesetzt. Der Ansatz wurde eingeengt und mit Wasser und Essigsäureethylester versetzt. Nach Phasentrennung wurde die wässrige Phase mit Essigsäureethylester extrahiert. Die vereinigten organischen Phasen wurde über Natriumsulfat getrocknet, filtriert und das Lösungsmittel wurde im Vakuum entfernt. Das Rohprodukt wurde durch Säulenchromatographie (Kieselgel, Hexan/Essigsäureethylester) gereinigt. Es wurden 1.2 g (27 %) 4-[2-(Toluol-4-sulfonyloxy)-ethoxy]-pyrrolidin-1,2-dicarbonsäure-1-tert-butylester-2-methylester erhalten.
Elementaranalyse C₂₀H₂₉NO₈S: gefunden C: 54,20; H: 6,66; N: 3,23; berechnet: C: 54,16; H: 6,59; N: 3,16.

### Synthese von 5-Oxo-4-[2-(toluol-4-sulfonyloxy)-ethoxy]-pyrrolidin-1,2-dicarbonsäure-1-tert-butylester-2-methylester (23)

Zu 0.44 g (1 mmol) 4-[2-(Toluol-4-sulfonyloxy)-ethoxy]-pyrrolidin-1,2-dicarbonsäure-1-tert-butylester-2-methylester in 20 mL Dichlormethan wurde eine Lösung von 1.07 g (5.0 mmol) Natriumperiodat und 0.338 g (0.15 mmol) Ruthenium(III)-chlorid-Hydrat in 12.5 mL Wasser gegeben. Die Mischung wurde unter kräftigem Rühren drei Tage bei Raumtemperatur belassen. Anschließend wurden die Phasen getrennt, die wässrige Phase wurde zweimal mit Essigsäureethylester (20 mL) extrahiert und die vereinigten organischen Phasen wurden mit 5 mL Isopropanol 30 min gerührt. Es wurde über Magnesiumsulfat getrocknet, filtriert und das Lösungsmittel wurde im Vakuum entfernt. Das Rohprodukt wurde durch Säulenchromatographie (Kieselgel, Hexan/Essigsäureethylester) gereinigt. Es wurden 0.11 g (24 %) 5-Oxo-4-[2-(toluol-4-sulfonyloxy)-ethoxy]-pyrrolidin-1,2-dicarbonsäure-1-tert-butylester-2-methylester (**23**) als farbloses Öl erhalten.
Elementaranalyse C₂₀H₂₇NO₉S: gefunden C: 52,37; H: 6,02; N: 3,11; berechnet: C: 52,51; H: 5,95; N: 3,06.

### Synthese von 2-tert-Butoxycarbonylamino-4-[2-(toluol-4-sulfonyloxy)-ethoxy]-pentanedicarbonsäuredimethylester (25)

100 mg (0.22 mmol) 5-Oxo-4-[2-(toluol-4-sulfonyloxy)-ethoxy]-pyrrolidin-1,2-dicarbonsäure-1-tert-butylester-2-methylester wurden in 3 mL Dichlormethan gelöst. Es wurden 1 mL Methanol und 6 mg (0.04 mmol) Kaliumcarbonat zugegeben. Die resultierende Mischung wurde drei Stunden bei Raumtemperatur gerührt. Anschließend wurde das Lösungsmittel im Vakuum entfernt und das Rohprodukt wurde durch Säulenchromatographie (Kieselgel, Dichlormethan/Methanol). Es wurden 97 mg (91 %) 2-tert-Butoxycarbonylamino-4-[2-(toluol-4-sulfonyloxy)-ethoxy]-pentanedicarbonsäuredimethylester (**25**) als farbloses Öl erhalten. Elementaranalyse C₂₁H₃₁NO₁₀S: gefunden C: 51.48; H: 6.36; N: 2,88; berechnet: C: 51.52; H: 6,38; N: 2,86.

**F-18 Markierung von 2-tert-Butoxycarbonylamino-4-[2-(toluol-4-sulfonyloxy)-ethoxy]-pentanedicarbonsäuredimethylester (25)** [F-18]Fluorid wurde über die [O-18](p,n)[F-18]-Reaktion in einem Zyklotron hergestellt. Die Isotopenlösung (1.57 GBq) wurde auf eine Sep-Pack Light QMA-Kartusche gegeben. Das [F-18]Fluorid wurde mit einer Kryptofix 2.2.2/ K₂CO₃-Lösung (5 g K2.2.2, 1mg K₂CO₃, MeCN (1,5ml), Wasser (0,5ml)) von der Kartusche eluiert. Das Lösungsmittel wurde bei 120 °C in einem Stickstoffstrom unter Zugabe von Acetonitril (dreimal 1 mL) entfernt.

5 mg (10.2 µmol) 2-tert-Butoxycarbonylamino-4-[2-(toluol-4-sulfonyloxy)-ethoxy]-pentanedicarbonsäuredimethylester (**25**) in 1 mL Acetonitril wurden zugegeben und die resultierende Mischung wurde 10 min bei 100 °C gerührt. Nach Abkühlen auf ca. 60 °C wurde die Mischung durch eine Silica-Plus Kartusche gegeben.

Das Intermediat wurde durch HPLC (C18, Acetonitril/Wasser) gereinigt. Die HPLC-Fraktion wurde mit Wasser (ca. 50 mL) verdünnt und über eine C18-Kartusche gegeben. Das Intermediat wurde mit 1 mL Acetonitril eluiert. In einer Synthesezeit von 78 min wurden 337 MBq (35 % d.c.) 2-tert-Butoxycarbonylamino-4-(2-[F-18]fluorethoxy)-pentandicarbonsäuredimethylester erhalten (**22**).

### Synthese von 2-Amino-4-(2-[F-18]fluorethoxy)-pentandicarbonsäure (20) durch Entschützung von 2-tert-Butoxycarbonylamino-4-(2-[F-18]fluorethoxy)-pentandicarbon-säuredimethyl-ester (22)

337 MBq 2-tert-Butoxycarbonylamino-4-(2-[F-18]fluorethoxy)-pentandi-carbonsäuredimethylester (**22**) in 1 mL Acetonitril wurden mit 0.5 mL 4N HCl versetzt. Die Mischung wurde für 10 min unter Rühren auf 130 °C (Ölbadtemperatur) erhitzt. Nach Abkühlen auf Raumtemperatur wurde die Lösung durch Zugabe von ca. 700 µL 2N NaOH neutralisiert. Es wurden 288 MBq (98 % d.c.) 2-Amino-4-(2-[F-18]fluorethoxy)-pentandicarbonsäure (**20**) erhalten.

### Synthese von 4-(2-Fluorethoxy)-pyrrolidin-1,2-dicarbonsäure 1-tert-butylester 2-methylester

Zu einer Suspension von 0.65 g (15 mmol) Natriumhydrid in DMF (20 mL) wurde eine Lösung von 2.45 g (10.0 mmol) 4-[Hydroxy]-pyrrolidin-1,2-dicarbonsäure-1-tert-butylester-2-methylester (**24**) in DMF (10 mL) gegeben. Nach 15 min wurde eine Lösung von 1.90 g (15.0 mmol) 1-Brom-2-fluorethan in DMF (10 mL) zugegeben. Der Ansatz wurde anschließend in 3 Portionen 45 Minuten bei 100°C in der Mikrowelle umgesetzt. Der Ansatz wurde eingeengt und mit Wasser und Essigsäureethylester versetzt. Nach Phasentrennung wurde die wässrige Phase mit Essigsäureethylester extrahiert. Die vereinigten organischen Phasen wurde über Natriumsulfat getrocknet, filtriert und das Lösungsmittel wurde im Vakuum entfernt. Das Rohprodukt wurde durch Säulenchromatographie (Kieselgel, Hexan/Essigsäureethylester) gereinigt. Es wurden 2.10 g (48 %) 4-(2-Fluorethoxy)-pyrrolidin-1,2-dicarbonsäure 1-tert-butylester 2-methylester erhalten.
Elementaranalyse C₁₃H₂₂FNO₅: gefunden C: 53,48; H: 7,70; N: 4,85; berechnet: C: 53,60; H: 7,61; N: 4,81.

### 4-(2-Fluorethoxy)-5-oxo-pyrrolidin-1,2-dicarbonsäure-1-tert-butylester-2-methylester (26)

Zu 1.45 g (5 mmol) 4-(2-Fluorethoxy)-pyrrolidin-1,2-dicarbonsäure 1-tert-butylester 2-methylester in 80 mL Dichlormethan wurde eine Lösung von 4,3 g (20.0 mmol) Natriumperiodat und 1,7 g (0.6 mmol) Ruthenium(III)-chlorid-Hydrat in 50 mL Wasser gegeben. Die Mischung wurde unter kräftigem Rühren drei Tage bei Raumtemperatur belassen. Anschließend wurden die Phasen getrennt, die wässrige Phase wurde zweimal mit Essigsäureethylester (25 mL) extrahiert und die vereinigten organischen Phasen wurden mit 10 mL Isopropanol 30 min gerührt. Es wurde über Natriumsulfat getrocknet, filtriert und das Lösungsmittel wurde im Vakuum entfernt. Das Rohprodukt wurde durch Säulenchromatographie (Kieselgel, Hexan/Essigsäureethylester) gereinigt. Es wurden 0.26 g (17 %) 4-(2-Fluorethoxy)-5-oxo-pyrrolidin-1,2-dicarbonsäure-1-tert-butylester-2-methylester (**26**) als erhalten.
Elementaranalyse C₁₃H₂₀FNO₆: gefunden C: 51,18; H: 6,55; N: 3,54; berechnet: C: 51,14; H: 6.,60; N: 4,59.

### 2-tert-Butoxycarbonylamino-4-(2-fluorethoxy)-pentanedicarbonsäuredimethylester (27)

150 mg (0.49 mmol) 4-(2-Fluorethoxy)-5-oxo-pyrrolidin-1,2-dicarbonsäure-1-tert-butylester-2-methylester (**26**) wurden in 5 mL Dichlormethan gelöst. Es wurden 2 mL Methanol und 6 mg (0.04 mmol) Kaliumcarbonat zugegeben. Die resultierende Mischung wurde drei Stunden bei Raumtemperatur gerührt. Anschließend wurde das Lösungsmittel im Vakuum entfernt und das Rohprodukt wurde durch Säulenchromatographie (Kieselgel, Dichlormethan/Methanol). Es wurden 145 mg (88 %) 2-tert-Butoxycarbonylamino-4-(2-fluorethoxy)-pentanedicarbonsäuredimethylester (**27**) erhalten.
Elementaranalyse C₁₄H₂₄FNO₇: gefunden C: 40,06; H: 7,11; N: 4,12; berechnet: C: 49,85; H: 7,17; N: 4,15

### 2-Amino-4-(2-fluorethoxy)-pentandicarbonsäure (28)

100 mg 3-tert-Butoxycarbonylamino-4-(2-fluorethoxy)-pentanedicarbonsäuredimethylester (**27**) wurden in 50 ml MeOH gelöst und unter Rühren mit 1 ml HCl (6N) versetzt. Die Reaktionsmischung wurde für 15-20 min. am Rückfluß erhitzt, zur Trockene eingeengt, mit 50 ml Wasser verdünnt, das Hydrochlorid abfiltriert und vorsichtig mit 3 × 5 ml Wasser gewaschen. Die Aminosäure **28** wurde mittels loneneaustauschchromatographie über eine Dowex-Säule 50w×8 in H⁺-Form (Eluent: 5% NH₃ aq) isoliert.

### Beispiel 8

### Synthese von 2-(3-Brompropyl)-4-tert-butoxycarbonylamino-pentandicarbonsäuredimethylester (33) (nach: S. Hanessian, et al. J. Org. Chem. 2005, 70, 5070-5085.)

Zu einer Lösung von 1.00 g (3.63 mmol) N-Boc-glutaminsäuredimethylester (**26**) in trockenem THF (20 mL) wurde bei -78 C LiHMDS (7.8 mL, 1M Lösung in THF) zugegeben. die resultierende Mischung wurde 45 min bei -78 °C gerührt. Anschließend wurde bei -78 °C langsam eine Lösung von 1.10 g (5.45 mmol) 1,3-Dibrompropan in THF (10 mL) zugetropft. Die Mischung wurde 60 min gerührt. Es wurde durch Zugabe von Ammoniumchloridlösung gequenchet, auf RT aufgewärmt und mit Dichlormethan extrahiert. Die vereinigten organischen Phasen wurden mit gesättigter Natriumchlordlösung gewaschen und über Natriumsulfat getrocknet. Das Lösungsmittel wurde im Vakuum entfernt und das Rohprodukt wurde durch Säulenchromatographie (Kieselgel, Essigsäureethylester/Hexan 10:90 bis 40:60) gereinigt. Es wurden 0.490 g (34 %) 2-(3-Brompropyl)-4-tert-butoxycarbonylaminopentandicarbonsäure-dimethylester (**33**) als farbolses Öl erhalten. Elementaranalyse C15H26BrNO6: gefunden C: 45,21; H: 6,53; N: 3,60; berechnet: C: 45,46; H: 6,61; N: 3,53.

### F-18 Markierung von 2-(3-Brompropyl)-4-tert-butoxycarbonylamino-pentandicarbonsäuredimethylester (33)

[F-18]Fluorid wurde über die [O-18](p,n)[F-18]-Reaktion in einem Zyklotron hergestellt. Die Isotopenlösung (1.33 GBq) wurde auf eine Sep-Pack Light QMA-Kartusche gegeben. Das [F-18]Fluorid wurde mit einer Kryptofix 2.2.2/ K₂CO₃-Lösung (5 g K2.2.2, 1mg K₂CO₃, MeCN (1,5ml), Wasser (0,5ml)) von der Kartusche eluiert. Das Lösungsmittel wurde bei 120 °C in einem Stickstoffstrom unter Zugabe von Acetonitril (dreimal 1 mL) entfernt.

5 mg (12.6 µmol) 2-(3-Brompropyl)-4-tert-butoxycarbonylamino-pentandicarbonsäuredimethylester (**33**) in 1 mL Acetonitril wurden zugegeben und die resultierende Mischung wurde 10 min bei 100 °C gerührt. Nach Abkühlen auf ca. 60 °C wurde die Mischung durch eine Silica-Plus Kartusche gegeben.

Das Intermediat wurde durch HPLC (C18, Acetonitril/Wasser) gereinigt. Die HPLC-Fraktion wurde mit Wasser (ca. 50 mL) verdünnt und über eine C18-Kartusche gegeben. Das Intermediat wurde mit 1 mL Acetonitril eluiert. In einer Synthesezeit von 90 min wurden 346 MBq (46 % d.c.) 2-tert-Butoxycarbonylamino-4-(3-[F-18]fluoropropyl)-pentandicarbonsäuredimethylester (**31**) erhalten.

### Synthese von 2-Amino-4-(3-[F-18]fluorpropyl)-pentanedicarbonsäure (29) durch Entschützung von 2-tert-Butoxycarbonylamino-4-(3-[F-18]fluoropropyl)-pentandicarbonsäuredimethylester (31)

346 MBq 2-tert-Butoxycarbonylamino-4-(3-[F-18]fluoropropyl)-pentandicarbonsäuredimethyl-ester (**3**) in 1 mL Acetonitril wurden mit 0.5 mL 4N HCl versetzt. Die Mischung wurde für 10 min unter Rühren auf 130 °C (Ölbadtemperatur) erhitzt. Nach Abkühlen auf Raumtemperatur wurde die Lösung durch Zugabe von ca. 650 µL 2N NaOH neutralisiert.

Es wurden 288 MBq (96 % d.c.) 2-Amino-4-(3-[F-18]fluorpropyl)-pentanedicarbonsäure (**29**) erhalten.

### Synthese von 2-tert-Butoxycarbonylamino-4-(3-fluoropropyl)-pentandicarbonsäuredimethylester (36) (nach: S. Hanessian, et al. J. Org. Chem. 2005, 70, 5070-5085.)

Zu einer Lösung von 1.00 g (3.63 mmol) N-Boc-glutaminsäuredimethylester (**26**) in trockenem THF (20 mL) wurde bei -78 C LiHMDS (7.8 mL, 1M Lösung in THF) zugegeben. die resultierende Mischung wurde 45 min bei -78 °C gerührt. Anschließend wurde bei -78 °C langsam eine Lösung von 0.77 g (5.45 mmol) 1-Brom-3-fluorpropan in THF (10 mL) zugetropft. Die Mischung wurde 60 min gerührt. Es wurde durch Zugabe von Ammoniumchloridlösung gequenchet, auf RT aufgewärmt und mit Dichlormethan extrahiert. Die vereinigten organischen Phasen wurden mit gesättigter Natriumchlordlösung gewaschen und über Natriumsulfat getrocknet. Das Lösungsmittel wurde im Vakuum entfernt und das Rohprodukt wurde durch Säulenchromatographie (Kieselgel, Essigsäureethylester/Hexan 10:90 bis 40:60) gereinigt. Es wurden 0.318 g (29 %) 2-tert-Butoxycarbonylamino-4-(3-fluoropropyl)-pentandicarbonsäuredimethylester (**36**) erhalten. Elementaranalyse C₁₅H₂₆FNO₆: gefunden C: 53,88; H: 7.87; N: 4,13; berechnet: C: 53,72; H: 7.81; N: 4,18.

### 2-Amino-4-(3-fluorpropyl)-pentandicarbonsäure (38)

200 mg 2-tert-Butoxycarbonylamino-4-(3-fluoropropyl)-pentandicarbonsäuredimethylester (**36**) wurden in 75 ml MeOH gelöst und unter Rühren mit 1.5 ml HCl (6N) versetzt. Die Reaktionsmischung wurde für 15-20 min. am Rückfluß erhitzt, zur Trockene eingeengt, mit 50 ml Wasser verdünnt, das Hydrochlorid abfiltriert und vorsichtig mit 3 × 10 ml Wasser gewaschen. Die Aminosäure **38** wurde mittels loneneaustauschchromatographie über eine Dowex-Säule 50w×8 in H⁺-Form (Eluent: 5% NH₃ aq) isoliert.

### Beispiel 9

### Synthese von 2-tert-Butoxycarbonylamino-4-[4-(toluol-4-sulfonyloxy)-butyl]-pentandicarbonsäuredimethylester (34) (nach: S. Hanessian, et al. J. Org. Chem. 2005, 70, 5070-5085.)

Zu einer Lösung von 1.00 g (3.63 mmol) N-Boc-glutaminsäuredimethylester (**35**) in trockenem THF (20 mL) wurde bei -78 C LiHMDS (7.8 mL, 1M Lösung in THF) zugegeben. die resultierende Mischung wurde 45 min bei -78 °C gerührt. Anschließend wurde bei -78 °C langsam eine Lösung von 2.17 g (5.45 mmol) 1,4-Butandiol-ditosylat in THF (10 mL) zugetropft. Die Mischung wurde 60 min gerührt. Es wurde durch Zugabe von Ammoniumchloridlösung gequenchet, auf RT aufgewärmt und mit Dichlormethan extrahiert. Die vereinigten organischen Phasen wurden mit gesättigter Natriumchlordlösung gewaschen und über Natriumsulfat getrocknet. Das Lösungsmittel wurde im Vakuum entfernt und das Rohprodukt wurde durch Säulenchromatographie (Kieselgel, Essigsäureethylester/Hexan 10:90 bis 20:80) gereinigt. Es wurden 0.418 g (23 %) 2-tert-Butoxycarbonylamino-4-[4-(toluol-4-sulfonyloxy)-butyl]-pentan-dicarbonsäuredimethylester (**34**) erhalten.
Elementaranalyse C₂₃H₃₅NO₉S: gefunden C: 54,9; H: 7,1; N: 2,7; berechnet: C: 55,07; H: 7,03; N: 2,79.

### F-18 Markierung von 2-tert-Butoxycarbonylamino-4-[4-(toluol-4-sulfonyloxy)-butyl]-pentan-dicarbonsäuredimethylester (34)

[F-18]Fluorid wurde über die [O-18](p,n)[F-18]-Reaktion in einem Zyklotron hergestellt. Die Isotopenlösung (3.08 GBq) wurde auf eine Sep-Pack Light QMA-Kartusche gegeben. Das [F-18]Fluorid wurde mit einer Kryptofix 2.2.2/ K₂CO₃-Lösung (5 g K2.2.2, 1mg K₂CO₃, MeCN (1,5ml), Wasser (0,5ml)) von der Kartusche eluiert. Das Lösungsmittel wurde bei 120 °C in einem Stickstoffstrom unter Zugabe von Acetonitril (dreimal 1 mL) entfernt.

5 mg (10.0 µmol) 2-tert-Butoxycarbonylamino-4-[4-(toluol-4-sulfonyloxy)-butyl]-pentandicarbonsäuredimethylester (**34**) in 1 mL Acetonitril wurden zugegeben und die resultierende Mischung wurde 10 min bei 100 °C gerührt. Nach Abkühlen auf ca. 60 °C wurde die Mischung durch eine Silica-Plus Kartusche gegeben.

Das Intermediat wurde durch HPLC (C18, Acetonitril/Wasser) gereinigt. Die HPLC-Fraktion wurde mit Wasser (ca. 50 mL) verdünnt und über eine C18-Kartusche gegeben. Das Intermediat wurde mit 1 mL Acetonitril eluiert. In einer Synthesezeit von 92 min wurden 812 MBq (48 % d.c.) 2-tert-Butoxycarbonylamino-4-(4-[F-18]fluorobutyl)-pentandicarbonsäuredimethylester (**32**) erhalten.

### Synthese von 2-Amino-4-(4-fluorbutyl)-pentandicarbonsäure (30) durch Entschützung von 2-tert-Butoxycarbonylamino-4-(4-[F-18]fluorobutyl)-pentandicarbonsäuredimethyl-ester (32)

812 MBq 2-tert-Butoxycarbonylamino-4-(4-[F-18]fluorobutyl)-pentandicarbonsäuredimethylester (**32**) in 1 mL Acetonitril wurden mit 0.5 mL 4N HCl versetzt. Die Mischung wurde für 10 min unter Rühren auf 130 °C (Ölbadtemperatur) erhitzt. Nach Abkühlen auf Raumtemperatur wurde die Lösung durch Zugabe von ca. 700 µL 2N NaOH neutralisiert. Es wurden 691 MBq (97 % d.c.) 2-Amino-4-(4-fluorbutyl)-pentanedicarbonsäure (**30**)

### Synthese von 2-tert-Butoxycarbonylamino-4-(4-fluorobutyl)-pentandicarbonsäuredimethylester (37) (nach: S. Hanessian, et al. J. Org. Chem. 2005, 70, 5070-5085.)

Zu einer Lösung von 1.00 g (3.63 mmol) N-Boc-glutaminsäuredimethylester (**26**) in trockenem THF (20 mL) wurde bei -78 C LiHMDS (7.8 mL, 1M Lösung in THF) zugegeben. die resultierende Mischung wurde 45 min bei -78 °C gerührt. Anschließend wurde bei -78 °C langsam eine Lösung von 0.84 g (5.45 mmol) 1-Brom-3-fluorpropan in THF (10 mL) zugetropft. Die Mischung wurde 60 min gerührt. Es wurde durch Zugabe von Ammoniumchloridlösung gequenchet, auf RT aufgewärmt und mit Dichlormethan extrahiert. Die vereinigten organischen Phasen wurden mit gesättigter Natriumchlordlösung gewaschen und über Natriumsulfat getrocknet. Das Lösungsmittel wurde im Vakuum entfernt und das Rohprodukt wurde durch Säulenchromatographie (Kieselgel, Essigsäureethylester/Hexan 10:90 bis 40:60) gereinigt. Es wurden 0.596 g (47 %) 2-tert-Butoxycarbonylamino-4-(3-fluorobutyl)-pentandicarbonsäuredimethylester (**37**) erhalten. Elementaranalyse C₁₆H₂₈FNO₆: gefunden C: 54,94; H: 8.01; N: 4,04; berechnet: C: 55,00; H: 8,08; N: 4,01.

### 2-Amino-4-(4-fluorpropyl)-pentandicarbonsäure (39)

300 mg 2-tert-Butoxycarbonylamino-4-(4-fluorobutyl)-pentandicarbonsäuredimethylester (**37**) wurden in 100 ml MeOH gelöst und unter Rühren mit 5 ml HCl (6N) versetzt. Die Reaktionsmischung wurde für 15-20 min. am Rückfluß erhitzt, zur Trockene eingeengt, mit 100 ml Wasser verdünnt, das Hydrochlorid abfiltriert und vorsichtig mit 3 × 25 ml Wasser gewaschen. Die Aminosäure **38** wurde mittels Ioneneaustauschchromatographie über eine Dowex-Säule 50w×8 in H⁺-Form (Eluent: 5% NH₃ aq) isoliert.

### Beispiel 10

### Biologische Charakterisierung:

Zur Evaluierung der Tumorzellaufnahme von [¹⁸F]Glutaminsäure wurden Zellaufnahme experimente in Humanen A549 (Nichtkleinzelliges Bronchialkarzinom) und HT29 (Kolonkarzinom) Zellen durchgeführt. Die Tumorzellaufnahme der Glutaminsäurederivate wurde zu [¹⁸F]FDG (Goldstandard für onkologische PET-Untersuchungen) verglichen. Die Ergebnisse sind in Abbildung 1 dargestellt.

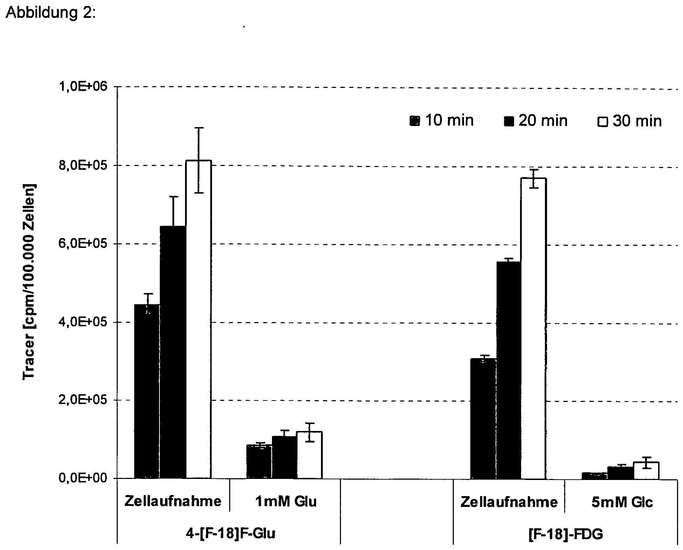

Abbildung 2: Vergleich der zeitabhängigen Tumorzellaufnahme von [¹⁸F]-4-Glutaminsäure [20 - 35 µM] (links) und [F-18]FDG [2 µM] (rechts) in A549 Zellen. Die Zellen wurden mit 250 kBq/ well inkubiert. Für Verdrängungsexperimente wurde L-Glutaminsäure (1 mM) beziehungsweise Glukose (5 mM) verwendet (Mittelwert ± Standardabweichung, n = 3).

Die überraschend hohe Aufnahme von [¹⁸F]-4-Glutaminsäure in A549 und HT29 Tumorzellen zeigen, dass diese fluorierten Glutaminsäurederivate Potential für die Tumordarstellung im Sinne der Erfindung bieten sollten.
Analoge Aufnahmeergebnisse wurden für [¹⁸F]-4-Glutamin, 2-Amino-4-(2-[F-18]fluorethoxy)-pentandicarbonsäure und 2-Amino-4-(3-[F-18]fluoropropyl)-pentandicarbonsäure erzielt.

Zur Evaluierung der Tumoranreicherung und der Gewebeverteilung in Versuchstieren wurde [¹⁸F]-4-Glutaminsäure in einem murinen F9-Teratokarzinom.Modell in NMRI-Nacktmäusen und einem murinen B16F1-Melanom-Modell in C57BI6-Mäusen getestet. Dazu wurden 1 x 10⁶ Zellen (F9) beziehungsweise 5 x 10⁵ Zellen (B16F1) in 100 µl phosphatgepufferter physiologischer Kochsalzlösung suspendiert und subkutan in der rechten, hinteren Flanke der entsprechenden Versuchstierspezies (NMRI für F9 und C57-BI6 für B16F1) inokuliert (Berndorff et al. Clin. Cancer Res. 2005, 11, 2005). Nach 14 Tagen (F9) beziehungsweise 10 Tagen (B16) erreichten die Tumore eine Größe von ca. 80 - 100 mm². [¹⁸F]-4-Glutaminsäure (370 kBq, in 100 µl physiologischer Kochsalzlösung) wurde intravenös in die Schwanzvene appliziert. Nach jeweils 15; 60 und 120 min. wurden die Tiere getötet, Organ- und Tumorgewebe entnommen, gewogen und auf radioaktiven Gehalt vermessen. Die entsprechenden Daten sind in den Tabellen 1 - 4 zusammengefasst.

Die Gewebeverteilungen von [¹⁸F]-4-Glutaminsäure wurden in den gleichen Tumormodellen mit denen von C-14 markierter Glutaminsäure verglichen, wobei 111 kBq [¹⁴C]5-Glutaminsäure intravenös appliziert wurde und die Tiere nach 30, 60 und 240 min (F9) sowie nach 15, 60 und 120 min(B16F1) getötet und analysiert wurden (Tabellen 5-8).

Im Vergleich zu diesen Ergebnissen wurde C-14 markierte Glutaminsäure in beiden Tumormodellen untersucht. Die entsprechenden Organverteilungsresultate sind in Tab. 5 - 9 abgebildet.

Überraschenderweise zeigt [¹⁸F]4-Glutaminsäure nach intravenöser Injektion (15 min.) eine maximale Tumoranreicherung von 2,90% ID/ g (F9 Teratokarzinom) beziehungsweise 3,55% ID/ g (B16 Melanom) während die maximale Tumoranreicherung zum ähnlich frühen Zeitpunkt (30 min) für das natürliche Substrat [¹⁴C]5-Glutaminsäure nur 0,81% ID/ g beim F9 Teratokarzinom und zum gleichen Zeitpunkt 1,23 beim B16F1 Melanom beträgt.

Auch 1 h nach intravenöser Applikation liegen die Werte mit 1,75% ID/ g beim F9 Teratokarzinom beziehungsweise 2,14% ID/ g beim B16F1 Melanom deutlich höher als für [¹⁴C]5-Glutaminsäure mit nur 0,98% ID/ g (F9 Teratokarzinom) beziehungsweise 1,03% ID/ g (B16F1 Melanom).
Die höhere Tumoranreicherung der fluorierten Verbindung verknüpft mit einer raschen Ausscheidung aus physiologisch, vitalem Gewebe/ Organen führt zu einem deutlich verbesserten Tumor/ Hintergrundverhältnis von [¹⁸F]4-Glutaminsäure gegenüber [¹⁴C]5-Glutaminsäure. Der Tumor/ Blutquotient im B16F1 Melanommodell liegt für die fluorierte Verbindung bei 5,3 (1 h p.i.) und 7,9 (2 h p.i.), und ist damit um den Faktor 2 beziehungsweise 3,5 höher als für [¹⁴C]5-Glutaminsäure.
Der Tumor/ Leberquotient, ein wichtiger Parameter für die Eignungsbewertung eines PET-Tracers, liegt für die fluorierte Verbindung im B16F1 Melanommodell bei 3,6 (1 h p.i.) und 4,4 (2 h p.i.), und ist damit um den Faktor 4,6 beziehungsweise 5,5 höher als für [¹⁴C]5-Glutaminsäure.
[¹⁸F]4-Glutaminsäure hat deutlich verbesserte pharmakokinetische Eigenschaften verglichen mit dem C-14 substituierten natürlichen Substrat [¹⁴C]5-Glutaminsäure.
In Abbildung 1 sind die Tumoranreicherung und die Aufnahme in ausgewählten relevanten Organen nach 1 h (a) beziehungsweise 2 h (b) dargestellt.

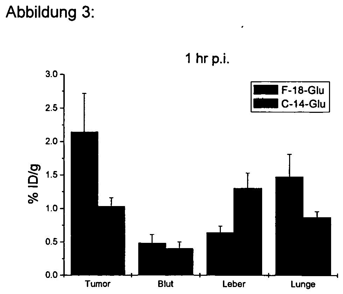

Abbildung 3 zeigt die Verteilung nach 1h.

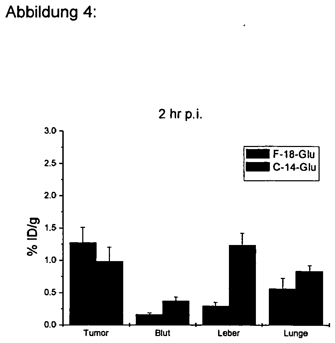

In Abbildung 4 ist die Verteilung nach 2 h dargestellt.

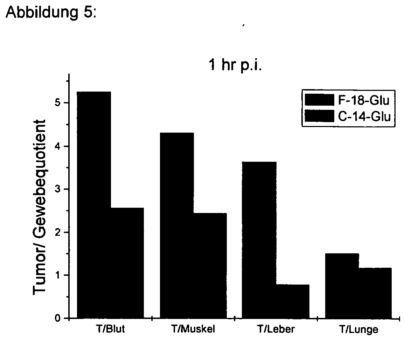

Abbildung 5 zeigt die resultierenden Gewebequotienten inklusive Tumor/ Muskelgewebe nach 1h.

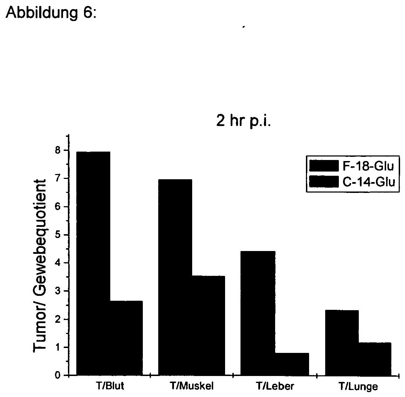

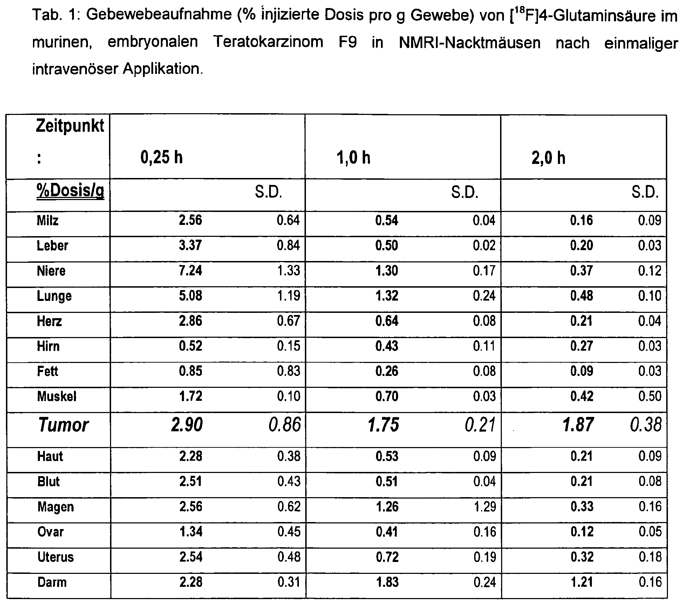

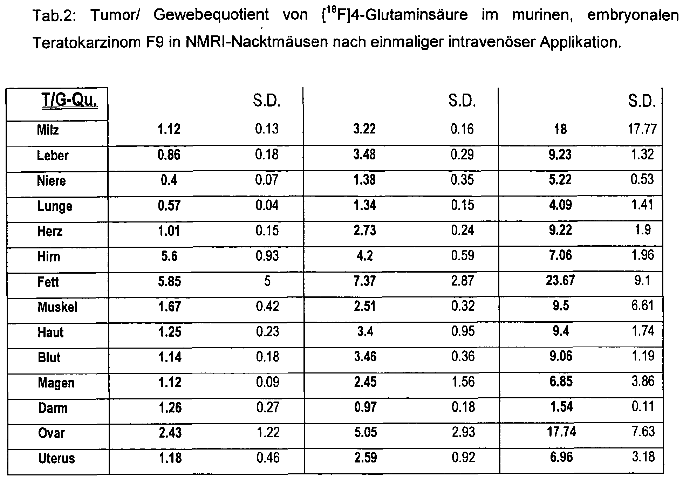

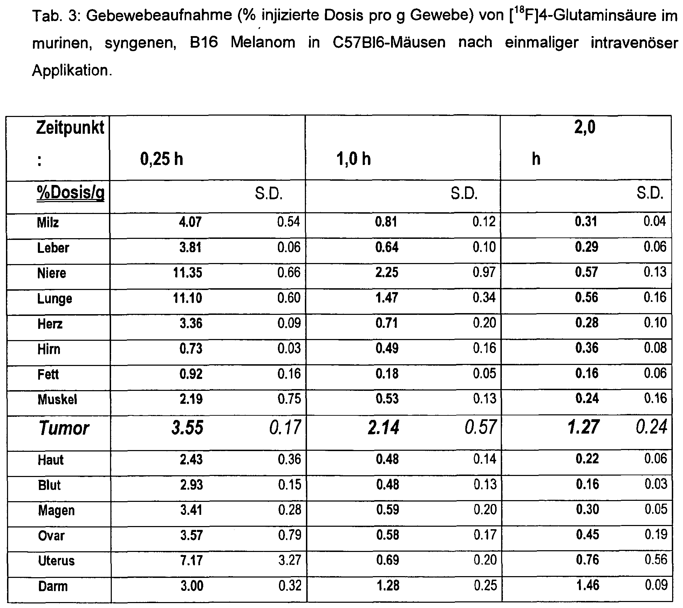

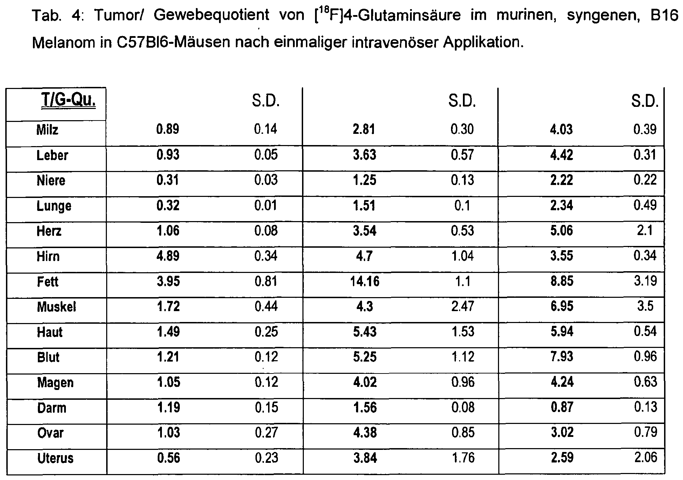

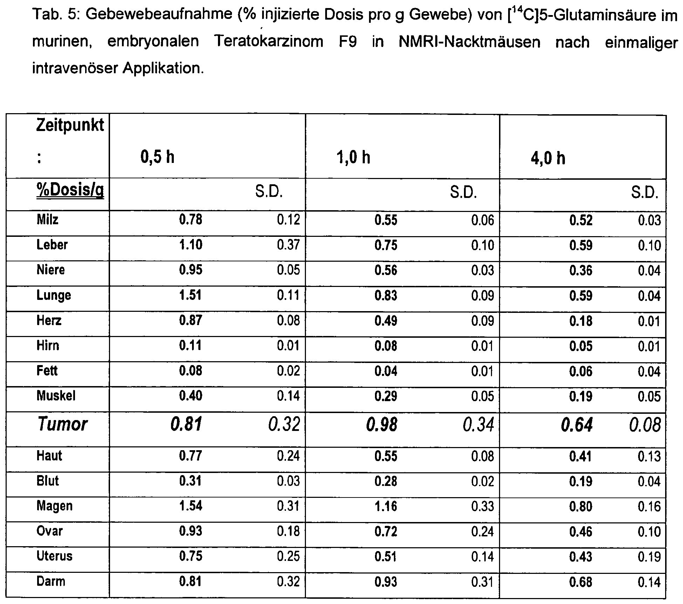

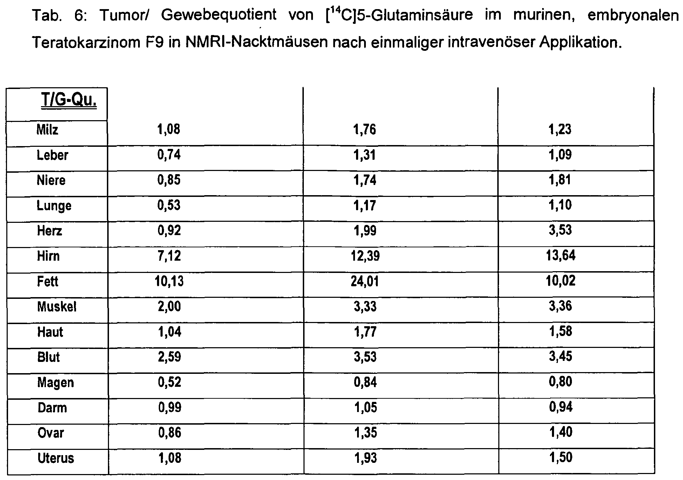

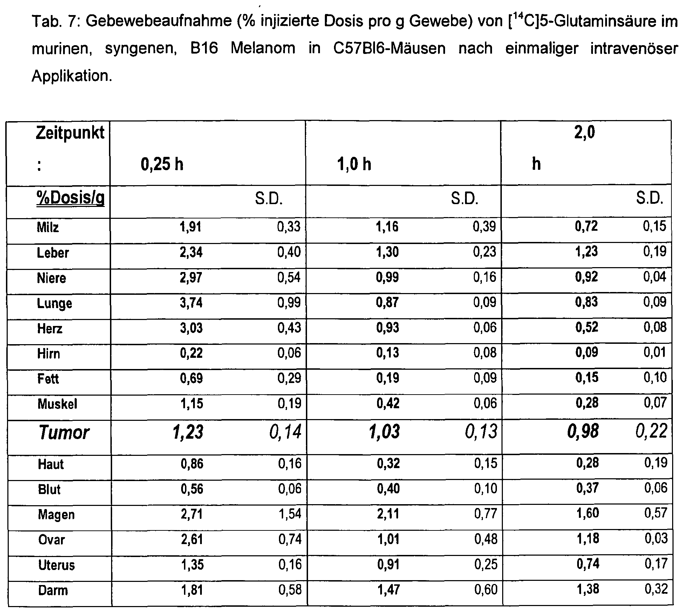

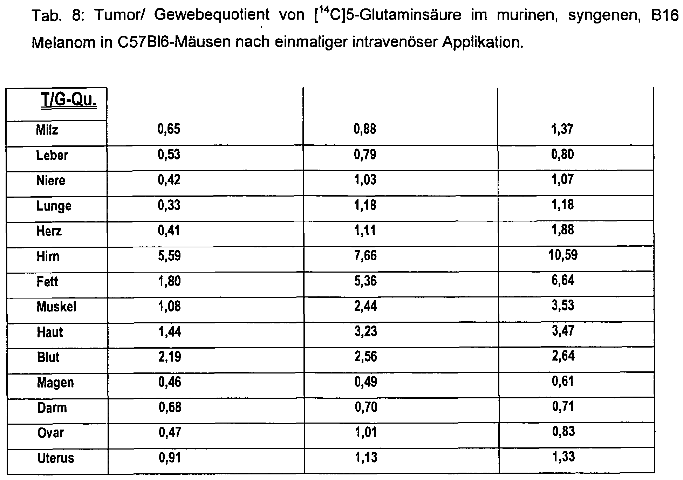

Abbildung 6 zeigt die resultierenden Gewebequotienten inklusive Tumor/ Muskelgewebe nach 2h.

## Patentansprüche

1. Verbindungen der allgemeinen Formel I 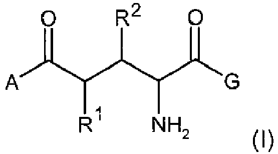 worin
A für
a) Hydroxyl,
b) verzweigtes oder unverzweigtes Hydroxy C₁-C₅ Alkoxy,
c) N(C₁-C₅ Alkyl)₂,
d) NH₂,
e) N(H)-L,
f) O-L oder
g) O-Z
steht,
G für
a) Hydroxyl,
b) verzweigtes oder unverzweigtes O-C₁-C₅ Alkyl,
c) verzweigtes oder unverzweigtes O-C₂-C₅ Alkenyl,
d) verzweigtes oder unverzweigtes O-C₁-C₅ Alkyl-(O-C₁-C₄ alkyl)ₙ-O-C₁-C₄ alkyl oder
e) verzweigtes oder unverzweigtes O-C₂-C₅ Alkinyl,
steht,
R¹ und R² für
a) Wasserstoff,
b) verzweigtes oder unverzweigtes ¹⁸F-C₁-C₅ Alkoxy,
c) verzweigtes oder unverzweigtes ¹⁸F-C₁-C₅ Alkyl,
d) verzweigtes oder unverzweigtes ¹⁸F-hydroxy-C₁-C₅ Alkyl,
e) verzweigtes oder unverzweigtes ¹⁸F-C₂-C₅ Alkenyl, oder
f) verzweigtes oder unverzweigtes ¹⁸F-C₂-C₅ Alkinyl,
g) Hydroxyl,
h) verzweigtes oder unverzweigtes C₁-C₅ Alkyl oder
i) verzweigtes oder unverzweigtes C₁-C₅ Alkoxy
steht,
mit der Maßgabe, dass einer der Substituenten R¹ oder R² genau ein ¹⁸F Isotop beinhaltet und der jeweils andere Substituent kein ¹⁸F Isotop beinhaltet,
L für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl oder
d) verzweigtes oder unverzweigtes C₂-C₅ Alkinyl,
steht, und
Z für ein Metallkationenequivalent steht, welches ein- oder zweiwertig sein kann, wobei zweiwertige Metalle zur ionischen Bindung mit zwei Resten der erfindungsgemäßen Strukturen führen können,
wobei
n = 0, 1, 2 oder 3 ist und
Diastereomere und Enantiomere.

2. Verbindungen nach Anspruch 1, **dadurch gekennzeichnet, dass** A für OH, methoxy oder NH₂ steht.

3. Verbindung nach Ansprüchen 1 oder 2, **dadurch gekennzeichnet, dass** A für OH steht.

4. Verbindung nach Ansprüchen 1 oder 2, **dadurch gekennzeichnet, dass** A für NH₂ steht.

5. Verbindungen nach Ansprüchen 1 bis 4, **dadurch gekennzeichnet, dass** R¹ und R² ausgewählt ist aus der Gruppe Wasserstoff, ¹⁸F-methoxy, ¹⁸F-ethoxy, ¹⁸F-propoxy, ¹⁸F-methyl, ¹⁸F-ethyl und ¹⁸F-propyl mit der Maßgabe, dass einer der Substituenten R¹ oder R² genau ein ¹⁸F Isotop beinhaltet und der jeweils andere Substituent Wasserstoff ist.

6. Verbindung nach Ansprüchen 1 bis 4, **dadurch gekennzeichnet, dass** R^{1 18}F-Propyl ist, und R² für Wasserstoff steht.

7. Verbindung nach Anspruch 1 mit der Formel

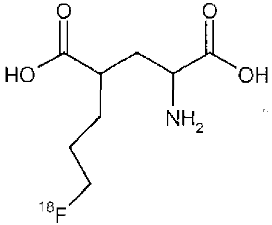

8. Verbindung nach Anspruch 1, **dadurch gekennzeichnet, dass** genau einer der Substituenten R¹ oder R^{2 18}F-Propyl ist, mit der Maßgabe, dass einer der Substituenten R¹ oder R² genau ein ¹⁸F Isotop beinhaltet und der jeweils andere Substituent Wasserstoff ist.

9. Verbindung nach Ansprüchen 1 bis 8, **dadurch gekennzeichnet, dass** G ausgewählt ist aus der Gruppe OH, Methoxy oder Ethoxy.

10. Verbindung nach Ansprüchen 1 bis 9, **dadurch gekennzeichnet, dass** Z ausgewählt ist aus der Gruppe Mg²⁺, Ca²⁺, Na⁺ und K⁺

11. Verbindung nach Ansprüchen 1 bis 9, **dadurch gekennzeichnet, dass** Z Ni²⁺ ist

12. Verbindung nach Anspruch 1 ausgewählt aus der Gruppe von Verbindungen mit der Formel:
a) 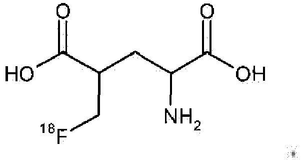
b) 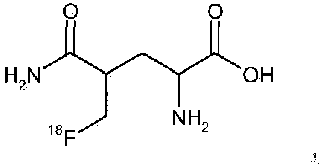
c) 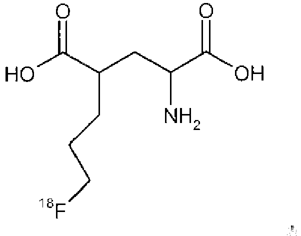
d) 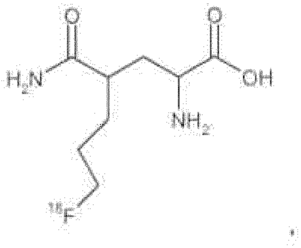
e) 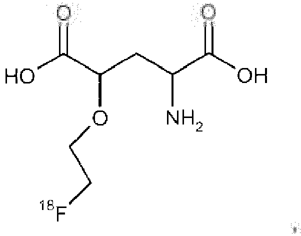
f) 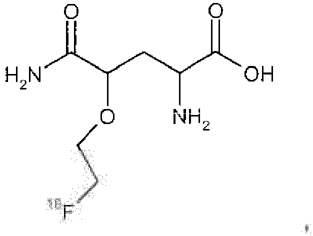
g) 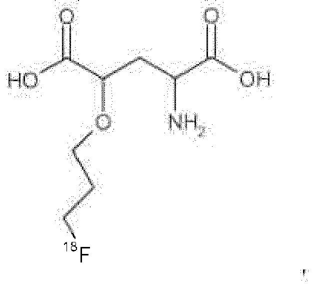
h)

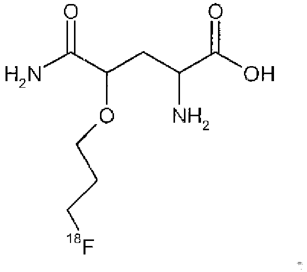

13. Verbindungen der allgemeinen Formel (II): 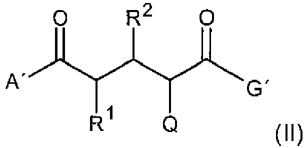 worin,
A' für
a) Hydroxyl,
b) verzweigtes oder unverzweigtes Hydroxy C₁-C₅ Alkoxy,
c) N(C₁-C₅ Alkyl)₂,
d) NH₂,
e) N(H)-U
f) N(H)-L', oder
g) O-L'
steht,
G' für
a) hydroxyl,
b) O-Z',
c) verzweigtes oder unverzweigtes O-C₁-C₅ Alkyl,
d) verzweigtes oder unverzweigtes O-C₂-C₅ Alkenyl,
e) verzweigtes oder unverzweigtes O-C₁-C₅ Alkyl-(O-C₁-C₄ alkyl)ₙ-O-C₁-C₄ alkyl, oder
f) verzweigtes oder unverzweigtes O-C₂-C₅ Alkinyl steht,
R¹ und R² für
a) Wasserstoff,
b) verzweigtes oder unverzweigtes ¹⁸F C₁-C₅ Alkoxy,
c) verzweigtes oder unverzweigtes ¹⁸F-C₁-C₅ Alkyl,
d) verzweigtes oder unverzweigtes ¹⁸F-C₁-C₅-hydroxy-alkyl,
e) verzweigtes oder unverzweigtes ¹⁸F-C₂-C₅ Alkenyl,
f) verzweigtes oder unverzweigtes ¹⁸F-C₂-C₅ Alkinyl,
g) Hydroxyl,
h) verzweigtes oder unverzweigtes C₁-C₅ Alkyl, oder
i) verzweigtes oder unverzweigtes C₁-C₅ Alkoxy
steht,
mit der Maßgabe, dass genau einer der Substituenten R¹ oder R² genau ein ¹⁸F Isotop und der jeweils andere Substituent kein ¹⁸F Isotop beinhaltet,
Q für
a) N(H)-tert-Butoxycarbonyl,
b) N(H)-Allyloxycarbonyl,
c) N(H)-Benzyloxycarbonyl,
d) N(H)-Ethoxycarbonyl,
e) N(H)-Methoxycarbonyl,
f) N(H)-Propoxycarbonyl,
e) N(H)-2,2,2-Trichlorethoxycarbonyl,
f) N(H)-1,1-Dimethylpropinyl,
g) N(H)-1-Methyl-1-phenyl-ethoxycarbonyl,
h) N(H)-1-Methyl-1-(4-biphenylyl)-ethoxycarbonyl,
i) N(H)-cyclobutylcarbonyl,
j) N(H)-1-Methylcyclobutylcarbonyl,
k) N(H)-Vinylcarbonyl,
l) N(H)-Allylcarbonyl,
m) N(H)-Adamantylcarbonyl,
n) N(H)-Diphenylmethylcarbonyl,
o) N(H)-Cinnamylcarbonyl,
p) N(H)-Formyl,
q) N(H)-Benzoyl,
r) N(H)-Trityl,
s) N(H)-p-Methoxyphenyl-diphenylmethyl,
t) N(H)-di-(p-Methoxyphenyl)-phenylmethyl,
u) 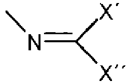 oder
v) N-(tert-Butoxycarbonyl)₂,
steht,
L' für
a) verzweigtes oder unverzweigtes C₁-C₅ Alkyl,
b) verzweigtes oder unverzweigtes C₁-C₅ Alkenyl,
c) verzweigtes oder unverzweigtes C₁-C₅ Alkyl-(O-C₁-C₄ alkyl)ₙ-O-C₁-C₄ alkyl, oder
d) verzweigtes oder unverzweigtes C₂-C₅ Alkinyl
steht,
U für
a) tert-Butoxycarbonyl,
b) Allyloxycarbonyl,
c) Benzyloxycarbonyl,
d) Ethoxycarbonyl,
e) Methoxycarbonyl oder
f) Propoxycarbonyl
steht,
X' und X" unabhängig voneinander für
a) verzweigtes oder unverzweigtes C₁-C₅ Alkyl,
b) substituiertes oder unsubstituiertes Aryl oder
c) Aralkyl
steht, und
Z' für ein Metallkationenequivalent
steht, welches ein- oder zweiwertig sein kann, wobei zweiwertige Metalle zur ionischen Bindung mit zwei Resten der erfindungsgemäßen Strukturen führen können oder koordinative Bindungen mit Carboxyl- und Aminfunktion des Aminosäurederivates ausbilden können,
wobei n = 0, 1, 2 oder 3 ist und
Diastereomere und Enantiomere.

14. Verbindungen nach Anspruch 13, **dadurch gekennzeichnet, dass** A' für OH, verzweigtes oder unverzweigtes C₁-C₅ Alkoxy, -NH-tert-Butoxycarbonyl oder NH₂ steht.

15. Verbindungen nach Ansprüchen 13 oder 14, **dadurch gekennzeichnet, dass** A' für Ethoxy steht.

16. Verbindungen nach Ansprüchen 13 bis 15, **dadurch gekennzeichnet, dass** A' für NH₂ steht.

17. Verbindungen nach Ansprüchen 13 bis 16, **dadurch gekennzeichnet, dass** R¹ und R² ausgewählt ist aus der Gruppe Wasserstoff, ¹⁸F-methoxy, ¹⁸F-ethoxy, ¹⁸F-propoxy, ¹⁸F-methyl, ¹⁸F-ethyl und ¹⁸F-propyl mit der Maßgabe, dass einer der Substituenten R¹ oder R² genau ein ¹⁸F Isotop beinhaltet und der jeweils andere Substituent Wasserstoff ist.

18. Verbindung nach Ansprüchen 13 bis 16, **dadurch gekennzeichnet, dass** R^{1 18}F-propyl ist und R² für Wasserstoff steht.

19. Verbindung nach Ansprüchen 13 bis 18, **dadurch gekennzeichnet, dass** G' ausgewählt ist aus der Gruppe OH, Ethoxy, Methoxy und OZ'.

20. Verbindung nach Ansprüchen 13 bis 19, **dadurch gekennzeichnet, dass** G' für Ethoxy steht.

21. Verbindung nach Ansprüchen 13 bis 20, **dadurch gekennzeichnet, dass** Z' ausgewählt ist aus der Gruppe Na⁺, K⁺, Ca²⁺ und Mg²⁺.

22. Verbindung nach Ansprüchen 13 bis 21, **dadurch gekennzeichnet, dass** Z' Ni²⁺ ist.

23. Verbindung nach Ansprüchen 13 bis 22, **dadurch gekennzeichnet, dass** Q ausgewählt ist aus der Gruppe N(H)-tert-Butoxycarbonyl, N(H)-Benzyloxycarbonyl und 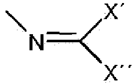 worin
X und X' unabhängig voneinander für
a) verzweigtes oder unverzweigtes C₁-C₅ Alkyl,
b) substituiertes oder unsubstituiertes Aryl oder
c) Aralkyl
steht.

24. Verbindung nach Ansprüchen 13 bis 23 **dadurch gekennzeichnet, dass** Q für N(H)-tert-Butoxycarbonyl steht.

25. Verbindung nach Ansprüchen 13 bis 24 **gekennzeichnet dadurch, dass** Q für 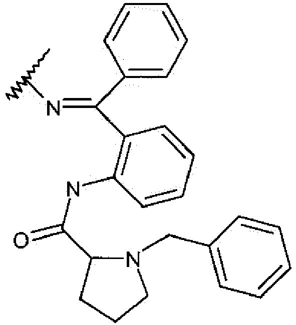 steht.

26. Verbindung nach Anspruch 14 ausgewählt aus der Gruppe von Verbindungen mit der Formel:
a) 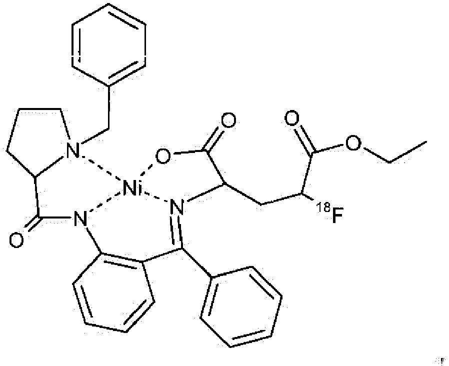
b)

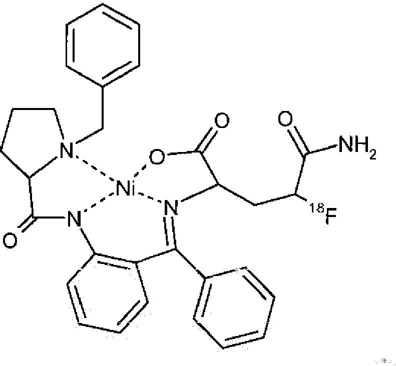

27. Verbindungen gemäß den Ansprüchen 1 bis 26 zur Verwendung als Arzneimittel.

28. Verbindungen gemäß den Ansprüchen 1 bis 26 zur Verwendung in der Diagnose von Tumorerkrankungen.

29. Verwendung von Verbindungen gemäß den Ansprüchen 1 bis 26 zur Herstellung eines Arzneimittels für die Diagnose von Tumorerkrankungen.

30. Verbindungen der Formel (III) 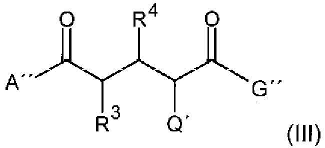 worin
A" für
a) Hydroxyl,
b) verzweigtes oder unverzweigtes Hydroxy C₁-C₅ Alkoxy,
c) N(C₁-C₅ Alkyl)₂,
d) NH₂,
e) N(H)-U'
f) N(H)-L" oder
g) O-L"
steht,
G" für
a) hydroxyl,
b) O-Z",
c) verzweigtes oder unverzweigtes O-C₁-C₅ Alkyl,
d) verzweigtes oder unverzweigtes O-C₂-C₅ Alkenyl,
e) verzweigtes oder unverzweigtes O-C₁-C₅ Alkyl-(O-C₁-C₄ alkyl)ₙ-O-C₁-C₄ alkyl,
f) verzweigtes oder unverzweigtes O-C₂-C₅ Alkinyl, oder
g) triphenylmethoxy
steht,
R³ und R⁴ für
a) Wasserstoff,
b) verzweigtes oder unverzweigtes E-C₁-C₅ Alkoxy,
c) verzweigtes oder unverzweigtes E-C₁-C₅ Alkyl,
d) verzweigtes oder unverzweigtes E- Hydroxy-C₁-C₅ -alkyl,
e) verzweigtes oder unverzweigtes E-C₂-C₅ Alkenyl,
f) verzweigtes oder unverzweigtes E-C₂-C₅ Alkinyl,
g) Hydroxyl,
h) verzweigtes oder unverzweigtes C₁-C₅ Alkyl, oder
i) verzweigtes oder unverzweigtes C₁-C₅ Alkoxy
steht, mit der Maßgabe, dass genau einer der Substituenten R³ oder R⁴ ein E beinhaltet und der jeweils andere Substituent kein E beinhaltet,
**E** für
a) Chloro,
b) Bromo,
c) Mesyloxy,
d) Trifluormesyloxy,
e) Nonafluorbutyloxy, oder
f) Tosyloxy
steht,
Q' für
a) N(H)-tert-Butoxycarbonyl,
b) N(H)-Allyloxycarbonyl,
c) N(H)-Benzyloxycarbonyl,
d) N(H)-Ethoxycarbonyl,
e) N(H)-Methoxycarbonyl,
f) N(H)-Propoxycarbonyl,
g) N(H)-2,2,2-Trichlorethoxycarbonyl,
h) N(H)-1,1-Dimethylpropinyl,
i) N(H)-1-Methyl-1-phenyl-ethoxycarbonyl,
j) N(H)-1-Methyl-1-(4-biphenylyl)-ethoxycarbonyl,
k) N(H)-Cyclobutylcarbonyl,
l) N(H)-1-Methylcyclobutylcarbonyl,
m) N(H)-Vinylcarbonyl,
n) N(H)-Allylcarbonyl,
o) N(H)-Adamantylcarbonyl,
p) N(H)-Diphenylmethylcarbonyl,
q) N(H)-Cinnamylcarbonyl,
r) N(H)-Formyl,
s) N(H)-Benzoyl,
t) N(H)-Trityl,
u) N(H)-p-Methoxyphenyl-diphenylmethyl,
v) N(H)-di-(p-Methoxyphenyl)-phenylmethyl,
w) 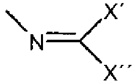 oder
x) N-(tert-Butoxycarbonyl)₂,
steht,
L" für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄alkyl)ₙ-O-C₁-C₄alkyl oder
d) verzweigtes oder unverzweigtes C₂-C₅ Alkinyl
steht,
U' für
a) tert-Butoxycarbonyl,
b) Allyloxycarbonyl,
c) Benzyloxycarbonyl, oder
d) Ethoxycarbonyl
steht,
X' and X" unabhängig voneinander für
a) verzweigtes oder unverzweigtes C₁-C₅ Alkyl,
b) substituiertes oder unsubstituiertes Aryl
c) Alkylaryl oder
d) Heteroaryl
steht, und
Z" für ein Metallkationenequivalent steht, welches ein- oder zweiwertig sein kann, wobei zweiwertige Metalle zur ionischen Bindung mit zwei Resten der erfindungsgemäßen Strukturen führen können oder koordinative Bindungen mit Carboxyl- und Aminfunktion des Aminosäurederivates ausbilden können,
wobei n = 0, 1 oder 2 ist und
Diastereomere und Enantiomere.

31. Verwendung von Verbindungen der Formel (IV) zur Herstellung von Verbindungen der Formel (I) oder (II): 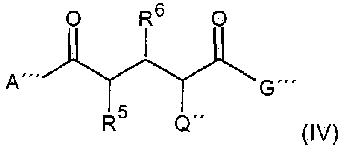 worin
A'" für
a) Hydroxyl,
b) verzweigtes oder unverzweigtes Hydroxy C₁-C₅ Alkoxy,
c) N(C₁-C₅ Alkyl)₂,
d) NH₂,
e) N(H)-U"
f) N(H)-L'" oder
g) O-L'"
steht,
G"' für
a) hydroxyl,
b) verzweigtes oder unverzweigtes O-C₁-C₅ Alkyl,
c) verzweigtes oder unverzweigtes O-C₂-C₅ Alkenyl,
d) verzweigtes oder unverzweigtes O-C₁-C₅ Alkyl-(O-C₁-C₄ alkyl)ₙ-C₁-C₄ alkyl,
e) verzweigtes oder unverzweigtes O-C₂-C₅ Alkinyl, oder
f) triphenylmethoxy
steht,
R⁵ und R⁶ für
a) Wasserstoff oder
b) E'
steht, mit der Maßgabe, dass genau einer der Substituenten R⁵ oder R⁶ E' beinhaltet und der jeweils andere Substituent Wasserstoff beinhaltet,
E' für
a) Chloro,
b) Bromo,
c) Mesyloxy,
d) Trifluormesyloxy,
e) Nonafluorbutyloxy oder
f) Tosyloxy
steht,
Q" für
a) N(H)-tert-Butoxycarbonyl,
b) N(H)-Allyloxycarbonyl,
c) N(H)-Benzyloxycarbonyl,
d) N(H)-Ethoxycarbonyl,
e) N(H)-Methoxycarbonyl,
f) N(H)-Propoxycarbonyl,
g) N(H)-2,2,2-Trichlorethoxycarbonyl,
h) N(H)-1,1-Dimethylpropinyl,
i) N(H)-1-Methyl-1-phenyl-ethoxycarbonyl,
j) N(H)-1-Methyl-1-(4-biphenylyl)-ethoxycarbonyl,
k) N(H)-cyclobutylcarbonyl,
l) N(H)-1-Methylcyclobutylcarbonyl,
m) N(H)-Vinylcarbonyl,
n) N(H)-Allylcarbonyl,
o) N(H)-Adamantylcarbonyl,
p) N(H)-Diphenylmethylcarbonyl,
q) N(H)-Cinnamylcarbonyl,
r) N(H)-Formyl,
s) N(H)-Benzoyl,
t) N(H)-Trityl,
u) N(H)-p-Methoxyphenyl-diphenylmethyl,
v) N(H)-di-(p-Methoxyphenyl)-phenylmethyl,
w) 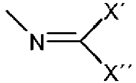 oder
x) N-(tert-Butoxycarbonyl)₂,
steht,
L'" für
a) verzweigtes oder unverzweigtes C₁-C₅ Alkyl,
b) verzweigtes oder unverzweigtes C₂-C₅ Alkenyl,
c) verzweigtes oder unverzweigtes C₁-C₅ Alkyl-(O-C₁-C₄ alkyl)ₙ-O-C₁-C₄ alkyl, oder
d) verzweigtes oder unverzweigtes C₂-C₅ Alkinyl
steht,
U" für
a) tert-Butoxycarbonyl,
b) Allyloxycarbonyl,
c) Benzyloxycarbonyl, oder
d) Ethoxycarbonyl
steht,
X" and X'" unabhängig voneinander für
a) verzweigtes oder unverzweigtes C₁-C₅ Alkyl,
b) substituiertes oder unsubstituiertes Aryl
c) Alkylaryl, oder
d) Heteroaryl
steht,
wobei n = 0, 1 oder 2 ist und
Diastereomere und Enantiomere.

32. Verfahren zur Herstellung von Verbindungen der allgemeinen Formel (II) gemäß Ansprüchen 13 bis 26
indem
eine Vorläuferverbindung der Verbindung nach Formel (III) gemäß Anspruch 30 mit F-18 Fluorid umgesetzt wird.

33. Verfahren zur Herstellung der Verbindungen der allgemeinen Forme! (I) oder (II) gemäß der Ansprüche 1 bis 26, gezeichnet dadurch, dass die Mehrzahl der Verbindungen nach Formel (I) und (II) aus einer Vorläuferverbindung der Verbindung nach Formel (IV) gemäß Anspruch 31 nach Einführung des ¹⁸F-Isotops entstehen kann.

## Claims

1. Compounds of the general formula I 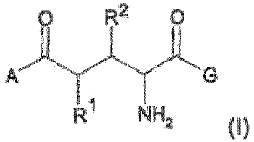 **characterized in that**
A represents
a) hydroxyl,
b) branched or unbranched hydroxy C₁-C₅ alkoxy
c) N(C₁-C₅ alkyl)₂,
d) NH₂,
e) N(H)-L,
f) O-L or
g) O-Z,
G represents
a) hydroxyl,
b) branched or unbranched O-C₁-C₅ alkyl,
c) branched or unbranched O-C₂-C₅ alkenyl,
d) branched or unbranched O-C₁-C₅ alkyl-(O-C₁-C₄ alkyl)ₙ-O-C₁-C₄ alkyl or
e) branched or unbranched O-C₂-C₅ alkynyl,
R¹ and R² represent
a) hydrogen,
b) branched or unbranched ¹⁸F- C₁-C₅ alkoxy
c) branched or unbranched ¹⁸F- C₁-C₅ alkyl,
d) branched or unbranched ¹⁸F-hydroxy- C₁-C₅ alkyl,
e) branched or unbranched ¹⁸F-C₂-C₅ alkenyl, or
f) branched or unbranched ¹⁸F-C₂-C₅ alkynyl,
g) hydroxyl,
h) branched or unbranched C₁-C₅ alkyl, or
i) branched or unbranched C₁-C₅ alkoxy,
provided that one of the substituents R¹ or R² contains exactly one ¹⁸F isotope and the other substituent in each case contains no ¹⁸F isotope,
L represents
a) branched or unbranched C₁-C₅ alkyl,
b) branched or unbranched C₂-C₅ alkenyl,
c) branched or unbranched C₁-C₅ alkyl-(O-C₁-C₄ alkyl)ₙ-O-C₁-C₄ alkyl, or
d) branched or unbranched C₂-C₅ alkynyl , and
Z represents a metal cation equivalent which can be monovalent or divalent, where divalent metals may form an ionic bond with two residues of the proposed structures,
**characterized in that**
n = 0, 1, 2 or 3 and
diastereomers and enantiomers.

2. Compounds according to claim 1, **characterized in that** A represents OH, methoxy, or NH₂.

3. A compound according to claims 1 or 2, **characterized in that** A represents OH.

4. A compound according to claims 1 or 2, **characterized in that** A represents NH₂.

5. Compounds according to claims 1 to 4, **characterized in that** R¹ and R² are selected from the group consisting of hydrogen, ¹⁸F-methoxy, ¹⁸F-ethoxy, ¹⁸F-propoxy, ¹⁸F-methyl, ¹⁸F-ethyl, and ¹⁸F-propyl, provided that one of the substituents R¹ or R² contains exactly one ¹⁸F isotope and the other substituent in each case is hydrogen.

6. A compound according to claims 1 to 4, **characterized in that** R¹ is ¹⁸F-propyl and R² represents hydrogen.

7. A compound according to claim 1 having the formula

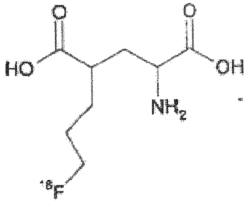

8. A compound according to claim 1, **characterized in that** exactly one of the substituents R¹ or R² is ¹⁸F-propyl, provided that one of the substituents R¹ or R² contains exactly one ¹⁸F isotope and the other substituent in each case is hydrogen.

9. A compound according to claims 1 to 8, **characterized in that** G is selected from the group containing OH, methoxy, or ethoxy.

10. A compound according to claims 1 to 9, **characterized in that** Z is selected from the group containing Mg²⁺, Ca²⁺, Na⁺, and K⁺.

11. A compound according to claims 1 to 9, **characterized in that** Z is Ni²⁺.

12. A compound according to claim 1 selected from the group of compounds having the formula:
a) 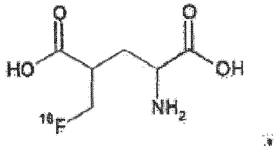
b) 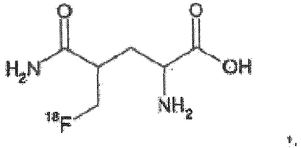
c) 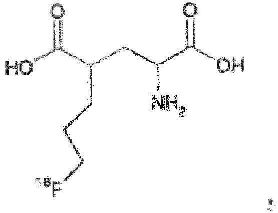
d) 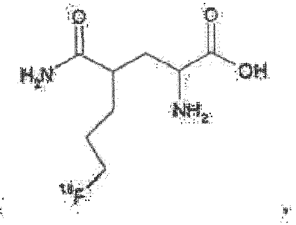
e) 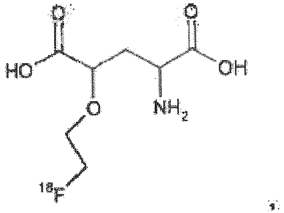
f) 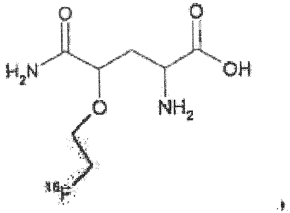
g) 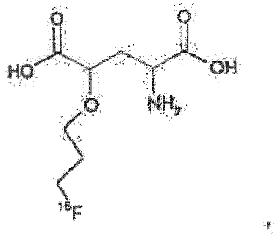
h)

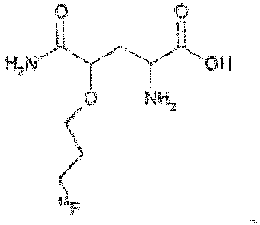

13. Compounds of the general formula (II): 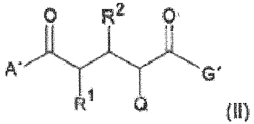 **characterized in that**
A' represents
a) hydroxyl,
b) branched or unbranched hydroxy C₁-C₅ alkoxy,
c) N(C₁-C₅alkyl)₂,
d) NH₂,
e) N(H)-U
f) N(H)-L', or
g) O-L',
G' represents
a) hydroxyl,
b) O-Z',
c) branched or unbranched O-C₁-C₅ alkyl,
d) branched or unbranched O-C₂-C₅ alkenyl,
e) branched or unbranched O-C₁-C₅ alkyl-(O-C₁-C₄ alkyl)ₙ-O-C₁-C₄ alkyl, or
f) branched or unbranched O-C₂-C₅ alkynyl,
R¹ and R² represent
a) hydrogen,
b) branched or unbranched ¹⁸F-C₁-C₅ alkoxy,
c) branched or unbranched ¹⁸F-C₁-C₅ alkyl,
d) branched or unbranched ¹⁸F-C₁-C₅-hydroxy-alkyl,
e) branched or unbranched ¹⁸F-C₂-C₅ alkenyl,
f) branched or unbranched ¹⁸F-C₂-C₅ alkynyl,
g) hydroxyl,
h) branched or unbranched C₁-C₅ alkyl, or
i) branched or unbranched C₁-C₅ alkoxy,
provided that exactly one of the substituents R¹ or R² contains exactly one ¹⁸F isotope and the other substituent in each case contains no ¹⁸F isotope,
Q represents
a) N(H)-tert-butoxycarbonyl,
b) N(H)-allyloxycarbonyl,
c) N(H)-benzyloxycarbonyl,
d) N(H)-ethoxycarbonyl,
e) N(H)-methoxycarbonyl,
f) N(H)-propoxycarbonyl,
e) N(H)-2,2,2-trichlorethoxycarbonyl,
f) N(H)-1,1-dimethylpropinyl,
g) N(H)-1-methyl-1-phenyl-ethoxycarbonyl,
h) N(H)-1-methyl-1-(4-biphenylyl)-ethoxycarbonyl,
i) N(H)-cyclobutylcarbonyl,
j) N(H)-1-methylcyclobutylcarbonyl,
k) N(H)-vinylcarbonyl,
l) N(H)-allylcarbonyl,
m) N(H)-adamantylcarbonyl,
n) N(H)-diphenylmethylcarbonyl,
o) N(H)-cinnamylcarbonyl,
p) N(H)-formyl,
q) N(H)-benzoyl,
r) N(H)-trityl,
s) N(H)-p-methoxyphenyl-diphenylmethyl,
t) N(H)-di-(p-methoxyphenyl)-phenylmethyl,
u) 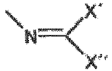 or
v) N-(tert-butoxycarbonyl)₂,
L' represents
a) branched or unbranched C₁-C₅ alkyl,
b) branched or unbranched C₂-C₅ alkenyl,
c) branched or unbranched C₁-C₅ alkyl-(O-C₁-C₄ alkyl)ₙ-O-C₁-C₄ alkyl,
or
d) branched or unbranched C₂-C₅ alkynyl,
U represents
a) tert-butoxycarbonyl,
b) allyloxycarbonyl,
c) benzyloxy carbonyl,
d) ethoxycarbonyl,
e) methoxycarbonyl, or
f) propoxycarbonyl,
X' and X", independently of one another, represent
a) branched or unbranched C₁-C₅ alkyl,
b) substituted or unsubstituted aryl, or
c) aralkyl, and
Z' represents a metal cation equivalent
which can be monovalent or divalent, where divalent metals may form an ionic bond with two residues of the proposed structures or form coordinative bonds with the carboxylic and amine functions of the amino acid derivate,
where n = 0, 1,2, or 3 and
diastereomers and enantiomers.

14. Compounds according to claim 13, **characterized in that** A' represents OH, branched or unbranched C₁-C₅ alkoxy, -NH-tert-butoxycarbonyl, or NH₂.

15. Compounds according to claims 13 or 14, **characterized in that** A' represents ethoxy.

16. Compounds according to claims 13 to 15, **characterized in that** A' represents NH₂.

17. Compounds according to claims 13 to 16, **characterized in that** R¹ and R² are selected from the group consisting of hydrogen, ¹⁸F-methoxy, ¹⁸F-ethoxy, ¹⁸F-propoxy, ¹⁸F-methyl, ¹⁸F-ethyl, and ¹⁸F-propyl, provided that one of the substituents R¹ or R² contains exactly one ¹⁸F isotope and the other substituent in each case is hydrogen.

18. A compound according to claims 13 to 16, **characterized in that** R¹ is ¹⁸F-propyl and R² represents hydrogen.

19. A compound according to claims 13 to 18, **characterized in that** G' is selected from the group containing OH, ethoxy, methoxy, and OZ'.

20. A compound according to claims 13 or 19, **characterized in that** G' represents ethoxy.

21. A compound according to claims 13 to 20, **characterized in that** Z' is selected from the group containing Na⁺, K⁺, Ca²⁺, and Mg²⁺.

22. A compound according to claims 13 to 21, **characterized in that** Z' is Ni²⁺.

23. A compound according to claims 13 to 22, **characterized in that** Q is selected from the group containing N(H)-tert-butoxycarbonyl, N(H)-benzyloxycarbonyl, and 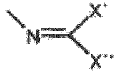 **characterized in that**
X and X', independently of one another, represent
a) branched or unbranched C₁-C₅ alkyl,
b) substituted or unsubstituted aryl, or
c) aralkyl

24. A compound according to claims 13 to 23, **characterized in that** Q represents N(H)-tert-butoxycarbonyl.

25. A compound according to claims 13 to 24, **characterized in that** Q represents

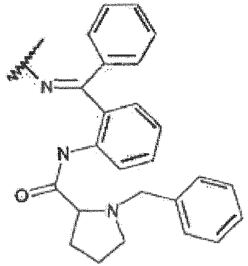

26. A compound according to claim 14 selected from the group of compounds having the formula:
a) 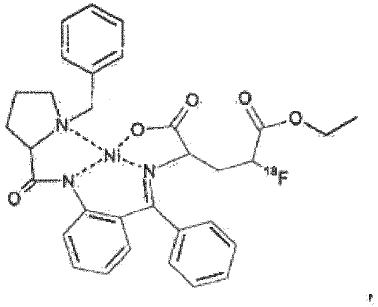
b)

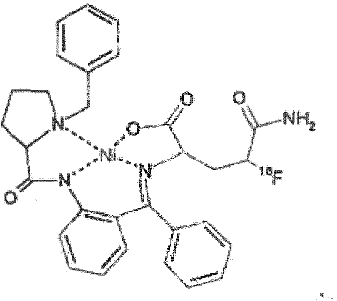

27. Compounds according to claims 1 to 26 for use as a pharmaceutical.

28. Compounds according to claims 1 to 26 for use in the diagnosis of tumors.

29. The use of compounds according to claims 1 to 26 in the preparation of a drug for the diagnosis of tumors.

30. Compounds of formula (III) 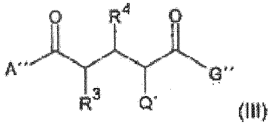 **characterized in that**
A" represents
a) hydroxyl,
b) branched or unbranched hydroxy C₁-C₅ alkoxy,
c) N(C₁-C₅ alkyl)₂,
d) NH₂,
e) N(H)-U'
f) N(H)-L", or
g) O-L",
G" represents
a) hydroxyl,
b) O-Z",
c) branched or unbranched O-C₁-C₅ alkyl,
d) branched or unbranched O-C₂-C₅ alkenyl
e) branched or unbranched O-C₁-C₅ alkyl-(O-C₁-C₄ alkyl)ₙ-O-C₁-C₄ alkyl,
f) branched or unbranched O-C₂-C₅ alkynyl, or
g) triphenylmethoxy,
R³ and R⁴ represent
a) hydrogen,
b) branched or unbranched E-C₁-C₅ alkoxy,
c) branched or unbranched E-C₁-C₅ alkyl,
d) branched or unbranched E-hydroxy-C₁-C₅-alkyl,
e) branched or unbranched E-C₂-C₅ alkenyl,
f) branched or unbranched E-C₂-C₅ alkynyl,
g) hydroxyl,
h) branched or unbranched C₁-C₅ alkyl, or
i) branched or unbranched C₁-C₅ alkoxy,
provided that exactly one of the substituents R³ or R⁴ contains E and the other substituent in each case contains no E,
E represents
a) chloro,
b) bromo,
c) mesyloxy,
d) trifluoromesyloxy,
e) nonafluorobutyloxy, or
f) tosyloxy,
Q' represents
a) N(H)-tert-butoxycarbonyl,
b) N(H)-allyloxycarbonyl,
c) N(H)-benzyloxycarbonyl,
d) N(H)-ethoxycarbonyl,
e) N(H)-methoxycarbonyl,
f) N(H)-propoxycarbonyl,
g) N(H)-2,2,2-trichlorethoxycarbonyl,
h) N(H)-1,1-dimethylpropinyl,
i) N(H)-1-methyl-1-phenyl-ethoxycarbonyl,
j) N(H)-1-methyl-1-(4-biphenylyl)-ethoxycarbonyl,
k) N(H)-cyclobutylcarbonyl,
l) N(H)-1-methylcyclobutylcarbonyl,
m) N(H)-vinylcarbonyl,
n) N(H)-allylcarbonyl,
o) N(H)-adamantylcarbonyl,
p) N(H)-diphenylmethylcarbonyl,
q) N(H)-cinnamylcarbonyl,
r) N(H)-formyl,
s) N(H)-benzoyl,
t) N(H)-trityl,
u) N(H)-p-methoxyphenyl-diphenylmethyl,
v) N(H)-di-(p-methoxyphenyl)-phenylmethyl,
w) 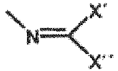 or
x) N-(tert-butoxycarbonyl)₂,
L" represents
a) branched or unbranched C₁-C₅ alkyl,
b) branched or unbranched C₂-C₅ alkenyl,
c) branched or unbranched C₁-C₅ alkyl-(O-C₁-C₄ alkyl)ₙ-C₁-C₄ alkyl, or
d) branched or unbranched C₂-C₅ alkynyl,
U' represents
a) tert-butoxycarbonyl,
b) allyloxycarbonyl,
c) benzyloxycarbonyl, or
d) ethoxycarbonyl,
X' and X", independently of one another, represent
a) branched or unbranched C₁-C₅ alkyl,
b) substituted or unsubstituted aryl,
c) alkylaryl, or
d) heteroaryl, and
Z" represents a metal cation equivalent which can be monovalent or divalent, where divalent metals may form an ionic bond with two residues of the proposed structures or form coordinative bonds with the carboxylic and amine functions of the amino acid derivate,
where n = 0, 1, or 2 and
diastereomers and enantiomers.

31. The use of compounds as set forth in formula (IV) for the preparation of compounds as set forth in formula (I) or (II): 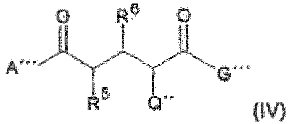 **characterized in that**
A'" represents
a) hydroxyl,
b) branched or unbranched hydroxy C₁-C₅ alkoxy,
c) N(C₁-C₅ alkyl)₂,
d) NH₂,
e) N(H)-U"
f) N(H)-L"', or
g) O-L'",
G'" represents
a) hydroxyl,
b) branched or unbranched O-C₁-C₅ alkyl,
c) branched or unbranched O-C₂-C₅ alkenyl
d) branched or unbranched O-C₁-C₅ alkyl-(O-C₁-C₄ alkyl)ₙ-O-C₁-C₄ alkyl,
e) branched or unbranched O-C₂-C₅ alkynyl, or
f) triphenylmethoxy,
R⁵ and R⁶ represent
a) hydrogen or
b) E',
provided that exactly one of the substituents R⁵ or R⁶ contains E' and the other substituent in each case contains hydrogen,
E' represents
a) chloro,
b) bromo,
c) mesyloxy,
d) trifluoromesyloxy,
e) nonafluorobutyloxy, or
f) tosyloxy,
Q" represents
a) N(H)-tert-butoxycarbonyl,
b) N(H)-allyloxycarbonyl,
c) N(H)-benzyloxycarbonyl,
d) N(H)-ethoxycarbonyl,
e) N(H)-methoxycarbonyl,
f) N(H)-propoxycarbonyl,
g) N(H)-2,2,2-trichlorethoxycarbonyl,
h) N(H)-1,1-dimethylpropinyl,
i) N(H)-1-methyl-1-phenyl-ethoxycarbonyl,
j) N(H)-1-methyl-1-(4-biphenylyl)-ethoxycarbonyl,
k) N(H)-cyclobutylcarbonyl,
l) N(H)-1-methylcyclobutylcarbonyl,
m) N(H)-vinylcarbonyl,
n) N(H)-allylcarbonyl,
o) N(H)-adamantylcarbonyl,
p) N(H)-diphenylmethylcarbonyl,
q) N(H)-cinnamylarbonyl,
r) N(H)-formyl,
s) N(H)-benzoyl,
t) N(H)-trityl,
u) N(H)-p-methoxyphenyl-diphenylmethyl,
v) N(H)-di-(p-methoxyphenyl)-phenylmethyl,
w) 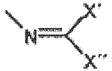 or
x) N-(tert-butoxycarbonyl)₂,
L'" represents
a) branched or unbranched C₁-C₅ alkyl,
b) branched or unbranched C₂-C₅ alkenyl,
c) branched or unbranched C₁-C₅ alkyl-(O-C₁-C₄ alkyl)ₙ-C₁-C₄ alkyl, or
d) branched or unbranched C₂-C₅ alkynyl,
U" represents
a) tert-butoxycarbonyl,
b) allyloxycarbonyl,
c) benzyloxycarbonyl, or
d) ethoxycarbonyl,
X" and X'", independently of one another, represent
a) branched or unbranched C₁-C₅ alkyl,
b) substituted or unsubstituted aryl,
c) alkylaryl, or
d) heteroaryl,
where n = 0, 1, or 2 and
diastereomers and enantiomers.

32. The process for the preparation of compounds of the general formula (II) according to claims 13 to 26,
**characterized in that**
a precursor compound of the compound as set forth in formula (III) according to claim 30 is reacted with F-18 fluoride.

33. The process for the preparation of the compounds of the general formula (I) or (II) according to claims 1 to 26, **characterized in that** the majority of the compounds as set forth in formula (I) and (II) can result from a precursor compound of the compound as set forth in formula (IV) according to claim 31 after introduction of the ¹⁸F-isotope.

## Revendications

1. Composés de la formule générale I 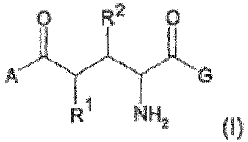 **caractérisé en ce que**
A représente
a) hydroxyle,
b) hydroxy alkoxy en C₁-C₅ ramifié ou non ramifié
c) N(alkyle en C₁-C₅)₂,
d) NH₂,
e) N(H)-L,
f) O-L, ou
g) O-Z,
G représente
a) hydroxyle,
b) O-alkyle en C₁-C₅ ramifié ou non ramifié,
c) O-alkényle en C₂-C₅ ramifié ou non ramifié,
d) O-alkyle en C₁-C₅ ramifié ou non ramifié -(O-alkyle en C₁-C₄)ₙ-O-alkyle en C₁-C₄ ou
e) O-alkynyle en C₂-C₅ ramifié ou non ramifié,
R¹ et R² représentent
a) hydrogène,
b) ¹⁸F-alkoxy en C₁-C₅ ramifié ou non ramifié,
c) ¹⁸F-alkyle en C₁-C₅ ramifié ou non ramifié,
d) ¹⁸F-hydroxy-alkyle en C₁-C₅ ramifié ou non ramifié,
e) ¹⁸F-alkenyle en C₂-C₅ ramifié ou non ramifié, ou
f) ¹⁸F-alkynyle en C₂-C₅ ramifié ou non ramifié,
g) hydroxyle,
h) alkyle en C₁-C₅ ramifié ou non ramifié, ou
i) alkoxy en C₁-C₅ ramifié ou non ramifié,
à condition que l'un des substituants R¹ ou R² contienne exactement un isotope ¹⁸F et que l'autre substituant dans chaque cas ne contienne aucun isotope ¹⁸F,
L représente
a) alkyle en C₁-C₅ ramifié ou non ramifié,
b) alkényle en C₂-C₅ ramifié ou non ramifié,
c) alkyle en C₁-C₅ ramifié ou non ramifié -(O-alkyle en C₁-C₄)ₙO-alkyle en C₁-C₄, ou
d) alkynyle en C₂-C₅, et
Z représente un équivalent de cation métallique qui peut être monovalent ou divalent, les métaux divalents pouvant former une liaison ionique avec deux restes des structures proposées,
**caractérisé en ce que**
n = 0, 1, 2 ou 3 et
diastéréoisomères et énantiomères.

2. Composés selon la revendication 1, **caractérisés en ce que** A représente OH, méthoxy ou NH₂.

3. Composé selon les revendications 1 ou 2, **caractérisé en ce que** A représente OH.

4. Composé selon les revendications 1 ou 2, **caractérisé en ce que** A représente NH₂.

5. Composés selon les revendications 1 à 4, **caractérisés en ce que** R¹ et R² sont choisis dans le groupe comportant hydrogène, ¹⁸F-méthoxy, ¹⁸F-éthoxy, ¹⁸F-propoxy, ¹⁸F-méthyle, ¹⁸F-éthyle et ¹⁸F-propyle, à condition que l'un des substituants R¹ ou R² contienne exactement un isotope ¹⁸F et l'autre substituant dans chaque cas soit hydrogène.

6. Composé selon les revendications 1 à 4, **caractérisé en ce que** R¹ est un ¹⁸F-propyle et R² représente hydrogène.

7. Composé selon la revendication 1 ayant la formule

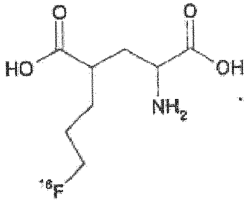

8. Composé selon la revendication 1, **caractérisé en ce qu'**exactement l'un des substituants R¹ ou R² est ¹⁸F-propyle, à condition que l'un des substituants R¹ ou R² contienne exactement un isotope ¹⁸F et l'autre substituant dans chaque cas soit l'hydrogène.

9. Composé selon les revendications 1 à 8, **caractérisé en ce que** G est choisi dans le groupe comprenant OH, méthoxy ou éthoxy.

10. Composé selon les revendications 1 à 9, **caractérisé en ce que** G est choisi dans le groupe comprenant Mg²⁺, Ca²⁺, Na⁺ et K⁺.

11. Composé selon les revendications 1 ou 9, **caractérisé en ce que** Z est Ni²⁺.

12. Composé selon la revendication 1 choisi dans le groupe de composés ayant la formule :
a) 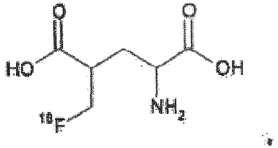
b) 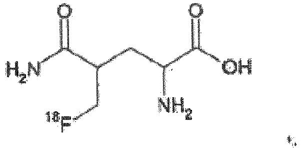
c) 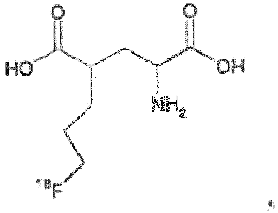
d) 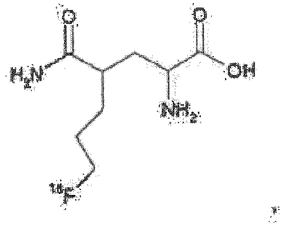
e) 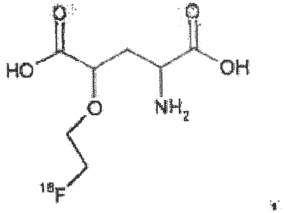
f) 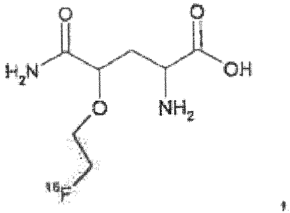
g) 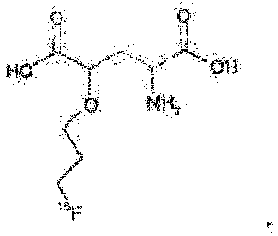
h)

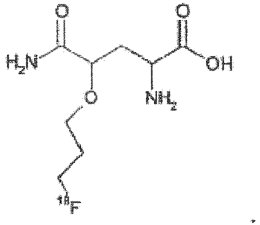

13. Composés de la formule générale (II) : 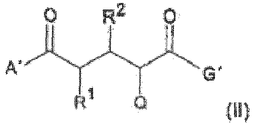 **caractérisé en ce que**
A' représente
a) hydroxyle,
b) hydroxy alkoxy en C₁-C₅ ramifié ou non ramifié,
c) N(alkyle en C₁-C₅)₂,
d) NH₂,
e) N(H)-U
f) N(H)-L', ou
g) O-L',
G' représente
a) hydroxyle,
b) O-Z',
c) O-alkyle en C₁-C₅ ramifié ou non ramifié,
d) O-alkényle en C₂-C₅ ramifié ou non ramifié,
e) O-alkyle en C₁-C₅ ramifié ou non ramifié -(O-alkyle en C₁-C₄)ₙ-O-alkyle en C₁-C₄, ou
f) O-alkynyle en C₂-C₅ ramifié ou non ramifié,
R¹ et R² représentent
a) hydrogène,
b) ¹⁸F-alkoxy en C₁-C₅ ramifié ou non ramifié,
c) ¹⁸F-alkyle en C₁-C₅ ramifié ou non ramifié,
d) ¹⁸F-hydroxy- alkyle-hydroxy en C₁-C₅ ramifié ou non ramifié,
e) ¹⁸F-alkényle en C₂-C₅ ramifié ou non ramifié,
f) ¹⁸F-alkynyle en C₂-C₅ ramifié ou non ramifié,
g) hydroxyle,
h) alkyle en C₁-C₅ ramifié ou non ramifié, ou
i) alkoxy en C₁-C₅ ramifié ou non ramifié,
à condition qu'exactement l'un des substituants R¹ ou R² contienne exactement un isotope ¹⁸F et que l'autre substituant dans chaque cas ne contienne aucun isotope ¹⁸F,
Q représente
a) N(H)-tert-butoxycarbonyle,
b) N(H)-allyloxycarbonyle,
c) N(H)-benzyloxycarbonyle,
d) N(H)-éthoxycarbonyle,
e) N(H)-méthoxycarbonyle,
f) N(H)-propoxycarbonyle,
e) N(H)-2,2,2-trichloréthoxycarbonyle,
f) N(H)-1,1-diméthylpropinyle,
g) N(H)-1-méthyl-1-phényl-éthoxycarbonyle,
h) N(H)-1-méthy)-1-(4-biphénylyl)-éthoxycarbonyle,
i) N(H)-cyclobutylcarbonyle,
j) N(H)-1-méthylcyclobutylcarbonyle,
k) N(H)-vinylcarbonyle,
l) N(H)-allylcarbonyle,
m) N(H)-adamantylcarbonyle,
n) N(H)-diphénylméthylcarbonyle,
o) N(H)-cinnamylcarbonyle,
p) N(H)-formyle,
q) N(H)-benzoyle,
r) N(H)-trityle,
s) N(H)-p-méthoxyphényl-diphénylméthyle,
t) N(H)-di-(p-méthoxyphényl)-phénylméthyle,
u) 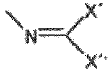 ou
v) N-(tert-butoxycarbonyle)₂,
L' représente
a) alkyle en C₁-C₅ ramifié ou non ramifié,
b) alkényle en C₂-C₅ ramifié ou non ramifié,
c) alkyle en C₁-C₅ ramifié ou non ramifié -(O-alkyle en C₁-C₄)ₙ-O-alkyle en C₁-C₄,
ou
d) alkynyle en C₂-C₅ ramifié ou non ramifié,
U représente
a) tert-butoxycarbonyle,
b) allyloxycarbonyle,
c) benzyloxycarbonyle,
d) éthoxycarbonyle,
e) méthoxycarbonyle, ou
f) propoxycarbonyle,
X' et X", indépendamment l'un de l'autre, représentent
a) alkyle en C₁-C₅ ramifié ou non ramifié,
b) aryle substitué ou non substitué, ou
c) aralkyle, et
Z' représente un équivalent de cation métallique
qui peut être monovalent ou divalent, les métaux divalents pouvant former une liaison ionique avec deux restes des structures proposées ou former des liaisons de coordination avec les fonctions carboxyliques et amines du dérivé d'acide aminé,
n = 0, 1, 2 ou 3 et
diastéréoisomères et énantiomères.

14. Composés selon la revendication 13, **caractérisés en ce que** A' représente OH, alcoxy en C₁-C₅ ramifié ou non ramifié, -NH-tert-butoxycarbonyle ou NH₂.

15. Composés selon les revendications 13 ou 14, **caractérisés en ce que** A' représente éthoxy.

16. Composés selon la revendication 13 à 15, **caractérisés en ce que** A' représente NH₂.

17. Composés selon les revendications 13 à 16, **caractérisés en ce que** R¹ et R² sont choisis dans le groupe comportant hydrogène, ¹⁸F-méthoxy, ¹⁸F-éthoxy, ¹⁸F-propoxy, ¹⁸F-méthyle, ¹⁸F-éthyle et ¹⁸F-propyle, à condition que l'un des substituants R¹ ou R² contienne exactement un isotope ¹⁸F et que l'autre substituant dans chaque cas soit hydrogène.

18. Composé selon les revendications 13 à 16, **caractérisé en ce que** R¹ est ¹⁸F-propyle et R² représente hydrogène.

19. Composé selon les revendications 13 à 18, **caractérisé en ce que** G' est choisi dans le groupe comprenant OH, éthoxy, méthoxy et OZ'.

20. Composé selon les revendications 13 ou 19, **caractérisé en ce que** G' représente éthoxy.

21. Composé selon les revendications 13 à 20, **caractérisé en ce que** Z' est choisi dans le groupe comprenant Na⁺, K⁺, Ca²⁺ et Mg²⁺.

22. Composé selon les revendications 13 à 21, **caractérisé en ce que** Z' est Ni²⁺.

23. Composé selon les revendications 13 à 22, **caractérisé en ce que** Q est choisi dans le groupe comprenant N(H)-tert-butoxycarbonyle, N(H)-benzyloxycarbonyle, et 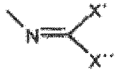 **caractérisé en ce que**
X et X', indépendamment l'un de l'autre, représentent
a) alkyle en C₁-C₅ ramifié ou non ramifié,
b) aryle substitué ou non substitué, ou
c) aralkyle

24. Composé selon les revendications 13 à 23, **caractérisé en ce que** Q représente N(H)-tert-butoxycarbonyle.

25. Composé selon les revendications 13 à 24, **caractérisé en ce que** Q représente

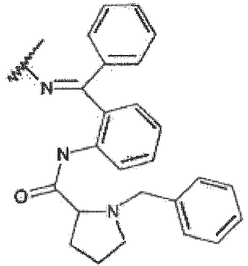

26. Composé selon la revendication 14 choisi dans le groupe de composés ayant la formule :
a) 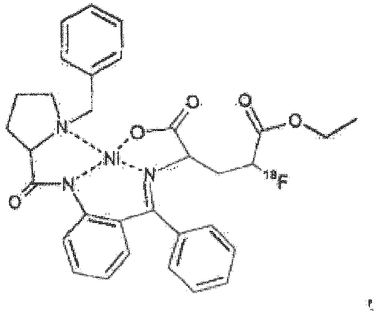
b)

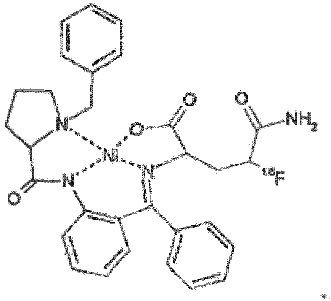

27. Composés selon les revendications 1 à 26 pour utilisation en tant que produit pharmaceutique.

28. Composés selon les revendications 1 à 26 pour utilisation dans le diagnostic des tumeurs.

29. Utilisation de composés selon les revendications 1 à 26 dans la préparation d'un médicament pour le diagnostic des tumeurs.

30. Composés de la formule (III) 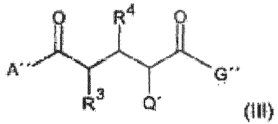 **caractérisé en ce que**
A" représente
a) hydroxyle,
b) hydroxy alkoxy en C₁-C₅ ramifié ou non ramifié ,
c) N(alkyle en C₁-C₅)₂,
d) NH₂,
e) N(H)-U'
f) N(H)-L", ou
g) O-L",
G" représente
a) hydroxyle,
b) O-Z",
c) O-alkyle en C₁-C₅ ramifié ou non ramifié,
d) O-alkényle en C₂-C₅ ramifié ou non ramifié
e) O-alkyle en C₁-C₅ ramifié ou non ramifié -(O-alkyle en C₁-C₄)ₙ-O-alkyle en C₁-C₄,
f) O-alkynyle en C₂-C₅ ramifié ou non ramifié, ou
g) triphénylméthoxy,
R³ et R⁴ représentent
a) hydrogène,
b) E-alkoxy en C₁-C₅ ramifié ou non ramifié,
c) E-alkyle en C₁-C₅ ramifié ou non ramifié,
d) E-alkyle en C₁-C₅ hydroxy ramifié ou non ramifié,
e) E-alkényle en C₂-C₅ ramifié ou non ramifié,
f) E-alkynyle en C₂-C₅ ramifié ou non ramifié,
g) hydroxyle,
h) alkyle en C₁-C₅ ramifié ou non ramifié, ou
i) alkoxy en C₁-C₅ ramifié ou non ramifié,
à condition qu'exactement l'un des substituants R³ ou R⁴ contienne exactement E et que l'autre substituant dans chaque cas ne contienne aucun E,
E représente
a) chloro,
b) bromo,
c) mésyloxy,
d) trifluoromésyloxy,
e) nonafluorobutyloxy, ou
f) tosyloxy,
Q' représente
a) N(H)-tert-butoxycarbonyle,
b) N(H)-allyloxycarbonyle,
c) N(H)-benzyloxycarbonyle,
d) N(H)-éthoxycarbonyle,
e) N(H)-méthoxycarbonyle,
f) N(H)-propoxycarbonyle,
g) N(H)-2,2,2-trichloréthoxycarbonyle,
h) N(H)-1,1-diméthylpropinyle,
i) N(H)-1-méthyl-1-phényl-éthoxycarbonyle,
j) N(H)-1-méthyl-1-(4-biphénylyl)-éthoxycarbonyle,
k) N(H)-cyclobutylcarbonyle,
l) N(H)-1-méthylcyclobutylcarbonyle,
m) N(H)-vinylcarbonyle,
n) N(H)-allylcarbonyle,
o) N(H)-adamantylcarbonyle,
p) N(H)-diphénylméthylcarbonyle,
q) N(H)-cinnamylcarbonyle,
r) N(H)-formyle,
s) N(H)-benzoyle,
t) N(H)-trityle,
u) N(H)-p-méthoxyphényl-diphénylméthyle,
v) N(H)-di-(p-méthoxyphényl)-phénylméthyle,
w) 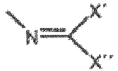 ou
x) N-(tert-butoxycarbonyle)₂,
L" représente
a) alkyle en C₁-C₅ ramifié ou non ramifié,
b) alkényle en C₂-C₅ ramifié ou non ramifié,
c) alkyle en C₁-C₅ ramifié ou non ramifié -(O-alkyle en C₁-C₄)ₙ-O-alkyle en C₁-C₄, ou
d) alkynyle en C₂-C₅ ramifié ou non ramifié,
U' représente
a) tert-butoxycarbonyle,
b) allyloxycarbonyle,
c) benzyloxycarbonyle, ou
d) éthoxycarbonyle,
X' et X", indépendamment l'un de l'autre, représentent
a) alkyle en C₁-C₅ ramifié ou non ramifié,
b) aryle substitué ou non substitué,
c) alkylaryle, ou
d) hétéroaryle, et
Z" représente un équivalent de cation métallique qui peut être monovalent ou divalent, les métaux divalents pouvant former une liaison ionique avec deux restes des structures proposées ou former des liaisons de coordination avec les fonctions carboxyliques et amines du dérivé d'acide aminé,
n = 0, 1 ou 2 et
diastéréoisomères et énantiomères.

31. Utilisation de composés selon la formule (IV) pour la préparation de composés tels que définis à la formule (I) ou (II) : 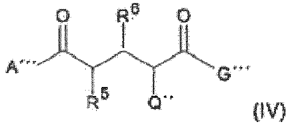 **caractérisé en ce que**
A''' représente
a) hydroxyle,
b) hydroxy alkoxy en C₁-C₅ ramifié ou non ramifié ,
c) N(alkyle en C₁-C₅)₂,
d) NH₂,
e) N(H)-U"
f) N(H)-L''', ou
g) O-L''',
G''' représente
a) hydroxyle,
b) O-alkyle en C₁-C₅ ramifié ou non ramifié,
c) O-alkényle en C₂-C₅ ramifié ou non ramifié
d) O-alkyle en C₁-C₅ ramifié ou non ramifié -(O-alkyle en C₁-C₄)ₙ-O-alkyle en C₁-C₄,
e) O-alkynyle en C₂-C₅ ramifié ou non ramifié, ou
f) triphénylméthoxy,
R⁵ et R⁶ représentent
a) hydrogène ou
b) E',
à condition qu'exactement l'un des substituants R⁵ ou R⁶ contienne E' et que l'autre substituant dans chaque cas contienne hydrogène,
E' représente
a) chloro,
b) bromo,
c) mésyloxy,
d) trifluoromésyloxy,
e) nonafluorobutyloxy, ou
f) tosyloxy,
Q" représente
a) N(H)-tert-butoxycarbonyle,
b) N(H)-allyloxycarbonyle,
c) N(H)-benzyloxycarbonyle,
d) N(H)-éthoxycarbonyle,
e) N(H)-méthoxycarbonyle,
f) N(H)-propoxycarbonyle,
g) N(H)-2,2,2-trichloréthoxycarbonyle,
h) N(H)-1,1-diméthylpropinyle,
i) N(H)-1-méthyl-1-phényl-éthoxycarbonyle,
j) N(H)-1-méthyl-1-(4-biphénylyl)-éthoxycarbonyle,
k) N(H)-cyclobutylcarbonyle,
l) N(H)-1-méthylcyclobutylcarbonyle,
m) N(H)-vinylcarbonyle,
n) N(H)-allylcarbonyle,
o) N(H)-adamantylcarbonyle,
p) N(H)-diphénylméthylcarbonyle,
q) N(H)-cinnamylarbonyle,
r) N(H)-formyle,
s) N(H)-benzoyle,
t) N(H)-trityle,
u) N(H)-p-méthoxyphényl-diphénylméthyle,
v) N(H)-di-(p-méthoxyphényl)-phénylméthyle,
w) 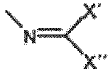 ou
x) N-(tert-butoxycarbonyle)₂,
L''' représente
a) alkyle en C₁-C₅ ramifié ou non ramifié,
b) alkényle en C₂-C₅ ramifié ou non ramifié,
c) alkyle en C₁-C₅ ramifié ou non ramifié -(O-alkyle en C₁-C₄)ₙ-O-alkyle en C₁-C₄,
d) alkynyle en C₂-C₅ ramifié ou non ramifié,
U" représente
a) tert-butoxycarbonyle,
b) allyloxycarbonyle,
c) benzyloxycarbonyle, ou
d) éthoxycarbonyle,
X" et X''', indépendamment l'un de l'autre, représentent
a) alkyle en C₁-C₅ ramifié ou non ramifié,
b) aryle substitué ou non substitué,
c) alkylaryle, ou
d) hétéroaryle,
n = 0, 1 ou 2 et
diastéréoisomères et énantiomères.

32. Procédé de préparation de composés de la formule générale (II) selon les revendications 13 à 26, **caractérisé en ce que**
un composé précurseur du composé tel que défini dans la formule (III) selon la revendication 30 est amené à réagir avec du fluorure de F-18.

33. Procédé de préparation des composés de la formule générale (I) ou (II) selon les revendications 1 à 26, **caractérisé en ce que** la majorité des composés tels que définis dans les formules (I) et (II) peuvent résulter d'un composé précurseur du composé tel que défini dans la formule (IV) selon la revendication 31 après introduction de l'isotope ¹⁸F.
